# EUROPEAN PATENT APPLICATION

(11) **EP 4 254 421 A1**
(43) Date of publication of application: **04.10.2023**
(21) Application number: 21897584.5
(22) Date of filing: 25.10.2021
(51) Int. Cl.: G16H 10/40, G16H 20/00, A61B 5/01, A61B 5/02, A61B 5/107, A61B 5/11, A61B 5/1455

(54) **HEALTH ASSISTANCE DEVICE, HEALTH ASSISTANCE METHOD, AND RECORDING MEDIUM**

(30) Priority: 24.11.2020 JP 2020194127; 24.11.2020 JP 2020194128; 24.11.2020 JP 2020194129
(71) Applicant: Suntory Holdings Limited, Osaka 530-8203 (JP)
(72) Inventor: YAMAMURA Junki, Soraku-gun, Kyoto 619-0284 (JP); ZEIDA Mitsuhiro, Soraku-gun, Kyoto 619-0284 (JP); SUZUKI Yuichi, Tokyo 135-8631 (JP); LEE Eunseo, Soraku-gun, Kyoto 619-0284 (JP); TAKAGI Motoshige, Osaka-shi Osaka 530-8204 (JP)
(74) Representative: Vossius & Partner Patentanwälte Rechtsanwälte mbB
(86) International application number: PCT/JP2021/039321
(87) International publication number: WO 2022/113608

(57) **Abstract**

Conventionally, it is not possible to acquire appropriate information regarding a blood sugar level or alert a user. With a health support device A that includes a blood sugar level acquisition unit C31 that acquires a blood sugar level of a user; a judgment unit C33 that judges whether or not blood the sugar level satisfies a predetermined output condition; and an output unit C4 that outputs blood sugar level information regarding the blood sugar level when the judgment result indicates that the output condition is satisfied, it is possible to acquire appropriate information regarding the blood sugar level and alert the user.

## Description

### Technical Field

The present invention relates to, for example, a health support device that acquires and outputs information regarding a blood sugar level, using a user's blood sugar level.

### Background Art

In the conventional art, there is a non-invasive blood sugar level measuring device (see Patent Document 1, for example).

### Citation List

### Patent Document

Patent Document 1: JP 4052461B

### Summary of Invention

### Technical Problem

However, according to the conventional art, it is not possible to acquire appropriate information regarding the blood sugar level or alert the user. In addition, according to the conventional art, it is not possible to acquire appropriate information regarding blood sugar level fluctuations or alert the user.

### Solution to Problem

A health support device according to a first aspect of the present invention is a health support device including: a blood sugar level acquisition unit that acquires a blood sugar level of a user; a judgment unit that judges whether or not the blood sugar level satisfies a predetermined output condition to acquire a judgment result; and an output unit that outputs blood sugar level information regarding the blood sugar level when the judgment result indicates that the output condition is satisfied.

With such a condition, it is possible to alert the user by outputting blood sugar level information when the acquired blood sugar level satisfies the output condition.

A health support device according to a second aspect of the present invention is the health support device according to the first aspect of the invention, wherein the blood sugar level acquisition unit acquires a blood sugar level group constituted by two or more blood sugar levels of the user acquired in a time series, the health support device further includes a fluctuation information acquisition unit that acquires fluctuation information regarding blood sugar level fluctuations over time, using the blood sugar level group, the judgment unit judges whether or not the fluctuation information satisfies a predetermined output condition, and the output unit outputs the fluctuation information when the judgment result indicates that the output condition is satisfied.

With such a configuration, when fluctuation information regarding blood sugar level fluctuations satisfies a predetermined output condition, it is possible to output the fluctuation information.

A health support device according to a third aspect of the present invention is the health support device according to the second aspect of the invention, further including a reference information storage unit that stores reference information regarding a refence blood sugar level, wherein the fluctuation information acquisition unit uses the blood sugar level group and the reference information to acquire the fluctuation information.

With such a configuration, it is possible to output fluctuation information regarding blood sugar level fluctuations, using the reference information.

A health support device according to a fourth aspect of the present invention is the health support device according to the second or the third aspect of the invention, further including a past blood sugar level group storage unit that stores a past blood sugar level group constituted by two or more past blood sugar levels of the user, wherein the fluctuation information acquisition unit uses the blood sugar level group and the past blood sugar level group to acquire the fluctuation information.

With such a configuration, it is possible to output fluctuation information regarding blood sugar level fluctuations, using the past blood sugar level group.

A health support device according to a fifth aspect of the present invention is the health support device according to any one of the second to fourth aspects of the invention, wherein the blood sugar level group acquired by the blood sugar level acquisition unit includes two or more blood sugar levels that are consecutive in a time series.

With such a configuration, it is possible to output fluctuation information regarding blood sugar level fluctuations, using the two or more blood sugar levels that are consecutive in a time series.

A health support device according to a sixth aspect of the present invention is the health support device according to the fifth aspect of the invention, wherein the blood sugar level group is a blood sugar level group that includes two or more blood sugar levels from an ordinary blood sugar level to a peak blood sugar level, or a blood sugar level group that includes three or more blood sugar levels from an ordinary blood sugar level to the peak blood sugar level and from the peak blood sugar level to an ordinary blood sugar level.

With such a configuration, it is possible to output fluctuation information regarding blood sugar level fluctuations, using ordinary and peak blood sugar levels.

A health support device according to a seventh aspect of the present invention is the health support device according to any one of the second to fourth aspects of the invention, wherein the blood sugar level acquisition unit acquires two or more blood sugar level groups of the user, and the fluctuation information acquisition unit uses the two or more blood sugar level groups to acquire the fluctuation information.

With such a configuration, it is possible to output fluctuation information regarding blood sugar level fluctuations, using the two or more blood sugar level groups.

A health support device according to an eighth aspect of the present invention is the health support device according to the seventh aspect of the invention, wherein the two or more blood sugar level groups are two or more blood sugar level groups acquired in a same time period on different days, or two or more blood sugar level groups corresponding to an occurrence of a same event.

With such a configuration, it is possible to output fluctuation information regarding blood sugar level fluctuations, using two or more blood sugar level groups acquired in the same time period, or two or more blood sugar level groups corresponding to the occurrence of the same event.

A health support device according to a ninth aspect of the present invention is the health support device according to any one of the second to eighth aspects of the invention, wherein the fluctuation information acquisition unit acquires fluctuation information that is a score corresponding to blood sugar level fluctuations over time, using a blood sugar level group.

With such a configuration, it is possible to output a score regarding blood sugar level fluctuations.

A health support device according to a tenth aspect of the present invention is the health support device according to any one of the second to ninth aspects of the invention, wherein the output condition is a warning condition for outputting a warning, and the fluctuation information acquisition unit acquires fluctuation information regarding the warning when the judgment result indicates that the warning condition is satisfied.

With such a configuration, it is possible to output a warning regarding blood sugar level fluctuations.

A health support device according to an eleventh aspect of the present invention is the health support device according to any one of the first to tenth aspects of the invention, wherein the health support device has a shape of a wristwatch.

With such a configuration, it is possible to alert the user regarding the blood sugar level anytime.

A health support device according to an aspect of the present invention is a health support device including: a blood sugar level acquisition unit that acquires a blood sugar level of one user; a judgment unit that judges whether or not blood sugar level-related information regarding the blood sugar level satisfies a predetermined suggestion condition, to acquire a judgment result; a suggestion information acquisition unit that acquires suggestion information when the judgment result indicates that the suggestion condition is satisfied; and a suggestion information output unit that outputs the suggestion information.

With such a configuration, it is possible to acquire information regarding the blood sugar level, and provide the user with an appropriate suggestion, using the information.

A health support device according to an aspect of the present invention is the health support device according to the above-described aspects of the invention, wherein the blood sugar level acquisition unit acquires a blood sugar level group constituted by two or more blood sugar levels of one user acquired in a time series on one day, the health support device further includes a fluctuation information acquisition unit that acquires fluctuation information regarding blood sugar level fluctuations over time, using the blood sugar level group, the judgment unit judges whether or not the fluctuation information satisfies the suggestion condition, and the suggestion information acquisition unit acquires suggestion information corresponding to the fluctuation information when the judgment result indicates that the suggestion condition is satisfied.

With such a configuration, it is possible to acquire fluctuation information regarding the blood sugar level, and provide the user with an appropriate suggestion, using the fluctuation information.

A health support device according to an aspect of the present invention is the health support device according to the above-described aspects of the invention, further including a reference information storage unit that stores reference information regarding a refence blood sugar level, wherein the fluctuation information acquisition unit uses the blood sugar level group and the reference information to acquire the fluctuation information.

With such a configuration, it is possible to acquire fluctuation information regarding the blood sugar level, using the reference information, and provide the user with an appropriate suggestion, using the fluctuation information.

A health support device according to an aspect of the present invention is the health support device according to the above-described aspects of the invention, further including a past blood sugar level group storage unit that stores a past blood sugar level group constituted by two or more past blood sugar levels of one user, wherein the fluctuation information acquisition unit uses the blood sugar level group and the past blood sugar level group to acquire the fluctuation information.

With such a configuration, it is possible to acquire fluctuation information regarding the blood sugar level, using the past blood sugar level group, and provide the user with an appropriate suggestion, using the fluctuation information.

A health support device according to an aspect of the present invention is the health support device according to the above-described aspects of the invention, wherein the suggestion information acquisition unit determines a suggestion condition that matches acquired blood sugar level-related information from a correspondence information storage unit that stores two or more pieces of correspondence information that each indicate a correspondence between a suggestion condition regarding blood sugar level related information and suggestion information or suggestion basis information that is a basis of suggestion information, and acquires suggestion information corresponding to the suggestion condition or suggestion information that uses suggestion basis information corresponding to the suggestion condition.

With such a configuration, it is possible to acquire information regarding the blood sugar level, and provide the user with an appropriate suggestion, using the information.

A health support device according to an aspect of the present invention is the health support device according to the above-described aspects of the invention, wherein the suggestion information includes information specifying advice on foods to be ingested, information specifying advice on lifestyle habits, information identifying advice on exercise, or information specifying advice on diet.

With such a configuration, it is possible to acquire information regarding the blood sugar level, and provide the user with an appropriate suggestion, using the information.

A health support device according to an aspect of the present invention is the health support device according to the above-described aspects of the invention, the suggestion information acquisition unit immediately acquires the suggestion information when the judgment result acquired by the judgment unit in response to the blood sugar level acquisition unit acquiring the blood sugar level indicates that the suggestion condition is satisfied, and the suggestion information output unit immediately outputs the suggestion information when the suggestion information acquisition unit acquires the suggestion information.

With such a configuration, it is possible to provide the user with an appropriate real-time suggestion when the blood sugar level is acquired.

A health support device according to an aspect of the present invention is the health support device according to the above-described aspects of the invention, wherein the judgment unit judges whether or not the blood sugar level or the fluctuation information satisfies the suggestion condition, using the blood sugar level and information other than the fluctuation information.

With such a configuration, it is possible to more appropriately judge whether or not to output the suggestion information, using information other than the information regarding the blood sugar level.

A health support device according to an aspect of the present invention is the health support device according to the above-described aspects of the invention, wherein the suggestion information acquisition unit also uses the blood sugar level and information other than the fluctuation information to acquire the suggestion information.

With such a configuration, it is possible to acquire more appropriate suggestion information, using information other than the information regarding the blood sugar level as well.

A health support device according to an aspect of the present invention is the health support device according to the above-described aspects of the invention, further including an environment information acquisition unit that acquires environment information regarding user environment, wherein the other information is the environmental information.

With such a configuration, it is possible to acquire suggestion information, using the environmental information as well.

A health support device according to an aspect of the present invention is the health support device according to the above-described aspects of the invention, wherein the blood sugar level group acquired by the blood sugar level acquisition unit includes two or more blood sugar levels that are consecutive in a time series.

With such a configuration, it is possible to acquire fluctuation information, using an appropriate blood sugar level group.

A health support device according to an aspect of the present invention is the health support device according to the above-described aspects of the invention, wherein the blood sugar level group is a blood sugar level group that includes two or more blood sugar levels from an ordinary blood sugar level to a peak blood sugar level, or a blood sugar level group that includes three or more blood sugar levels from an ordinary blood sugar level to the peak blood sugar level and from the peak blood sugar level to an ordinary blood sugar level.

With such a configuration, it is possible to acquire fluctuation information, using an appropriate blood sugar level group.

A health support device according to an aspect of the present invention is the health support device according to the above-described aspects of the invention, wherein the blood sugar level acquisition unit acquires two or more blood sugar level groups of one user, and the fluctuation information acquisition unit uses the two or more blood sugar level groups to acquire the fluctuation information.

With such a configuration, by using two or more blood sugar level groups of one user, it is possible to detect an abnormality in the fluctuations of the user's blood sugar level, and to provide the user with a suggestion based on the result of the detection.

A health support device according to an aspect of the present invention is the health support device according to the above-described aspects of the invention, wherein the two or more blood sugar level groups are two or more blood sugar level groups acquired in a same time period on different days, or two or more blood sugar level groups corresponding to an occurrence of a same event.

With such a configuration, by using two or more appropriate blood sugar level groups of one user, it is possible to detect an abnormality in the fluctuations of the user's blood sugar level, and to provide the user with a suggestion based on the result of the detection.

A health support device according to an aspect of the present invention is the health support device according to the above-described aspects of the invention, wherein the fluctuation information acquisition unit acquires fluctuation information that is a score corresponding to blood sugar level fluctuations over time, using a blood sugar level group.

With such a configuration, it is possible to acquire a score regarding blood sugar level fluctuations within a day, and provide the user with an appropriate suggestion, using the score.

A blood sugar level estimation device according to an aspect of the present invention is a blood sugar level estimation device including: a learning information storage unit that stores learning information acquired using two or more pieces of training data that each include one or more pieces of NIRS information acquired using a reflected light of near-infrared light emitted to a human body and a blood sugar level; an NIRS acquisition unit that acquires one or more pieces of NIRS information that can be acquired by emitting near-infrared light to a user; an estimation unit that acquires an estimated blood sugar level, using the one or more pieces of NIRS information acquired by the NIRS acquisition unit and the learning information; and a blood sugar level output unit that outputs the blood sugar level acquired by the estimation unit.

With such a configuration, it is possible to acquire an appropriate blood sugar level of a person easily and non-invasively.

A blood sugar level estimation device according to an aspect of the present invention is the blood sugar level estimation device according to the above-described aspects of the invention, wherein the training data includes one or more pieces of NIRS information that can be acquired by emitting near-infrared light of two or more wavelengths to a human body, and the NIRS acquisition unit acquires one or more pieces of NIRS information that can be acquired by emitting near-infrared light of two or more wavelengths to a user.

With such a configuration, it is possible to acquire a more appropriate blood sugar level easily and non-invasively, using near-infrared light of two or more wavelengths.

A blood sugar level estimation device according to an aspect of the present invention is the blood sugar level estimation device according to the above-described aspects of the invention, wherein the two or more wavelengths are two or more wavelengths within the range of approximately 760 nm to approximately 1300 nm.

With such a configuration, it is possible to acquire a more appropriate blood sugar level easily and non-invasively, using near-infrared light of two or more appropriate wavelengths.

A blood sugar level estimation device according to an aspect of the present invention is the blood sugar level estimation device according to the above-described aspects of the invention, wherein the number of near-infrared light rays of two or more different wavelengths used to acquire learning information is larger than the number of near-infrared light rays of two or more different wavelengths used by the NIRS acquisition unit to acquire one or more pieces of NIRS information.

With such a configuration, it is possible to acquire a more appropriate blood sugar level easily and non-invasively.

A blood sugar level estimation device according to an aspect of the present invention is the blood sugar level estimation device according to the above-described aspects of the invention, wherein the training data includes one or more user dynamic attribute values of: a skin temperature; an acceleration; a pulse; a pulse wave; a pulse rate; a saturated oxygen concentration in blood; a blood pressure; a skin color; a muscle mass; and the amount of hemoglobin in blood, of a person with a human body, the blood sugar level estimation device further includes user dynamic attribute value acquisition unit that acquires one or more user dynamic attribute values of: a skin temperature; an acceleration; a pulse; a pulse wave; a pulse rate; a saturated oxygen concentration in blood; a blood pressure; a skin color; a muscle mass; and the amount of hemoglobin in blood, of one user, and the estimation unit uses the one or more pieces of NIRS information acquired by the NIRS acquisition unit, the one or more user dynamic attribute values acquired by the user dynamic attribute value acquisition unit, and the learning information, to acquire the estimated blood sugar level.

With such a configuration, it is possible to acquire a more appropriate blood sugar level easily and non-invasively, using one or more user dynamic attribute values.

A blood sugar level estimation device according to an aspect of the present invention is the blood sugar level estimation device according to the above-described aspects of the invention, wherein the training data includes one or more user static attribute values that are static attribute values of a person with the human body, the blood sugar level estimation device further includes a user static attribute value acquisition unit that acquires one or more user static attribute values of the user, and the estimation unit uses the one or more pieces of NIRS information acquired by the NIRS acquisition unit, the one or more user static attribute values acquired by the user static attribute value acquisition unit, and the learning information, to acquire the estimated blood sugar level.

With such a configuration, it is possible to acquire a more appropriate blood sugar level easily and non-invasively, using one or more user static attribute values as well.

A blood sugar level estimation device according to an aspect of the present invention is the blood sugar level estimation device according to the above-described aspects of the invention, wherein the one or more user static attribute values are one or more pieces of information of: an age; a height; a weight; and a forearm circumference, of the user.

With such a configuration, it is possible to acquire a more appropriate blood sugar level non-invasively, using one or more appropriate user static attribute values as well.

A blood sugar level estimation device according to an aspect of the present invention is the blood sugar level estimation device according to the above-described aspects of the invention, wherein the training data includes one or more device characteristic values that are characteristic values of a device that is used to acquire the one or more pieces of NIRS information, the blood sugar level estimation device further includes a device characteristic value acquisition unit that acquires one or more device characteristic values of a device that is used to acquire one or more pieces of NIRS information of the user, and the estimation unit uses the one or more pieces of NIRS information acquired by the NIRS acquisition unit, the one or more device characteristic values acquired by the device characteristic value acquisition unit, and the learning information, to acquire the estimated blood sugar level.

With such a configuration, it is possible to acquire a more appropriate blood sugar level non-invasively, using one or more device characteristic values.

A blood sugar level estimation device according to an aspect of the present invention is the blood sugar level estimation device according to the above-described aspects of the invention, the one or more device characteristic values are one or more pieces of information of: distance information specifying a distance between two or more light receiving units; the number of light receiving units; and the number of light emitting units.

With such a configuration, it is possible to acquire a more appropriate blood sugar level non-invasively, using one or more appropriate device characteristic values.

A blood sugar level estimation device according to an aspect of the present invention is the blood sugar level estimation device according to the above-described aspects of the invention, wherein the NIRS acquisition unit acquires one or more pieces of NIRS information or one or more NIRS information bases from two or more NIRS devices that each acquire one or more pieces of NIRS information or one or more NIRS information bases that are bases of one or more pieces of NIRS information, from a user, and the blood sugar level output unit transmits the blood sugar level acquired by the estimation unit of blood sugar level related information regarding the blood sugar level, to the NIRS devices or terminal devices corresponding to the NIRS devices.

With such a configuration, it is possible to acquire an appropriate blood sugar level of each of two or more users easily and non-invasively.

A blood sugar level estimation device according to an aspect of the present invention is the blood sugar level estimation device according to the above-described aspects of the invention, wherein the learning information is a learning model acquired through machine learning processing, using two or more pieces of training data.

With such a configuration, it is possible to acquire a more appropriate blood sugar level easily and non-invasively, using a machine learning algorithm.

A learning device according to one aspect of the present invention is a learning device including: an NIRS acquisition unit that, using reflected light of near-infrared light emitted from one or more light emitting units and received by one or more light receiving units, acquires one or more pieces of NIRS information regarding the reflected light; a measured blood sugar level acquisition unit that acquires a blood sugar level of a user; a training data forming unit that forms training data, using the one or more pieces of NIRS information and the blood sugar level; a learning unit that acquires learning information, using the training data; and an accumulation unit that accumulates the learning information.

With such a configuration, it is possible to acquire learning information that is used to acquire an appropriate blood sugar level of a person non-invasively.

A learning device according to an aspect of the present invention is the learning device according to the above-described aspects of the invention, wherein the one or more light emitting units emit near-infrared light of two or more wavelengths, the one or more light receiving units receive reflected light of the near-infrared light of two or more wavelengths, the NIRS acquisition unit acquires one or more pieces of NIRS information regarding the reflected light of the near-infrared light of two or more wavelengths.

With such a configuration, it is possible to acquire learning information that is used to acquire an appropriate blood sugar level of a person non-invasively.

A learning device according to an aspect of the present invention is the learning device according to the above-described aspects of the invention, further including a user dynamic attribute value acquisition unit that acquires one or more user dynamic attribute values of: a skin temperature; an acceleration; a pulse; a pulse wave; a pulse rate; a saturated oxygen concentration in blood; a blood pressure; a skin color; a muscle mass; and the amount of hemoglobin in blood, of the user, and the training data forming unit uses the one or more pieces of NIRS information, the one or more user dynamic attribute values, and the blood sugar level, to form the training data.

With such a configuration, it is possible to acquire learning information that is used to acquire an appropriate blood sugar level of a person non-invasively, using one or more user dynamic attribute values.

A learning device according to an aspect of the present invention is the learning device according to the above-described aspects of the invention, further including a user static attribute value acquisition unit that acquires one or more user static attribute values that are static attribute values of the user, wherein the training data forming unit uses the one or more pieces of NIRS information, the one or more user static attribute values, and the blood sugar level, to form the training data.

With such a configuration, it is possible to acquire learning information that is used to acquire a more appropriate blood sugar level of a person non-invasively, using one or more user static attribute values.

A learning device according to an aspect of the present invention is the learning device according to the above-described aspects of the invention, further including a device characteristic value acquisition unit that acquires one or more device characteristic values of a device that includes one or more light receiving units and one or more light transmitting units, wherein the training data forming unit uses the one or more pieces of NIRS information, the one or more device characteristic values, and the blood sugar level, to form the training data.

With such a configuration, it is possible to acquire learning information that is used to acquire a more appropriate blood sugar level of a person non-invasively, using one or more device characteristic values.

### Advantageous Effects of Invention

With the health support device according to an aspect of the present invention, it is possible to acquire appropriate information regarding the blood sugar level and alert the user. Note that the alert is preferably a warning to the user, but it can also be, for example, raising the user's awareness of the blood sugar level by, for example, constantly or frequently presenting information regarding the blood sugar level or blood sugar level fluctuations to the user.

### Brief Description of Drawings

FIG. 1 is a block diagram for a health support device A according to a first embodiment.
FIG. 2 is a flowchart illustrating examples of operations of the health support device A according to the same.
FIG. 3 is a flowchart illustrating an example of fluctuation information acquisition processing according to the same.
FIG. 4 is a diagram showing an external appearance of the health support device A and an example output of information according to the same.
FIG. 5 is a diagram showing a warning condition management table according to the same.
FIG. 6 is a conceptual diagram for a health support system B according to a second embodiment.
FIG. 7 is a block diagram for the health support system B according to the same.
FIG. 8 is a conceptual diagram for the health support system B according to the same.
FIG. 9 is a block diagram for a health support device A according to a third embodiment.
FIG. 10 is a flowchart illustrating examples of operations of the health support device A according to the same.
FIG. 11 is a flowchart illustrating an example of fluctuation information acquisition processing according to the same.
FIG. 12 is a diagram showing an external appearance of the health support device A and an example output of information according to the same.
FIG. 13 is a diagram showing a correspondence information management table according to the same.
FIG. 14 is a diagram showing an example output of suggestion information according to the same.
FIG. 15 is a diagram showing an example output of suggestion information according to the same.
FIG. 16 is a conceptual diagram for a health support system B according to a fourth embodiment.
FIG. 17 is a block diagram for the health support system B according to the same.
FIG. 18 is a conceptual diagram for the health support system B according to the same.
FIG. 19 is a conceptual diagram for a blood sugar level estimation system C according to a fifth embodiment.
FIG. 20 is a diagram showing an example of a block diagram for the blood sugar level estimation system C according to the same.
FIG. 21 is a flowchart illustrating examples of operations of a blood sugar level estimation device 7 according to the same.
FIG. 22 is a diagram showing an example of a NIRS device 6 according to the same.
FIG. 23 is a diagram showing an example of the NIRS device 6 according to the same.
FIG. 24 is a diagram showing an example of the NIRS device 6 according to the same.
FIG. 25 is a diagram showing an example of a block diagram for a blood sugar level estimation system C according to the same.
FIG. 26 is a conceptual diagram for a blood sugar level estimation system D according to a sixth embodiment.
FIG. 27 is a block diagram for the blood sugar level estimation system D according to the same.
FIG. 28 is a flowchart illustrating examples of operations of a blood sugar level estimation device 7 according to the same.
FIG. 29 is a block diagram for a learning system E according to a seventh embodiment.
FIG. 30 is a block diagram for the learning system E according to the same.
FIG. 31 is a flowchart illustrating examples of operations of a learning device 9 according to the same.
FIG. 32 is a diagram illustrating a specific example operation of the learning system E according to the same.
FIG. 33 is an overview diagram for a computer system according to the above embodiments.
FIG. 34 is a block diagram for the computer system according to the same.

### Description of Embodiment

Hereinafter, embodiments of a health support device and so on will be described with reference to the drawings. Note that the components with the same reference numerals in the embodiments perform the same operations, and therefore the redundant descriptions thereof may be omitted.

### First Embodiment

The present embodiment describes a health support device that acquires and output blood sugar level information regarding a blood sugar level, using the user's blood sugar level. Note that blood sugar level information is, for example, a blood sugar level itself or warning information.

In addition, the present embodiment describes a health support device that acquires and outputs fluctuation information regarding blood sugar level fluctuations, using two or more blood sugar levels of the user acquired in a time series. Note that fluctuation information is, for example, a score or warning information.

In addition, the present embodiment describes a health support device that acquires fluctuation information based on a comparison with reference information.

In addition, the present embodiment describes a health support device that acquires fluctuation information based on a comparison with a set of blood sugar levels of the user acquired in the past.

In addition, the present embodiment describes a health support device that acquires and outputs fluctuation information regarding a blood sugar level, using a blood sugar level group that is a set of blood sugar levels that are consecutive in a time series. Note that such a blood sugar level group is, for example, a blood sugar level group that is a set of blood sugar levels acquired in a period between an ordinary time and a peak, or a blood sugar level group that is a set of blood sugar levels acquired in a period from an ordinary time to a peak and a period from the peak to an ordinary time.

Furthermore, the present embodiment describes a health support device that acquires fluctuation information, using two or more blood sugar level groups acquired at different times. Note that the two or more blood sugar level groups acquired at different times are two or more blood sugar level groups acquired in the same time period, or two or more blood sugar level groups corresponding to the occurrence of the same event such as a meal.

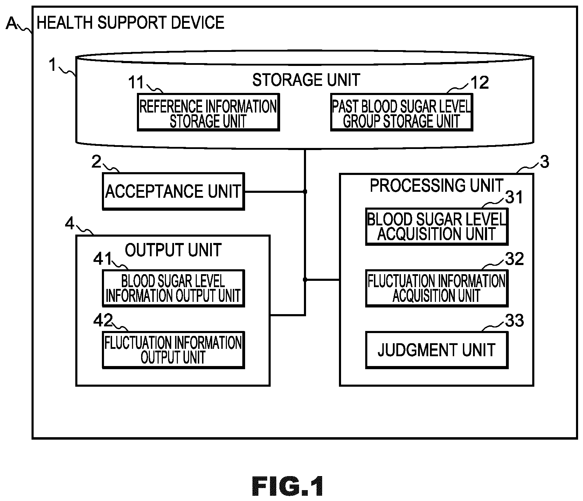

FIG. 1 is a block diagram for a health support device A according to the present embodiment. The health support device A includes a storage unit 1, an acceptance unit 2, a processing unit 3, and an output unit 4.

The storage unit 1 includes a reference information storage unit 11 and a past blood sugar level group storage unit 12. The processing unit 3 includes a blood sugar level acquisition unit 31, a fluctuation information acquisition unit 32, and a judgment unit 33. The output unit 4 includes a blood sugar level information output unit 41 and a fluctuation information output unit 42.

The storage unit 1 stores various kinds of information. Examples of the various kinds of information include reference information, which will be described later, a past blood sugar level group, which will be described later, an output condition, and a processing condition, which will be described later. The output condition may be common to all users or may be associated with user identifiers. The output condition may be the warning condition described below.

The output conditions are conditions for outputting information. Examples of the output conditions include a blood sugar level information output condition and a fluctuation information output condition.

The blood sugar level information output condition is a condition for outputting blood sugar level information. The blood sugar level information output condition may be a condition regarding the blood sugar level only, or a condition regarding the sugar level and timing. The timing is information regarding time or information regarding an event. The blood sugar level information output condition is, for example, "the blood sugar level is not in the normal range indicated by the reference information described below", "the blood sugar level is no lower than or higher than a threshold value", "the blood sugar level is no higher than or lower than a threshold value", "the blood sugar level is of an ordinary time and the blood sugar level is no lower than or higher than a threshold value", "the blood sugar level is no lower than or higher than a threshold value within a specified period (e.g. within 2 hours) after an event such as a meal", and "the blood sugar level is no lower than or higher than a threshold value at a predetermined time after bedtime (e.g. 12 midnight)".

The fluctuation information output condition is a condition for outputting fluctuation information. The blood sugar level information output condition may be a condition regarding the blood sugar level only, or a condition regarding the sugar level and the timing. The timing is information regarding time or information regarding an event. The fluctuation information output condition is, for example, "fluctuation information does not fall within the normal range indicated by the reference information", "fluctuation information is no lower than or higher than a threshold value", "fluctuation information is of a particular event and fluctuation information is no lower than or higher than a threshold value", or "fluctuation information is no lower than or higher than a threshold value in a predetermined time after bedtime (e.g. within one hour between 12 to 1 midnight). Details of the fluctuation information will be described later.

The reference information storage unit 11 stores reference information regarding a refence blood sugar level. Reference information is, for example, associated with information specifying timing. Examples of reference information include the ordinary blood sugar level, the representative value of the peak blood sugar level, the representative value of the difference between the ordinary blood sugar level and the peak blood sugar level, and the representative value of the time interval between the time corresponding to the ordinary blood sugar level and the time corresponding to the peak blood sugar level. Note that each representative value is, for example, an average value, a median value, or the like of two or more values (a blood sugar level, a difference, a time interval, etc.). Each representative value may be a reference normal value. Reference information may be, for example, information indicating a normal blood sugar level range, information indicating a normal blood sugar level fluctuation range, or information indicating a normal blood sugar level fluctuation trend range.

Reference information is, for example, stored in association with a user identifier. The user identifier is information identifying a user. The user identifier may be, for example, an ID, an email address, a telephone number, a name, a terminal identifier of the user's terminal device, or the like, and there is no limitation. The terminal identifier is, for example, an IP address, a MAC address, or the like, and there is no limitation.

The past blood sugar level group storage unit 12 stores a past blood sugar level group constituted by two or more past blood sugar levels. A past blood sugar level is a blood sugar level in the past of the user. Each of the blood sugar levels included in the past blood sugar level group is associated with time information regarding the time at which the blood sugar level was acquired. The past blood sugar level group is, for example, stored in association with a user identifier. The past blood sugar level group is a set of blood sugar levels in the past acquired when the user is in good health. The past blood sugar level group is information acquired from a set of blood sugar levels for each day of two or more days, and is a set of representative values (for example, average values, median values, or maximum values) of the blood sugar levels acquired at the same time of day. The same time of day is, for example, the same time after the start of a meal, the same time after the start of exercise, or the same time after sleep. Note that the time information is, for example, the time, but may be information specifying a relative time with respect to a certain point in time. The certain point in time is, for example, the point in time when an event identifier, which will be described later, was acquired.

The acceptance unit 2 accepts various kinds of instructions and information. Examples of the various kinds of instructions and information include a fluctuation information output instruction, a blood sugar level output instruction, and an event identifier.

The fluctuation information output instruction is an instruction to output fluctuation information. The blood sugar level output instruction is an instruction to output the current blood sugar level.

The event identifier is information identifying an event. Examples of the event identifier include meal start information, in-meal information, meal end information, exercise start information, in-exercise information, exercise end information, insulin injection information, and bedtime information.

Meal start information is information indicating that a meal has started, in-meal information is information indicating that a meal is in progress, and meal end information is information indicating that a meal has ended. Exercise start information is information indicating that exercise has started, in-exercise information is information indicating that exercise is in progress, and exercise end information is information indicating that exercise has ended. Insulin injection information is information indicating that an insulin injection has been given. Bedtime information is information indicating that the user is about to go to bed, or information indicating that the user is currently asleep.

The acceptance unit 2 accepts an event identifier from a user, for example. Note that the acceptance unit 2 may automatically acquire an event identifier, for example. For example, when the temperature acquired by the temperature sensor of the health support device A rises by a predetermined degree or more, and when the gyro sensor or acceleration sensor of the health support device A detects that the device is not moving, the acceptance unit 2 acquires an event identifier that is meal start information. When the start of movement is detected by the gyro sensor or acceleration sensor of the health support device A, the acceptance unit 2 acquires an event identifier that is exercise start information. The acceptance unit 2 may, for example, automatically detect that the user is asleep, based on the sensing results of a heart rate monitor, a temperature sensor, or the like. Note that the detection of sleep can be achieved using a well-known technique.

The means used by the user to input various kinds of instructions and information may be any means such as a touch panel, a keyboard, a mouse, a means that employs a menu screen, or the like.

The processing unit 3 performs various kinds of processing. Examples of the various kinds of processing include the processing that is performed by the blood sugar level acquisition unit 31, the fluctuation information acquisition unit 32, and the judgment unit 33.

The processing unit 3 acquires blood sugar level information, using the blood sugar level acquired by the blood sugar level acquisition unit 31. Examples of blood sugar level information include the blood sugar level itself, the difference between the blood sugar level and reference information, the difference between the blood sugar level and a blood sugar level included in the past blood sugar level group, and a score that is based on the blood sugar level.

For example, the processing unit 3 calculates the difference between the blood sugar level and reference information. For example, the processing unit 3 calculates the difference between the blood sugar level and a blood sugar level that was acquired under the same condition as the condition corresponding to the blood sugar level, and is included in the past blood sugar level group. For example, the same condition means that the blood sugar level is associated with the same time information, or the blood sugar level is associated with the same event identifier and the time elapsed from the occurrence of the event is associated with approximately the same time, or the like.

For example, the processing unit 3 acquires a score, using the blood sugar level, or the difference between the blood sugar level and reference information, or the difference between the blood sugar level and a blood sugar level included in the past blood sugar level group. For example, the processing unit 3 acquires a lower score for a larger difference between the blood sugar level and reference information. For example, the processing unit 3 acquires a lower score for a larger difference between the blood sugar level and the blood sugar level included in the past blood sugar level group.

The blood sugar level acquisition unit 31 acquires the user's blood sugar levels. For example, the blood sugar level acquisition unit 31 acquires blood sugar levels generally on a regular basis. It is preferable that the blood sugar level acquisition unit 31 acquires blood sugar levels in combination with time information. For example, the blood sugar level acquisition unit 31 acquires time information from a clock or a timer (not shown), but may acquire time information in any manner. Note that any method or device may be used by the blood sugar level acquisition unit 31 to acquire the blood sugar level. In addition, there is no limitation on the time at which the blood sugar level acquisition unit 31 acquires the blood sugar level.

For example, the blood sugar level acquisition unit 31 acquires a blood sugar level group constituted by two or more blood sugar levels of the user acquired in a time series on one day. It is preferable that the blood sugar level acquisition unit 31 acquires two or more blood sugar level groups of the user in different time periods. Note that the different time periods are, for example, different days, morning and afternoon, a predetermined time before or after breakfast, a predetermined time before or after lunch, or a predetermined time before or after dinner. It is preferable that the interval between the times at which two or more blood sugar levels are acquired in the time series is short, such as every second, but the time interval is not limited.

For example, the blood sugar level acquisition unit 31 may acquire a blood sugar level group in a section that satisfies a predetermined condition from the blood sugar level group that includes the two or more acquired blood sugar levels. That is to say, the blood sugar level acquisition unit 31 acquires ordinary blood sugar levels whose fluctuation value is no higher than a threshold value from the acquired blood sugar level group. In addition, the blood sugar level acquisition unit 31 acquires the maximum blood sugar level in the blood sugar level group. The blood sugar level acquisition unit 31 may acquire a blood sugar level group that includes two or more blood sugar levels from the ordinary blood sugar level to the peak blood sugar level.

In addition, the blood sugar level acquisition unit 31 may further acquire a blood sugar level group that include blood sugar levels from the blood sugar level acquired at the time after the time corresponding to the peak blood sugar level and closest to the time, to the ordinary blood sugar level. That is to say, the blood sugar level group is, for example, a blood sugar level group that includes two or more blood sugar levels from the ordinary blood sugar level to the peak blood sugar level, or a blood sugar level group that includes three or more blood sugar levels from the ordinary blood sugar level to the peak blood sugar level and from the peak blood sugar level to the ordinary blood sugar level.

For example, the blood sugar level acquisition unit 31 may acquire two or more blood sugar level groups in the same time period (for example, two hours from 7:00 including breakfast time) from blood sugar level groups respectively corresponding to different days. In addition, for example, the blood sugar level acquisition unit 31 may acquire two or more blood sugar level groups corresponding to the occurrence of the same event from blood sugar level groups respectively corresponding to different days. A blood sugar level group corresponding to the occurrence of the same event is usually a set of blood sugar levels that are paired with the same event identifier. That is to say, two or more blood sugar level groups are, for example, two or more blood sugar level groups acquired in the same time period on different days, or two or more blood sugar level groups corresponding to the occurrence of the same event. The event is, for example, a meal, an exercise, having an insulin injection, or the like. Note that two or more blood sugar level groups corresponding to the occurrence of the same event are two or more blood sugar level groups corresponding to the same event identifier.

Note that, for example, the blood sugar level acquisition unit 31 acquires a blood sugar level acquired after the acceptance unit 2 accepts an event identifier, in association with the event identifier. In addition, the blood sugar level acquisition unit 31 accumulates the blood sugar level in a buffer (not shown) or the storage unit 1, in association with the event identifier.

The fluctuation information acquisition unit 32 acquires fluctuation information, using the blood sugar level group acquired by the blood sugar level acquisition unit 31. Fluctuation information is information regarding blood sugar level fluctuations over time.

Information that is used by the fluctuation information acquisition unit 32 to acquire fluctuation information can be (a) to (c) below, for example.
(a) In the case of only using the acquired blood sugar level group

For example, the fluctuation information acquisition unit 32 only uses the blood sugar level group acquired by the blood sugar level acquisition unit 31, to acquire fluctuation information. In the case of only using the blood sugar level group acquired by the blood sugar level acquisition unit 31, the fluctuation information to be acquired can be (a-1) to (a-4) below, for example.

### (a-1) In the case where fluctuation information is the difference between the lowest blood sugar level and the highest blood sugar level

Fluctuation information is, for example, the difference between the lowest blood sugar level and the highest blood sugar level in the blood sugar level group. For example, the fluctuation information acquisition unit 32 acquires the lowest blood sugar level and the highest blood sugar level in the acquired blood sugar level group, calculates the difference between the lowest blood sugar level and the highest blood sugar level, and acquires the difference as fluctuation information. Note that the difference between the lowest blood sugar level and the highest blood sugar level in the blood sugar level group may be referred to as a blood sugar level fluctuation degree.

### (a-2) In the case where fluctuation information is a blood sugar level fluctuation trend

Fluctuation information is, for example, a blood sugar level fluctuation trend. The blood sugar level fluctuation trend is information indicating the trend of the blood sugar level fluctuations. The blood sugar level fluctuation trend is, for example, calculated using the lowest blood sugar level and the highest blood sugar level in the acquired blood sugar level group. The blood sugar level fluctuation trend is, for example, calculated using the lowest blood sugar level and the highest blood sugar level acquired within a predetermined time (for example, 5 minutes or 30 minutes) in the acquired blood sugar level group. The blood sugar level fluctuation trend is, for example, information indicating the slope of the graph drawn using the acquired blood sugar level group.

The blood sugar level fluctuation trend is, for example, a blood sugar level fluctuation speed or a blood sugar level fluctuation acceleration. The blood sugar level fluctuation speed is the speed of blood sugar level fluctuations. The blood sugar level fluctuation acieration is the acceleration of blood sugar level fluctuations.

The fluctuation information acquisition unit 32 acquires the lowest blood sugar level and the highest blood sugar level in the acquired blood sugar level group. In addition, the fluctuation information acquisition unit 32 acquires time information that is paired with the highest blood sugar level and time information that is paired with the lowest blood sugar level. Next, for example, the fluctuation information acquisition unit 32 acquires the blood sugar level fluctuation speed, using an arithmetic formula "(Highest blood sugar level in blood sugar level group - Lowest blood sugar level in blood sugar level group) / Time information paired with highest blood sugar level - Time information paired with lowest blood sugar level)".

For example, the fluctuation information acquisition unit 32 may calculate blood sugar level fluctuation speeds at predetermined time intervals, using the lowest blood sugar level and the highest blood sugar level within a predetermined time period (for example, 5 minutes or 30 minutes) in the acquired blood sugar level group, to acquire two or more blood sugar level fluctuation speeds, and acquire the representative value (for example, the maximum value, the average value, the median value, or the like) of the two or more blood sugar level fluctuation speeds as the blood sugar level fluctuation trend.

For example, the fluctuation information acquisition unit 32 may acquire blood sugar level speed (the slope of the graph) in the acquired blood sugar level group, differentiate the speed to acquire the acceleration, and acquire the representative value (for example, the maximum value, the average value, the median value, or the like) of the acceleration as the blood sugar level fluctuation acceleration.

For example, the fluctuation information acquisition unit 32 may acquire the lowest blood sugar level and the highest blood sugar level in the rising range of the graph constituted by the acquired blood sugar level group, and acquire the speed obtained by dividing the difference between these two blood sugar levels by the time interval between the two as the blood sugar level fluctuation trend.

Note that the blood sugar level fluctuation trend may be information indicating a change level (for example, "rapid", "normal", "blood sugar spike", or the like) acquired using the blood sugar level fluctuation degree, the blood sugar level fluctuation speed, the blood sugar level fluctuation acceleration, or the like.

### (a-3) In the case where fluctuation information is a score

Fluctuation information is, for example, a score indicating the favorability of the blood sugar level fluctuations over time. The fluctuation information acquisition unit 32 acquires fluctuation information that is a score corresponding to blood sugar fluctuations over time, using the blood sugar level group.

For example, the fluctuation information acquisition unit 32 acquires a lower score for a larger difference between the lowest blood sugar level and the highest blood sugar level in (a-1). For example, the fluctuation information acquisition unit 32 calculates a score, using a decreasing function whose parameter is the difference between the lowest blood sugar level and the highest blood sugar level in (a-1). For example, the fluctuation information acquisition unit 32 acquires a score that is paired with the difference between the lowest blood sugar level and the highest blood sugar level in (a-1), from a correspondence table. Note that, in such a case, the correspondence table contains two or more pieces of correspondence information that each include information indicating the range of the difference between the lowest blood sugar level and the highest blood sugar level, and a score.

For example, the fluctuation information acquisition unit 32 acquires a lower score for a higher blood sugar level fluctuation trend in (a-2). For example, the fluctuation information acquisition unit 32 calculates a score, using a decreasing function whose parameter is the blood sugar level fluctuation trend in (a-2). For example, the fluctuation information acquisition unit 32 acquires a score that is paired with the blood sugar level fluctuation trend in (a-2), from a correspondence table. Note that, in such a case, the correspondence table contains two or more pieces of correspondence information that each include information indicating the range of the blood sugar level fluctuation trend, and a score.

### (a-4) In the case where fluctuation information is warning information

Fluctuation information is, for example, warning information. For example, the fluctuation information acquisition unit 32 acquires warning information when judging that the difference between the lowest blood sugar level and the highest blood sugar level in (a-1), the blood sugar level fluctuation trend in (a-2), or the score in (a-3) satisfies a warning condition (the user is not in good health). The warning condition is, for example, that the difference between the lowest blood sugar level and the highest blood sugar level is no less than or greater than a threshold value, the blood sugar level fluctuation trend is no less than or greater than a threshold value, or the score is no less than or greater than a threshold value. Warning information is, for example, a buzzer sound, a string for calling attention, an image for calling attention, or the like. Warning information may be any information as long as it alerts the user.

### (b) In the case of using the acquired blood sugar level group and reference information

For example, the fluctuation information acquisition unit 32 acquires fluctuation information, using a blood sugar level group and reference information. In such a case, fluctuation information is, for example, the result of comparison between the degree of a change in the blood sugar level indicated by reference information and the degree of change in the blood sugar level acquired from the current blood sugar level group.

### (b-1) In the case where fluctuation information is the difference between the blood sugar level group and reference information (a blood sugar level group) under the same condition (for example, "for 2 hours from the start of a meal", "for 1 hour from the start of exercise", or the like)

For example, the fluctuation information acquisition unit 32 acquires a condition (for example, time information or an event identifier) corresponding to the blood sugar level group acquired by the blood sugar level acquisition unit 31. Next, the fluctuation information acquisition unit 32 acquires a blood sugar level group that is reference information that satisfies the condition (for example, reference information that corresponds to the same time information and/or the same event identifier). For example, the fluctuation information acquisition unit 32 acquires information regarding the difference between two blood sugar level groups. Note that the information regarding the difference is, for example, a set of two blood sugar levels included in two blood sugar level groups, and the fluctuation information acquisition unit 32 acquires multiple sets of two blood sugar levels corresponding to the same time information or acquired after the same time period from the start of the event, and acquires the difference between the blood sugar levels for each set. Thereafter, the fluctuation information acquisition unit 32 acquires fluctuation information, using the difference between the two acquired blood sugar levels. In such a case, fluctuation information is, for example, the cumulative value of the difference between the two blood sugar levels, the average value of the difference between the two blood sugar levels, the median value of the difference between the two blood sugar levels, or the maximum value of the difference between the two blood sugar levels.

### (b-2) In the case where fluctuation information is the difference between blood sugar level fluctuation trends under the same condition

For example, as in the processing in (b-1), the fluctuation information acquisition unit 32 acquires the blood sugar level group acquired by the blood sugar level acquisition unit 31 and the blood sugar level group that is reference information that satisfies the condition corresponding to the aforementioned blood sugar level group. Next, the fluctuation information acquisition unit 32 acquires the blood sugar level fluctuation trend of each of the two blood sugar level groups. Next, the fluctuation information acquisition unit 32 acquires fluctuation information that is the difference between the respective blood sugar level fluctuation trends of the two blood sugar level groups.

### (b-3) In the case where fluctuation information is the difference between scores

Fluctuation information is, for example, a score corresponding to reference information. The fluctuation information acquisition unit 32 acquires fluctuation information that is a score corresponding to blood sugar fluctuations over time, using the blood sugar level group and reference information.

The fluctuation information acquisition unit 32 acquires a lower score for a larger value of information that is based on the difference acquired in (b-1) (for example, the cumulative value of the difference between the two blood sugar levels, the average value of the difference between the two blood sugar levels, the median value of the difference between the two blood sugar levels, or the maximum value of the difference between the two blood sugar levels). For example, the fluctuation information acquisition unit 32 calculates a score, using a decreasing function whose parameter is the difference acquired in (b-1). For example, the fluctuation information acquisition unit 32 acquires a score that is paired with the information that is based on the difference acquired in (b-1), from the correspondence table. Note that, in such a case, the correspondence table contains two or more pieces of correspondence information that each include information indicating the range of the information that is based on the difference acquired in (b-1), and a score.

For example, the fluctuation information acquisition unit 32 acquires a lower score for a larger difference between the blood sugar level fluctuation trends in (b-2). For example, the fluctuation information acquisition unit 32 calculates a score, using a decreasing function whose parameter is the difference between the blood sugar level fluctuation trends in (b-2). For example, the fluctuation information acquisition unit 32 acquires a score that is paired with the difference between the blood sugar level fluctuation trends in (b-2), from the correspondence table. Note that, in such a case, the correspondence table contains two or more pieces of correspondence information that each include information indicating the range of the difference between the blood sugar level fluctuation trends, and a score.

For example, the fluctuation information acquisition unit 32 acquires the differences between each blood sugar level in the blood sugar level group acquired in a predetermined period after a meal and the blood sugar level in reference information acquired in a predetermined period after a meal corresponding to the same elapsed time, and acquires a score using the multiple differences. The fluctuation information acquisition unit 32 typically acquires a lower score for a larger difference value.

For example, the fluctuation information acquisition unit 32 acquires the representative value (for example, the average value, the median value, or the maximum value) of the above-described plurality of difference values or the cumulative value of the differences, and acquires a score, using a decreasing function whose parameter is the representative value or the cumulative value of the differences.

For example, the fluctuation information acquisition unit 32 acquires a blood sugar level fluctuation trend from the blood sugar level group acquired in a predetermined period after a meal. Next, for example, the fluctuation information acquisition unit 32 acquires the difference between the blood sugar level fluctuation trend and the blood sugar level fluctuation trend that is reference information, and acquires a lower score for a lager difference. Note that the blood sugar level fluctuation trend is information indicating the degree of fluctuation of the blood sugar level within a unit time.

### (b-4) In the case where fluctuation information is warning information

Fluctuation information is, for example, warning information. For example, the fluctuation information acquisition unit 32 acquires warning information when judging that the difference between the blood sugar levels in (b-1), the difference between the blood sugar level fluctuation trends in (b-2), or the score in (b-3) satisfies a warning condition (the user is not in good health). The warning condition is, for example, the blood sugar level difference relative to the reference information is no less than or greater than a threshold value, the blood sugar level fluctuation trend difference relative to the reference information is no less than or greater than a threshold value, or the score relative to the reference information is no greater than or less than a threshold value.

### (c) In the case of using the acquired blood sugar level group and the past blood sugar level group

For example, the fluctuation information acquisition unit 32 acquires fluctuation information, using the blood sugar level group and the past blood sugar level group. Note that the user for which the blood sugar level group is acquired and the user for which the past blood sugar level group is acquired are the same person.

In such a case, fluctuation information is, for example, the result of comparison between the degree of a change in the blood sugar level acquired from the past blood sugar level group and the degree of change in the blood sugar level acquired from the current blood sugar level group. It is preferable that the past blood sugar level group here is the past blood sugar level group in good health, but may be the past blood sugar level group in average health.

### (c-1) In the case where fluctuation information is the difference between the blood sugar level group and the past blood sugar level group under the same condition

For example, the fluctuation information acquisition unit 32 acquires a condition (for example, time information or an event identifier) corresponding to the blood sugar level group acquired by the blood sugar level acquisition unit 31. Next, the fluctuation information acquisition unit 32 acquires the past blood sugar level group that satisfies the condition (for example, the past blood sugar level group corresponding to the same time information and/or the same event identifier). For example, the fluctuation information acquisition unit 32 acquires information regarding the difference between two blood sugar level groups. Note that the information regarding the difference is, for example, a set of two blood sugar levels included in two blood sugar level groups, and the fluctuation information acquisition unit 32 acquires multiple sets of two blood sugar levels corresponding to the same time information or acquired after the same time period from the start of the event, and acquires the difference between the blood sugar levels for each set. Thereafter, the fluctuation information acquisition unit 32 acquires fluctuation information, using the difference between the two acquired blood sugar levels. In such a case, fluctuation information is, for example, the cumulative value of the difference between the two blood sugar levels, the average value of the difference between the two blood sugar levels, the median value of the difference between the two blood sugar levels, or the maximum value of the difference between the two blood sugar levels.

### (c-2) In the case where fluctuation information is the difference between blood sugar level fluctuation trends under the same condition

For example, as in the processing in (c-1), the fluctuation information acquisition unit 32 acquires the blood sugar level group acquired by the blood sugar level acquisition unit 31 and the past blood sugar level group that satisfies the condition corresponding to the aforementioned blood sugar level group. Next, the fluctuation information acquisition unit 32 acquires the blood sugar level fluctuation trend of the two blood sugar level groups. Next, the fluctuation information acquisition unit 32 acquires fluctuation information that is the difference between the respective blood sugar level fluctuation trends of the two blood sugar level groups.

### (c-3) In the case where fluctuation information is the difference between scores

Fluctuation information is, for example, a score corresponding to the past blood sugar level group. The fluctuation information acquisition unit 32 acquires fluctuation information that is a score corresponding to blood sugar fluctuations over time, using the blood sugar level group and the past blood sugar level group.

The fluctuation information acquisition unit 32 acquires a lower score for a larger value of information that is based on the difference acquired in (c-1) (for example, the cumulative value of the difference between the two blood sugar levels, the average value of the difference between the two blood sugar levels, the median value of the difference between the two blood sugar levels, or the maximum value of the difference between the two blood sugar levels). For example, the fluctuation information acquisition unit 32 calculates a score, using a decreasing function whose parameter is the difference acquired in (c-1). For example, the fluctuation information acquisition unit 32 acquires a score that is paired with the information that is based on the difference acquired in (c-1), from the correspondence table. Note that, in such a case, the correspondence table contains two or more pieces of correspondence information that each include information indicating the range of the information that is based on the difference acquired in (c-1), and a score.

For example, the fluctuation information acquisition unit 32 acquires a lower score for a larger difference between the blood sugar level fluctuation trends in (c-2). For example, the fluctuation information acquisition unit 32 calculates a score, using a decreasing function whose parameter is the difference between the blood sugar level fluctuation trends in (c-2). For example, the fluctuation information acquisition unit 32 acquires a score that is paired with the difference between the blood sugar level fluctuation trends in (c-2), from the correspondence table. Note that, in such a case, the correspondence table contains two or more pieces of correspondence information that each include information indicating the range of the difference between the blood sugar level fluctuation trends, and a score.

For example, the fluctuation information acquisition unit 32 acquires the differences between each blood sugar level in the blood sugar level group acquired in a predetermined period after a meal and the blood sugar level in the past blood sugar level group of the same user acquired in a predetermined period after a meal corresponding to the same elapsed time, and acquires a score using the multiple differences. The fluctuation information acquisition unit 32 typically acquires a lower score for a larger difference value.

### (c-4) In the case where fluctuation information is warning information

Fluctuation information is, for example, warning information. For example, the fluctuation information acquisition unit 32 acquires warning information when judging that the difference between the blood sugar levels in (c-1), the difference between the blood sugar level fluctuation trends in (c-2), or the score in (c-3) satisfies a warning condition (the user is not in good health). The warning condition is, for example, the blood sugar level difference relative to the past blood sugar level group is no less than or greater than a threshold value, the blood sugar level fluctuation trend difference relative to the past blood sugar level group is no less than or greater than a threshold value, or the score relative to the past blood sugar level group is no greater than or less than a threshold value.

The judgment unit 33 judges whether or not the blood sugar level acquired by the blood sugar level acquisition unit 31 satisfies a predetermined output condition, and acquires a judgment result. The predetermined output condition here is a blood sugar level information output condition.

The judgment unit 33 judges whether or not the fluctuation information acquired by the fluctuation information acquisition unit 32 satisfies a predetermined output condition, and acquires a judgment result. The predetermined output condition here is a fluctuation information output condition.

Note that the output conditions may be the same as the warning condition.

The output unit 4 outputs various kinds of information. Examples of the various kinds of information include blood sugar level information, which will be described later, and fluctuation information. Here, the output is a concept that encompasses display on a display screen, projection using a projector, printing by a printer, the output of a sound, transmission to an external device, accumulation on a recording medium, delivery of a processing result to another processing device or another program, and the like.

The blood sugar level information output unit 41 outputs blood sugar level information regarding a blood sugar level. Blood sugar level information may be the blood sugar level itself or warning information indicating that the blood sugar level is not normal. Blood sugar level information may be a string, a graph, or the like, and there is no limitation on the data structure, content, or the like thereof.

The blood sugar level information output unit 41 may constantly output the blood sugar level acquired by the blood sugar level acquisition unit 31.

It is preferable that the blood sugar level information output unit 41 outputs blood sugar level information when the judgement result acquired by the judgment unit 33 indicates that the blood sugar level information output condition is satisfied.

The fluctuation information output unit 42 outputs the fluctuation information acquired by the fluctuation information acquisition unit 32. It is preferable that the fluctuation information output unit 42 outputs fluctuation information when the judgement acquired by the judgment unit 33 indicates that the fluctuation information output condition is satisfied. Note that fluctuation information is, for example, a score, or warning information indicating that fluctuation information is not normal. The fluctuation information output unit 42 may output fluctuation information when the judgment unit 33 does not make a judgment. The fluctuation information output unit 42 may constantly output fluctuation information.

For example, the fluctuation information output unit 42 displays a score. It can be said that a score is blood sugar level information or fluctuation information. For example, the fluctuation information output unit 42 acquires a buzzer sound, which is warning information. Warning information may be a sound, a string, or light, and there is no limitation on the data type thereof.

It is preferable that the storage unit 1, the reference information storage unit 11, and the past blood sugar level group storage unit 12 are non-volatile recording media, but they may be realized using volatile recording media.

There is no limitation on the process in which information is stored in the storage unit 1 or the like. For example, information may be stored in the storage unit 1 or the like via a recording medium, or information transmitted via a communication line or the like may be stored in the storage unit 1 or the like, or information input via an input device may be stored in the storage unit 1 or the like.

The acceptance unit 2 can be realized using a device driver for the input means such as a touch panel or a keyboard, or control software or the like for controlling the menu screen.

The processing unit 3, the fluctuation information acquisition unit 32, and the judgment unit 33 can typically be realized using a processor, a memory, or the like. The processing procedures performed by the processing unit 3 or the like typically are realized using software, and the software is recorded on a recording medium such as a ROM. However, such processing procedures may be realized using hardware (a dedicated circuit). Note that the processor is a CPU, an MPU, a GPU, or the like, and there is no limitation on the type thereof.

It is preferable that th blood sugar level acquisition unit 31 is a non-invasive blood sugar level sensor. It is preferable that th blood sugar level acquisition unit 31 is a non-invasive blood sugar level sensor using visible light, near-infrared light, or the like. One example of such a technology is the technology described on the Internet homepage (URL: https://www.qst.go.jp/site/press/1230.html). In addition, there is a technology for measuring a blood sugar level by simply applying a mid-infrared laser to a finger (Internet homepage (URL: https://dm-net.co.jp/calendar/2017/027218.php)). However, there is no limitation on the means or method used by the blood sugar level acquisition unit 31 to acquire a blood sugar level.

The output unit 4, the blood sugar level information output unit 41, and the fluctuation information output unit 42 may be regarded as including or not including an output device such as a display or a speaker. The output unit 4 or the like can be realized using the driver software of the output device, the driver software of the output device and the output device, or the like. The output unit 4 or the like may be realized using a wireless or wired communication means.

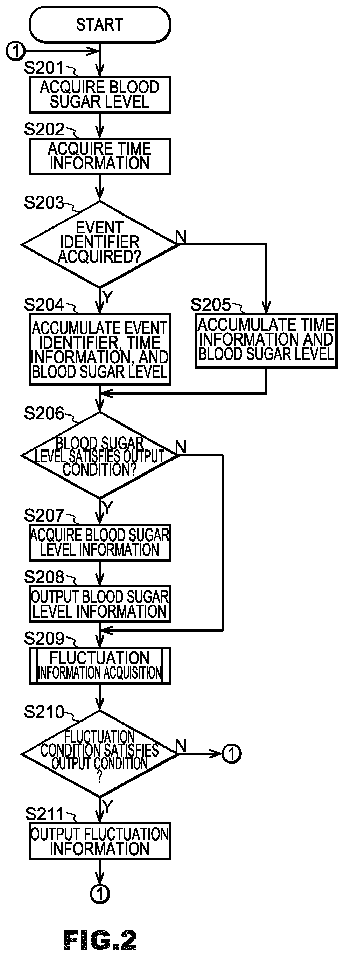

Next, examples of operations of the health support device A will be described with reference to the flowchart in FIG. 2.

(Step S201) The blood sugar level acquisition unit 31 acquires a blood sugar level. Note that there is no limitation on the time the time at which the blood sugar level acquisition unit 31 acquires the blood sugar level, or the trigger therefor. It is preferable that the blood sugar level acquisition unit 31 constantly acquires the blood sugar level at short intervals (for example, 1 second, 5 seconds, etc.).

The blood sugar level acquisition unit 31 may acquire the blood sugar level received by the acceptance unit 2. In such a case, it is preferable that the blood sugar level is associated with the user identifier.

(Step S202) The blood sugar level acquisition unit 31 acquires time information from a clock (not shown).

(Step S203) The acceptance unit 2 judges whether or not an event identifier has been acquired. If an event identifier has been acquired, processing proceeds to step S204, and if an event identifier has not been acquired, processing proceeds to step S205. Note that once an event identifier is acquired, processing may proceed to step S204 for a predetermined period of time (for example, 150 minutes, 2 hours, etc.). Alternatively, once an event identifier is acquired, processing may proceed to step S204 until an instruction to terminate the acquisition of the blood sugar level is accepted. The acceptance unit 2 may acquire an event identifier input from the user, or automatically acquire an event identifier. Note that proceeding to step S204 means that the judgment result in step S203 is "Y".

(Step S204) The blood sugar level acquisition unit 31 accumulates the blood sugar level acquired in step S201 in the storage unit 1 in association with the time information acquired in step S202 and the event identifier acquired in step S203. Processing proceeds to step S206. The blood sugar level acquisition unit 31 may accumulate the blood sugar level or the like in association with the user identifier.

(Step S205) The blood sugar level acquisition unit 31 accumulates the blood sugar level acquired in step S201 in the storage unit 1 in association with the time information acquired in step S202. The blood sugar level acquisition unit 31 may accumulate the blood sugar level or the like in association with the user identifier.

(Step S206) The judgment unit 33 judges whether or not the blood sugar level accumulated in step S204 or step S205 satisfies the blood sugar level information output condition. If the blood sugar level information output condition is satisfied, processing proceeds to step S207, and if the blood sugar level information output condition is not satisfied, processing proceeds to step S209. Here, the processing in this step may be omitted and processing may be proceeds to step S207. Note that the blood sugar level information output condition used here may be blood sugar level information output condition that is paired with the user identifier corresponding to the blood sugar level.

(Step S207) The processing unit 3 acquire blood sugar level information, using the blood sugar level accumulated in step S204 or step S205. Note that examples of blood sugar level information include the blood sugar level itself, the difference between the blood sugar level and reference information, the difference between the blood sugar level and a blood sugar level included in the past blood sugar level group, and a score that is based on the blood sugar level.

(Step S208) The blood sugar level information output unit 41 outputs the blood sugar level information acquired in step S207.

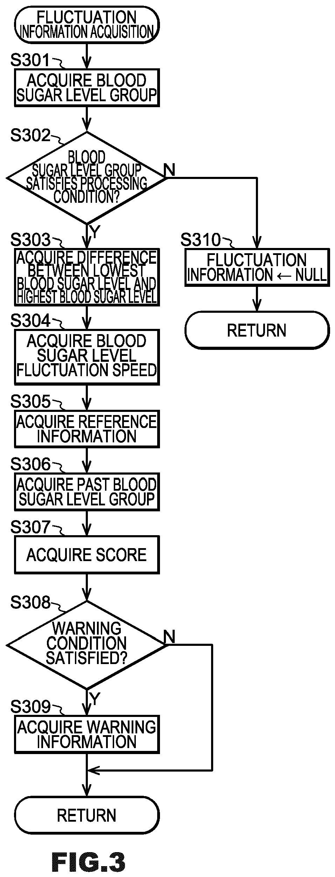

(Step S209) The fluctuation information acquisition unit 32 performs fluctuation information acquisition processing. An example of fluctuation information acquisition processing will be described with reference to the flowchart in FIG. 3.

(Step S210) The judgment unit 33 judges whether or not the fluctuation information acquired in step S209 satisfies the fluctuation information output condition. If the fluctuation information output condition is satisfied, processing proceeds to step S211, and if the fluctuation information output condition is not satisfied, processing returns to step S201. Note that the processing in this step may be omitted and processing may be proceeds to step S211.

(Step S211) The fluctuation information output unit 42 outputs the fluctuation information acquired in step S209. Processing returns to step S201.

Note that step S210 in the flowchart in FIG. 2 may be omitted. That is to say, the judgement processing by the judgment unit 33 may be omitted, and the fluctuation information output unit 42 may output the acquired fluctuation information.

Note that, in the flowchart in FIG. 2, processing ends when the power is turned off or an interruption occurs to end the processing.

Next, an example of the fluctuation information acquisition processing in step S209 will be described with reference to the flowchart in FIG. 3.

(Step S301) The fluctuation information acquisition unit 32 acquires a blood sugar level group that includes two or more blood sugar levels stored in the storage unit 1. Note that the blood sugar level group typically includes the immediately previous blood sugar level acquired. Here, the fluctuation information acquisition unit 32 may acquire a blood sugar level that is paired with the user identifier.

(Step S302) The fluctuation information acquisition unit 32 judges whether or not the blood sugar level group acquired in step S301 satisfies a processing condition. If the processing condition is satisfied, processing proceeds to step S303, and if the processing condition is not satisfied, processing proceeds to step S310. Note that the processing condition is a condition for acquiring fluctuation information. In addition, the processing condition is a condition for judging whether or not the blood sugar level group with which fluctuation information can be acquired has been acquired. The processing condition is, for example, that a predetermined number or more of blood sugar levels have been accumulated in the storage unit 1, that blood sugar levels corresponding to a period no shorter than or longer than a predetermined period have been accumulated in the storage unit 1, or that blood sugar levels corresponding to a period no shorter than or longer than a predetermined period from the occurrence of an event (for example, the start of a meal, the start of exercise, or an insulin injection has been given) have been accumulated in the storage unit 1.

(Step S303) The fluctuation information acquisition unit 32 acquires the lowest blood sugar level and the highest blood sugar level from the blood sugar level group acquired in step S301.

(Step S304) The fluctuation information acquisition unit 32 acquires pieces of time information respectively corresponding to the lowest blood sugar level and the highest blood sugar level acquired in step S303. The fluctuation information acquisition unit 32 calculates a blood sugar level fluctuation trend, using the lowest blood sugar level, the highest blood sugar level, and the two pieces of time information. Note that an example of a blood sugar level fluctuation trend is a blood sugar level fluctuation speed.

(Step S305) The fluctuation information acquisition unit 32 acquires reference information from the reference information storage unit 11.

(Step S306) The fluctuation information acquisition unit 32 acquires a past blood sugar level group from the past blood sugar level group storage unit 12.

(Step S307) The fluctuation information acquisition unit 32 acquires a score, using one or more pieces of information of: the blood sugar level group acquired in step S301; the lowest blood sugar level; the highest blood sugar level; the blood sugar level fluctuation trend; the reference information: and the past blood sugar level group. Note that an example of a score acquisition method is as described above.

(step S308) The judgment unit 33 acquires a warning condition from the storage unit 1. The judgment unit 33 judges whether or not one or more pieces of information of: the blood sugar level group acquired in step S301; the lowest blood sugar level; the highest blood sugar level; the blood sugar level fluctuation trend; the reference information; the past blood sugar level group; and the score acquired in step S307 satisfy the warning condition. If the warning condition is satisfied, processing proceeds to step S309, and if the warning condition is not satisfied, processing returns to higher-level processing. Note that the warning condition may be the output condition. In addition, the judgment unit 33 may acquire a warning condition that is paired with the user identifier, and use the warning condition to make the above-described judgment.

(Step S309) The fluctuation information acquisition unit 32 acquires warning information. Processing returns to higher-level processing. Note that warning information is, for example, one or more pieces of information of: information indicating a situation in which the blood sugar level is inappropriate; the blood sugar level group acquired in step S301; the lowest blood sugar level; the highest blood sugar level; the blood sugar level fluctuation trend; and a score. In addition, warning information is an example of fluctuation information.

(Step S310) The fluctuation information acquisition unit 32 assigns "NULL" to a variable "fluctuation information". Processing returns to higher-level processing.

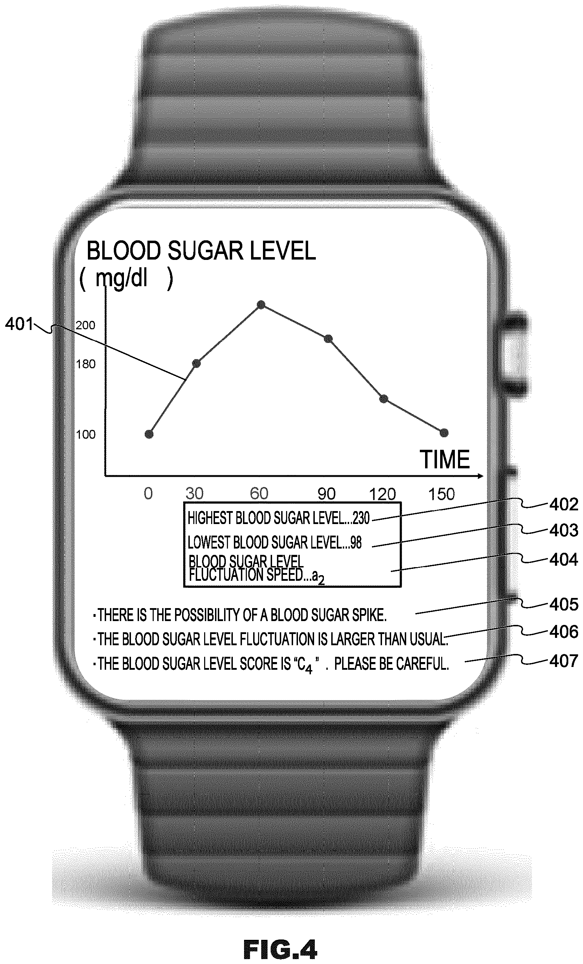

Hereinafter, specific operations of the health support device A according to the present embodiment will be described. An example of the external appearance of the health support device A is shown in FIG. 4. The health support device A is, for example, a device having the shape of a wristwatch.

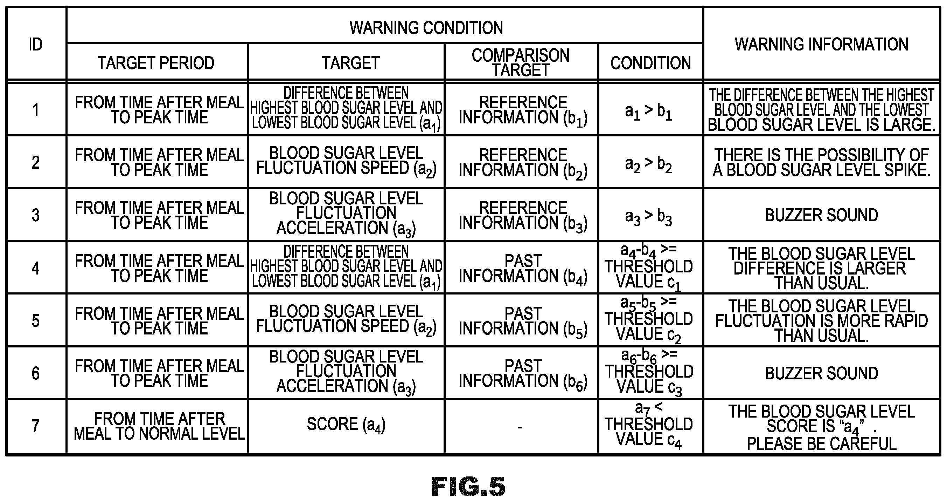

It is assumed that the storage unit 1 in the health support device A stores the warning condition management table shown in FIG. 5. The warning condition management table is a table for managing warning conditions. The warning condition management table contains one or more records that each include "ID", "Warning condition", and "Warning information". The "warning condition" includes "target period", "target", "comparison information", and "condition". "ID" is information identifying the record". "Warning condition" is information defining the warning condition. "Warning information" is warning information that is information to be output when the warning condition is satisfied, or information that is used to form warning information (for example, a warning information template, a program for acquiring warning information, or the like). Warning information typically is information that is output when the blood sugar level is unfavorable for the user. Note that "Warning condition" may also be referred to as an output condition.

"Target period" is information indicating the period in which the blood sugar level included in the blood sugar level group subjected to the judgment regarding whether or not the warning condition is satisfied is acquired, and indicates the period specified by the time information associated with the blood sugar level. "From meal to peak time" is a condition that is satisfied when it is detected that the blood sugar level reaches a peak after the start of a meal. "From meal to normal time" is a condition that is satisfied when it is detected that, after the blood sugar level reaches a peak after the start of a meal, the blood sugar level falls to the blood sugar level at the start of the meal or a value no higher than a threshold value (a normal or ordinary blood sugar level).

"Target" is information specifying the information that is used when the judgment unit 33 performs judgment processing. "Target" here is, for example, the difference between the lowest blood sugar level and the highest blood sugar level (a₁), a blood sugar level fluctuation speed (a₂), a blood sugar level fluctuation acceleration (a₃), or a score (a₄).

"Comparison information" is information regarding the comparison target. "Comparison information" is, for example, reference information (any one of b₁ to b₃), or past information (any one of b₃ to b₆). Past information is information acquired using the past blood sugar level group of the same user as the user for which the blood sugar level group subjected to the judgment was acquired, and is the same type of information as "Target" that is paired with the past information. Past information here is the difference between the lowest blood sugar level and the highest blood sugar level acquired from the past blood sugar level group, a blood sugar level fluctuation speed, or a blood sugar level fluctuation acceleration.

"Condition" is a condition for outputting warning information. Note that threshold values c₁ to c₄ in the condition may be common to all users, or may be set for each user.

In such a situation, it is assumed that the user has entered an event identifier "meal start information" into the health support device A and has started eating. As a result, the acceptance unit 2 of the health support device A accepts the event identifier "meal start information".

Next, the blood sugar level acquisition unit 31 starts acquiring blood sugar levels. For example, the blood sugar level acquisition unit 31 acquires blood sugar levels generally on a regular basis, and accumulates the blood sugar levels in the storage unit 1 in pair with time information. Note that the meal start information here is a relative time from the acceptance of the event identifier "meal start information".

Next, for example, the blood sugar level acquisition unit 31 acquires time information, using a timer (not shown). Thereafter, the blood sugar level acquisition unit 31 accumulates the blood sugar levels in the storage unit 1 in association with the time information and the event identifier "meal start information".

Next, the fluctuation information acquisition unit 32 acquires a blood sugar level group that is a set of blood sugar levels stored in the storage unit 1. Thereafter, the fluctuation information acquisition unit 32 judges whether or not the acquired blood sugar level group satisfies a processing condition. The processing condition here is the "target period" in the warning condition management table in FIG. 5. That is to say, the fluctuation information acquisition unit 32 judges whether or not the blood sugar levels included in the blood sugar level group have reached the peak blood sugar level and have started decreasing, from the acquired blood sugar level group. Here, the processing condition corresponding to the "target period" is not satisfied, and therefore "NULL" is assigned to the variable "fluctuation information". That is to say, fluctuation information is not output.

On the other hand, it is assumed that the blood sugar level information output unit 41 outputs the blood sugar levels acquired by the blood sugar level acquisition unit 31 in chronological order as a graph. An example of such an output is indicated by 401 in FIG. 4. It is also assumed that the blood sugar level information output unit 41 outputs the highest blood sugar level and the lowest blood sugar level after the start of the acquisition of blood sugar levels (402 and 403 in FIG. 4). Note that the output highest blood sugar level and lowest blood sugar level may vary.

It is assumed that, as a result of the health support device A continuously performing the above-described processing, the fluctuation information acquisition unit 32 detects that the blood sugar levels included in the blood sugar level group in the storage unit 1 have reached the peak blood sugar level and have started decreasing. That is to say, for example, after acquiring a blood sugar level lower than the immediately previous blood sugar level (a candidate for the highest blood sugar level), if the fluctuation information acquisition unit 32 detects that only blood sugar levels lower than the candidate for the highest blood sugar level are acquired for a predetermined period, the fluctuation information acquisition unit 32 judges that the time information indicated by the candidate for the highest blood sugar level is the peak time. Note that the method for judging the peak time of the graph is a well-known technique and there is no limitation.

Next, the fluctuation information acquisition unit 32 acquires the difference between the lowest blood sugar level and the highest blood sugar level (a₁), the blood sugar level fluctuation speed (a₂), and the blood sugar level fluctuation acceleration (a₃), which are "targets" paired with the target period "from meal to peak time", from the table in FIG. 5.

Next, for example, the fluctuation information output unit 42 outputs the blood sugar level fluctuation speed (a₂). An example of such an output is indicated by 404 in FIG. 4. Here, the fluctuation information output unit 42 may also output the difference between the lowest blood sugar level and the highest blood sugar level (a₁) and the blood sugar level fluctuation acceleration (a₃). Note that the blood sugar level fluctuation speed (a₂) may vary.

Next, the judgment unit 33 acquires information regarding the comparison targets and conditions indicated by the IDs "1" to "6". Next, the judgment unit 33 judges whether or not each of the warning conditions for the records indicated by the IDs "1" to "6" is satisfied. Here, for example, it is assumed that the judgment unit 33 judges that the warning conditions indicated by the IDs "2" and "5" are satisfied.

Next, the fluctuation information acquisition unit 32 acquires the warning information indicated by the IDs "2" and "5" from the warning condition management table in FIG. 5.

Next, the fluctuation information output unit 42 outputs the pieces of warning information indicated by the IDs "2" and "5". An example of such an output is indicated by 405 and 406 in FIG. 4.

Thereafter, the processing performed by the blood sugar level acquisition unit 31 to acquire and accumulate blood sugar levels, the processing performed by the blood sugar level information output unit 41 to output a blood sugar level graph (401 in FIG. 4), and so on progress.

It is assumed that the fluctuation information acquisition unit 32 detects that the blood sugar levels included in the blood sugar level group in the storage unit 1 have reached the peak, have started decreasing, and have returned to the normal blood sugar level. That is to say, for example, after acquiring the highest blood sugar level, if the fluctuation information acquisition unit 32 detects that the blood sugar level has decreased and the difference from the blood sugar level at the start of the meal is 0 or no higher than a threshold value, the fluctuation information acquisition unit 32 judges that the record matches the target period "from time after meal to normal level". Note that such a judgment method is a well-known technique and there is no limitation.

Next, the fluctuation information acquisition unit 32 acquires the score (a₄) that is the "target" paired with the target period "from time after meal to normal level". Note that the method for acquiring the score (a₄) can be realized using various algorithms as described above, and there is no limitation.

Next, the judgment unit 33 acquires the condition "score (a₄) < threshold value c₄" indicated by the ID "7" from the warning condition management table in FIG. 5. Next, the judgment unit 33 judges whether or not the acquired "score (a₄) satisfies the condition "score (a₄) < threshold value c₄". Here, for example, it is assumed that the judgment unit 33 judges that the condition is satisfied.

Next, the fluctuation information acquisition unit 32 acquires the warning information indicated by the ID "7" from the warning condition management table in FIG. 5. In addition, the fluctuation information acquisition unit 32 substitutes the acquired score for the variable "a₄" in the acquired in warning information (the template for information to be output), to form the warning information to be output.

Next, the fluctuation information output unit 42 outputs the acquired warning information. An example of such an output is indicated by 407 in FIG. 4.

As described above, according to the present embodiment, it is possible to provide the user with the state of the blood sugar level.

For example, according to the present embodiment, when the acquired blood sugar level satisfies an output condition, it is possible to alert the user regarding the blood sugar level by outputting blood sugar level information.

In addition, for example, according to the present embodiment, when fluctuation information regarding blood sugar level fluctuations satisfies a predetermined output condition, it is possible to output the fluctuation information. In this way, for example, it is possible to help eliminate the threat of a "rapid rise in postprandial blood sugar level", which is called a "blood sugar level spike". Note that a "blood sugar level spike" accumulates damage to blood vessels and is considered to be a risk factor for serious diseases such as dementia.

In addition, for example, according to the present embodiment, it is possible to output fluctuation information regarding blood sugar level fluctuations, using reference information and a past blood sugar level group.

In addition, for example, according to the present embodiment, it is possible to output fluctuation information regarding blood sugar level fluctuations, using ordinary and peak blood sugar levels.

In addition, for example, according to the present embodiment, it is possible to output fluctuation information regarding blood sugar level fluctuations, using two or more blood sugar level groups acquired in the same time period, or two or more blood sugar level groups corresponding to the occurrence of the same event.

In addition, for example, according to the present embodiment, it is possible to output a score regarding blood sugar level fluctuations.

In addition, for example, according to the present embodiment, it is possible to provide a warning regarding blood sugar level fluctuations.

Furthermore, for example, according to the present embodiment, it is possible to alert the user regarding the blood sugar level anytime.

Note that the processing in the present embodiment may be realized using software. This software may be distributed through software downloading or the like. Also, this software may be recorded on a recording medium such as a CD-ROM and distributed. Note that the same applies to the other embodiments in the present description. The software that realizes the health support device A according to the present embodiment is the program described below. That is to say, this program is a program for enabling a computer to function as: a blood sugar level acquisition unit that acquires a blood sugar level of a user; a judgment unit that judges whether or not the blood sugar level satisfies a predetermined output condition; and an output unit that outputs blood sugar level information regarding the blood sugar level when the judgment result indicates that the output condition is satisfied.

### Second Embodiment

The present embodiment is different from the first embodiment in that the health support device A functions together with a terminal device 5 used by the user, to realize a health support system B.

That is to say, the health support device A uses a blood sugar level received from the terminal device 5 to perform the processing described in the first embodiment and acquire blood sugar level information or fluctuation information, and transmits the information to the terminal device 5. The terminal device 5 receives and outputs blood sugar level information or fluctuation information.

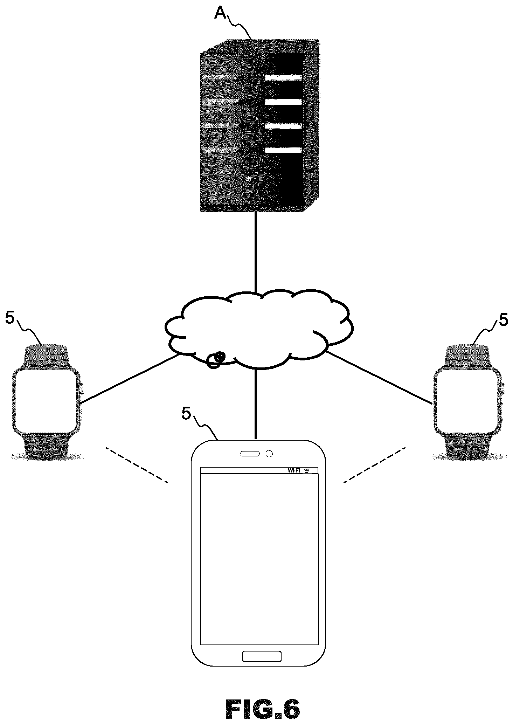

FIG. 6 is a conceptual diagram for the health support system B according to the second embodiment. The health support system B includes one or more health support devices A and one or more terminal devices 5. Although it is preferable that the health support device(s) A and the terminal device(s) 5 are in a one-to-n relationship (where n is 2 or more), they may be in a one-to-one relationship. For example, when the health support device(s) A and the terminal device(s) 5 are in a one-to-one relationship, each health support device A is, for example, a smart phone, a personal computer, a tablet terminal, or the like. Each terminal device 5 is, for example, a smart watch, a smart phone, a personal computer, a table terminal, or the like.

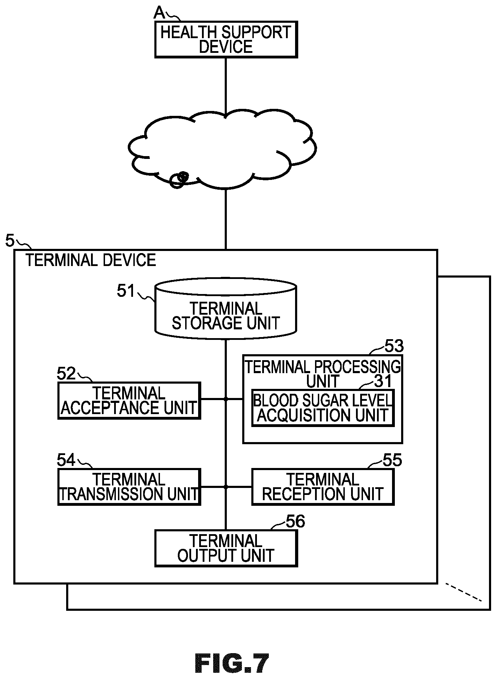

FIG. 7 is a block diagram for the health support system B according to the present embodiment. The terminal device 5 includes a terminal storage unit 51, a terminal acceptance unit 52, a terminal processing unit 53, a terminal transmission unit 54, a terminal reception unit 55, and a terminal output unit 56. The terminal processing unit 53 includes a blood sugar level acquisition unit 31.

The acceptance unit 2 included in the health support device A here receives various kinds of instructions and information from a terminal device 5. Examples of the various kinds of instructions and information include not only a fluctuation information output instruction, a blood sugar level output instruction, and an event identifier, but also a blood sugar level.

The output unit 4 included in the health support device A here transmits various kinds of information to a terminal device 5. Examples of the various kinds of information include blood sugar level information and fluctuation information. Note that the output unit 4 may display various kinds of information or output audio.

The terminal storage unit 51 included in each terminal device 5 stores various kinds of information. Examples of the various kinds of information include a user identifier, a blood sugar level, information including a pair consisting of a blood sugar level and time information, a blood sugar level group, and a group of pairs each consisting of a blood sugar level and time information.

It is preferable that the terminal storage unit 51 is a non-volatile recording medium, but may be realized using a volatile recording medium.

There is no limitation on the process in which information is stored in the terminal storage unit 51. For example, information may be stored in the storage unit 51 via a recording medium, or information transmitted via a communication line or the like may be stored in the storage unit 51, or information input via an input device may be stored in the storage unit 51.

The terminal acceptance unit 52 accepts various kinds of instructions and information. Examples of the various kinds of instructions and information include a fluctuation information output instruction a blood sugar level output instruction, and an event identifier.

The means for inputting various kinds of instructions and information may be any means such as a touch panel, a keyboard, a mouse, a means that employs a menu screen, or the like. The terminal acceptance unit 52 can be realized using a device driver for the input means such as a touch panel or a keyboard, or control software or the like for controlling the menu screen.

The processing unit 53 performs various kinds of processing. Examples of the various kinds of processing include the processing that is performed by the blood sugar level acquisition unit 31.

For example, the terminal processing unit 53 adds the user identifier of the terminal storage unit 51 to the various kinds of instructions and information accepted by the terminal acceptance unit 52, to form various kinds of instructions and information to be transmitted.

The terminal processing unit 53 may typically be realized using a processor, a memory, and so on. The processing procedures performed by the processing unit 13 or the like typically are realized using software, and the software is recorded on a recording medium such as a ROM. However, such processing procedures may be realized using hardware (a dedicated circuit). Note that the processor is a CPU, an MPU, a GPU, or the like, and there is no limitation on the type thereof.

The terminal transmission unit 54 transmits various kinds of instructions and information to the health support device A. Examples of the various kinds of instructions and information include a fluctuation information output instruction, a blood sugar level output instruction, an event identifier, a blood sugar level, information including a pair consisting of a blood sugar level and time information, a blood sugar level group, and a group of pairs each consisting of a blood sugar level and time information. It is preferable that the terminal transmission unit 54 transmits various kinds of instructions and information in association with the user identifier.

The terminal transmission unit 54 may typically be realized using a wireless or wired communication means, but may be realized using a broadcast means.

The terminal reception unit 55 receives various kinds of information from the health support device A. Examples of the various kinds of information include blood sugar level information and fluctuation information.

The terminal reception unit 55 may typically be realized using a wireless or wired communication means, but may be realized using a broadcast receiving means.

The terminal output unit 56 outputs various kinds of information. Examples of the various kinds of information include blood sugar level information and fluctuation information.

The terminal output unit 56 may be regarded as including or not including an output device such as a display or a speaker. The terminal output unit 56 can be realized using the driver software of the output device, the driver software of the output device and the output device, or the like.

Next, examples of operations of a terminal device 5 included in the health support system B will be described. The terminal acceptance unit 52 accepts an event identifier. Next, the terminal processing unit 53 reads out a user identifier in the terminal storage unit 51, and forms information that includes the user identifier and the event identifier. Next, the terminal transmission unit 54 transmits the information to the health support device A. Note that, through such processing, the health support device A accumulates the user identifier, the event identifier, and time information in association with each other.

Next, the blood sugar level acquisition unit 31 included in the terminal processing unit 53 acquires a blood sugar level. The terminal processing unit 53 reads out a user identifier in the terminal storage unit 51, and forms information that includes the user identifier and a blood sugar level. Next, the terminal transmission unit 54 transmits the information to the health support device A. Note that, through such processing, the health support device A accumulates the user identifier, the event identifier, the time information, and the blood sugar level in association with each other.

The terminal device 5 continues processing to transmit information that includes the user identifier and the blood sugar level. As a result, the terminal reception unit 55 receives blood sugar level information or fluctuation information from the health support device A. Next, the terminal output unit 56 outputs the received blood sugar level information or fluctuation information. An example output of the result of the above-described processing is shown in FIG. 4.

As described above, according to the present embodiment, it is possible to provide each of one or more users with the state of the blood sugar level thereof. Note that the state of a blood sugar level is represented as the various kinds of information described in the first embodiment.

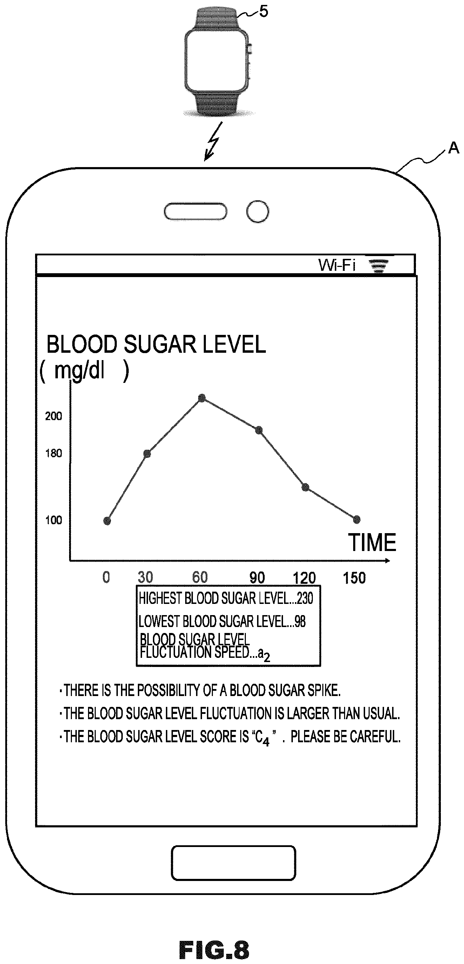

Note that, in the present embodiment, when the health support device(s) A and the terminal device(s) 5 are in a one-to-one relationship, each terminal device 5 performs processing to only acquire a blood sugar level and transmit it to a health support device A, for example. In such a case, the terminal reception unit 55 and the terminal output unit 56 may be omitted from each terminal device 5. In such a case, each health support device A is, for example, a smart phone, a personal computer, a server, or the like. The server is a so-called cloud server, an ASP server, or the like, and there is no limitation. In addition, when a health support device A is a smart phone or the like, the above-described processing is realized by an application in the smart phone or the like. A conceptual diagram for such a case is shown in FIG. 8. In FIG. 8, the communication means between the health support device A and the terminal device 5 may be a distance wireless communication such as Bluetooth (registered trademark), communication via the Internet, or the like, and there is no limitation on the communication means.

### Third Embodiment

The present embodiment describes a health support device that acquires blood sugar level-related information regarding the blood sugar level of one user, and provides the user with suggestion information when the blood sugar level-related information satisfies a suggestion condition. Note that blood sugar level information is, for example, a blood sugar level itself, or fluctuation information, which will be described later. Providing the user with suggestion information means, for example, transmitting the suggestion information to the terminal device of the user, and displaying the suggestion information on the display. Suggestion information is, for example, foods to be ingested, advice on improving lifestyle habits, advice on exercise, and advice on meals. Furthermore, it is preferable that the health support device outputs suggestion information immediately after acquiring the blood sugar level.

In addition, the present embodiment describes a health support device that acquires fluctuation information regarding blood sugar level fluctuations, using two or more blood sugar levels of the user acquired in a time series, and provides the user with suggestion information, using the fluctuation information. Note that fluctuation information is, for example, a score, or the amount of fluctuation in a blood sugar level within a unit time.

In addition, the present embodiment describes a health support device that acquires fluctuation information based on a comparison with reference information.

In addition, the present embodiment describes a health support device that acquires fluctuation information based on a comparison with a set of blood sugar levels of the user acquired in the past.

In addition, the present embodiment describes a health support device that judges whether or not to provide suggestion information, using other information as well.

In addition, the present embodiment describes a health support device that acquires suggestion information, using other information as well. It is preferable that the health support device acquire different suggestion information according to other information even if blood sugar level information or fluctuation information is the same.

In addition, the present embodiment describes a health support device that acquires fluctuation information regarding a blood sugar level, using a blood sugar level group that is a set of blood sugar levels that are consecutive in a time series. Note that such a blood sugar level group is, for example, a blood sugar level group that is a set of blood sugar levels acquired in a period between an ordinary time and a peak, or a blood sugar level group that is a set of blood sugar levels acquired in a period from an ordinary time to a peak and a period from the peak to an ordinary time.

Furthermore, the present embodiment describes a health support device that acquires fluctuation information, using two or more blood sugar level groups acquired at different times. Note that the two or more blood sugar level groups acquired at different times are two or more blood sugar level groups acquired in the same time period, or two or more blood sugar level groups corresponding to the occurrence of the same event such as a meal, sleep, or exercise.

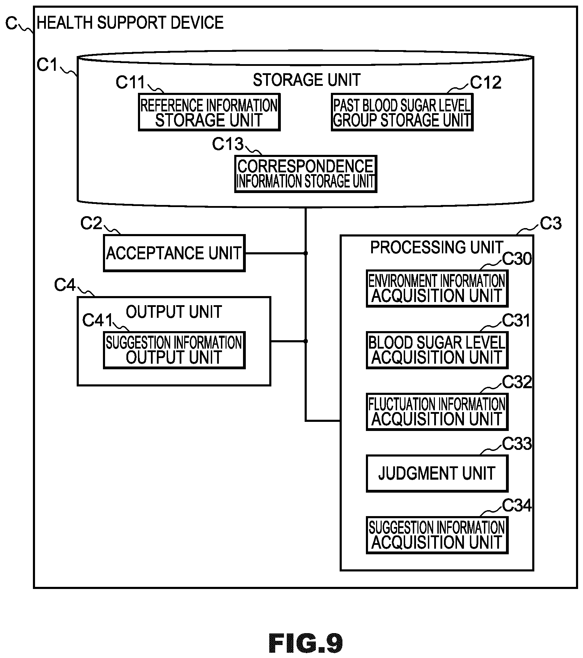

FIG. 9 is a block diagram for a health support device C according to the present embodiment. The health support device C includes a storage unit C1, an acceptance unit C2, a processing unit C3, and an output unit C4.

The storage unit C1 includes a reference information storage unit C11, a past blood sugar level group storage unit C12, and a correspondence information storage unit C13. The processing unit C3 includes an environment information acquisition unit C30, a blood sugar level acquisition unit C31, a fluctuation information acquisition unit C32, a judgment unit C33, and suggestion information acquisition unit C34. The output unit C4 includes a suggestion information output unit C41.

The storage unit C1 stores various kinds of information. Examples of the various kinds of information include reference information, which will be described later, a past blood sugar level group, which will be described later, one or more suggestion conditions, one or more pieces of suggestion information, and a processing condition, which will be described later. The suggestion conditions may be common to all users or may be associated with user identifiers. Each of the one or more pieces of suggestion information stored in the storage unit C1 is associated with a suggestion condition, for example, but may not be associated with a suggestion condition.

The reference information storage unit C11 stores reference information regarding a refence blood sugar level. Reference information is, for example, associated with information specifying timing. Examples of reference information include the ordinary blood sugar level, the representative value of the peak blood sugar level, the representative value of the difference between the ordinary blood sugar level and the peak blood sugar level, and the representative value of the time interval between the time corresponding to the ordinary blood sugar level and the time corresponding to the peak blood sugar level. Note that each representative value is, for example, an average value, a median value, or the like of two or more values (a blood sugar level, a difference, a time interval, etc.). Each representative value may be a reference normal value. Reference information may be, for example, information indicating a normal blood sugar level range, information indicating a normal blood sugar level fluctuation range, or information indicating a normal blood sugar level fluctuation trend range.

Reference information is, for example, stored in association with a user identifier. The user identifier is information identifying a user. The user identifier may be, for example, an ID, an email address, a telephone number, a name, a terminal identifier of the user's terminal device, or the like, and there is no limitation. The terminal identifier is, for example, an IP address, a MAC address, or the like, and there is no limitation.

The past blood sugar level group storage unit C12 stores a past blood sugar level group constituted by two or more past blood sugar levels. A past blood sugar level is a blood sugar level in the past of one user. Each of the blood sugar levels included in the past blood sugar level group is associated with time information regarding the time at which the blood sugar level was acquired. The past blood sugar level group is, for example, stored in association with a user identifier. The past blood sugar level group is a set of blood sugar levels in the past acquired when the user is in good health. The past blood sugar level group is information acquired from a set of blood sugar levels for each day of two or more days, and is a set of representative values (for example, average values, median values, or maximum values) of the blood sugar levels acquired at the same time of day. The same time of day is, for example, the same time after the start of a meal, the same time after the start of exercise, or the same time after sleep. Note that the time information is, for example, the time, but may be information specifying a relative time with respect to a certain point in time. The certain point in time is, for example, the point in time when an event identifier, which will be described later, was acquired.

The correspondence information storage unit C13 stores two or more pieces of correspondence information. The correspondence information here is, for example, information indicating a correspondence between suggestion conditions and suggestion information. The correspondence information here is, for example, information indicating a correspondence between suggestion conditions and suggestion information. The correspondence information here is, for example, information that includes suggestion conditions and suggestion information. The correspondence information here is, for example, information that includes suggestion conditions and suggestion basis information. Suggestion basis information is information that is the basis of the suggestion information. Suggestion basis information is, for example, a template for suggestion information having a variable. The variable is a variable to which blood sugar level-related information is assigned. Examples of the blood sugar level-related information include a blood sugar level and fluctuation information.

The suggestion conditions are conditions regarding blood sugar level-related information. The suggestion conditions may be conditions that include information regarding other information. Other information is, for example, environmental information and user dynamic attribute information. Environmental information is, for example, time, weather, and location information. User dynamic attribute information is, for example, information indicating that the user is eating or information indicating that the user is exercising.

Suggestion information includes, for example, information specifying advice on foods to be ingested, information specifying advice on lifestyle habits, information specifying advice on exercise, or information specifying advice on diet.

For example, suggestion information may be associated with other information. That is to say, even if the blood sugar level-related information is the same, different suggestion information may be acquired according to other information.

There is no limitation of the data type of the suggestion information or the suggestion basis information. The data type of the suggestion information or the suggestion basis information is, for example, a string, an image, a movie, audio, or a combination of two or more of them.

The acceptance unit C2 accepts various kinds of instructions and information. Examples of the various kinds of instructions and information include a suggestion output instruction and an event identifier.

The suggestion output instruction is an instruction to output suggestion information. Note that the suggestion output instruction includes, for example, the blood sugar level acquired by the blood sugar level acquisition unit C31.

The event identifier is information identifying an event. Examples of the event identifier include meal start information, in-meal information, meal end information, exercise start information, in-exercise information, exercise end information, insulin injection information, and bedtime information.

Meal start information is information indicating that a meal has started, in-meal information is information indicating that a meal is in progress, and meal end information is information indicating that a meal has ended. Exercise start information is information indicating that exercise has started, in-exercise information is information indicating that exercise is in progress, and exercise end information is information indicating that exercise has ended. Insulin injection information is information indicating that an insulin injection has been given. Bedtime information is information indicating that the user is about to go to bed, or information indicating that the user is currently asleep.

The acceptance unit C2 accepts an event identifier from a user, for example. Note that the acceptance unit C2 may automatically acquire an event identifier, for example. For example, when the temperature acquired by the temperature sensor of the health support device C rises by a predetermined degree or more, and when the gyro sensor or acceleration sensor of the health support device C detects that the device is not moving, the acceptance unit C2 acquires an event identifier that is meal start information. When the start of movement is detected by the gyro sensor or acceleration sensor of the health support device C, the acceptance unit C2 acquires an event identifier that is exercise start information. The acceptance unit C2 may, for example, automatically detect that the user is asleep, based on the sensing results of a heart rate monitor, a temperature sensor, or the like. Note that there are various possible methods for detecting eating, detecting exercise and detecting sleep, which are well-known techniques, and therefore detailed description thereof will be omitted.

The means used by the user to input various kinds of instructions and information may be any means such as a touch panel, a keyboard, a mouse, a means that employs a menu screen, or the like.

The processing unit C3 performs various kinds of processing. Examples of the various kinds of processing include the processing that is performed by the environment information acquisition unit C30, the blood sugar level acquisition unit C31, the fluctuation information acquisition unit C32, the judgment unit C33, and the suggestion information acquisition unit C34.

The processing unit C3 acquires blood sugar level information, using the blood sugar level acquired by the blood sugar level acquisition unit C31. Examples of blood sugar level information include the blood sugar level itself, the difference between the blood sugar level and reference information, the difference between the blood sugar level and a blood sugar level included in the past blood sugar level group, and a score that is based on the blood sugar level.

For example, the processing unit C3 calculates the difference between the blood sugar level and reference information. For example, the processing unit C3 calculates the difference between the blood sugar level and a blood sugar level that was acquired under the same condition as the condition corresponding to the blood sugar level, and is included in the past blood sugar level group. For example, the same condition means that the blood sugar level is associated with the same time information, or the blood sugar level is associated with the same event identifier and the time elapsed from the occurrence of the event is associated with approximately the same time, or the like.

For example, the processing unit C3 acquires a score, using the blood sugar level, or the difference between the blood sugar level and reference information, or the difference between the blood sugar level and a blood sugar level included in the past blood sugar level group. For example, the processing unit C3 acquires a lower score for a larger difference between the blood sugar level and reference information. For example, the processing unit C3 acquires a lower score for a larger difference between the blood sugar level and the blood sugar level included in the past blood sugar level group.

The environment information acquisition unit C30 acquires environment information regarding user environment. Environmental information is, for example, time, season, weather, and location information. Environmental information is an example of other information, which will be described later.

For example, the environment information acquisition unit C30 acquires the time (or date and time) from a clock (not shown). For example, the environmental information acquisition unit C30 acquires the date and time from a clock (not shown), and uses the date and time to acquire the season (for example, spring, summer, autumn, winter, or year-end). The technique for acquiring the season using the date and time is a well-known technique. For example, the season can be acquired using a correspondence table including a relationship between the date and time range and the season. For example, the environment information acquisition unit C30 acquires the weather from an external server (not shown). For example, the environment information acquisition unit C30 acquires location information, and uses the location information to acquire the weather at the location indicated by the location information from an external server (not shown). For example, the environment information acquisition unit C30 acquires location information using, for example, a GPS receiver.

The blood sugar level acquisition unit C31 acquires blood sugar levels of one user. For example, the blood sugar level acquisition unit C31 acquires blood sugar levels generally on a regular basis. It is preferable that the blood sugar level acquisition unit C31 acquires blood sugar levels in combination with time information. For example, the blood sugar level acquisition unit C31 acquires time information from a clock or a timer (not shown), but may acquire time information in any manner. Note that any method or device may be used by the blood sugar level acquisition unit C31 to acquire the blood sugar level. In addition, there is no limitation on the time at which the blood sugar level acquisition unit C31 acquires the blood sugar level.

For example, the blood sugar level acquisition unit C31 acquires a blood sugar level group constituted by two or more blood sugar levels of the user acquired in a time series on one day. It is preferable that the blood sugar level acquisition unit C31 acquires two or more blood sugar level groups of the user in different time periods. Note that the different time periods are, for example, different days, morning and afternoon, a predetermined time before or after breakfast, a predetermined time before or after lunch, or a predetermined time before or after dinner. It is preferable that the interval between the times at which two or more blood sugar levels are acquired in the time series is short, such as every second, but the time interval is not limited.

For example, the blood sugar level acquisition unit C31 may acquire a blood sugar level group in a section that satisfies a predetermined condition from the blood sugar level group that includes the two or more acquired blood sugar levels. That is to say, the blood sugar level acquisition unit C31 acquires ordinary blood sugar levels whose fluctuation value is no higher than a threshold value from the acquired blood sugar level group. In addition, the blood sugar level acquisition unit C31 acquires the maximum blood sugar level in the blood sugar level group. The blood sugar level acquisition unit C31 may acquire a blood sugar level group that includes two or more blood sugar levels from the ordinary blood sugar level to the peak blood sugar level.

In addition, the blood sugar level acquisition unit C31 may further acquire a blood sugar level group that include blood sugar levels from the blood sugar level acquired at the time after the time corresponding to the peak blood sugar level and closest to the time, to the ordinary blood sugar level. That is to say, the blood sugar level group is, for example, a blood sugar level group that includes two or more blood sugar levels from the ordinary blood sugar level to the peak blood sugar level, or a blood sugar level group that includes three or more blood sugar levels from the ordinary blood sugar level to the peak blood sugar level and from the peak blood sugar level to the ordinary blood sugar level.

For example, the blood sugar level acquisition unit C31 may acquire two or more blood sugar level groups in the same time period (for example, two hours from 7:00 including breakfast time) from blood sugar level groups respectively corresponding to different days. In addition, for example, the blood sugar level acquisition unit C31 may acquire two or more blood sugar level groups corresponding to the occurrence of the same event from blood sugar level groups respectively corresponding to different days. A blood sugar level group corresponding to the occurrence of the same event is usually a set of blood sugar levels paired with the same event identifier. That is to say, two or more blood sugar level groups are, for example, two or more blood sugar level groups acquired in the same time period on different days, or two or more blood sugar level groups corresponding to the occurrence of the same event. The event is, for example, a meal, an exercise, having an insulin injection, or the like. Note that two or more blood sugar level groups corresponding to the occurrence of the same event are two or more blood sugar level groups corresponding to the same event identifier.

Note that, for example, the blood sugar level acquisition unit C31 acquires a blood sugar level acquired after the acceptance unit C2 accepts an event identifier, in association with the event identifier. In addition, the blood sugar level acquisition unit C31 accumulates the blood sugar level in a buffer (not shown) or the storage unit C1, in association with the event identifier.

The fluctuation information acquisition unit C32 acquires fluctuation information, using the blood sugar level group acquired by the blood sugar level acquisition unit C31. Fluctuation information is information regarding blood sugar level fluctuations over time.

Information that is used by the fluctuation information acquisition unit C32 to acquire fluctuation information can be (a) to (c) below, for example.

### (a) In the case of only using the acquired blood sugar level group

For example, the fluctuation information acquisition unit C32 acquires fluctuation information, only using the blood sugar level group acquired by the blood sugar level acquisition unit C31. In the case of only using the blood sugar level group acquired by the blood sugar level acquisition unit C31, the fluctuation information to be acquired can be (a-1) to (a-3) below, for example.

### (a-1) In the case where fluctuation information is the difference between the lowest blood sugar level and the highest blood sugar level

Fluctuation information is, for example, the difference between the lowest blood sugar level and the highest blood sugar level in the blood sugar level group. For example, the fluctuation information acquisition unit C32 acquires the lowest blood sugar level and the highest blood sugar level in the acquired blood sugar level group, calculates the difference between the lowest blood sugar level and the highest blood sugar level, and acquires the difference as fluctuation information. Note that the difference between the lowest blood sugar level and the highest blood sugar level in the blood sugar level group may be referred to as a blood sugar level fluctuation degree.

### (a-2) In the case where fluctuation information is a blood sugar level fluctuation trend

Fluctuation information is, for example, a blood sugar level fluctuation trend. The blood sugar level fluctuation trend is information indicating the trend of the blood sugar level fluctuations. The blood sugar level fluctuation trend is, for example, calculated using the lowest blood sugar level and the highest blood sugar level in the acquired blood sugar level group. The blood sugar level fluctuation trend is, for example, calculated using the lowest blood sugar level and the highest blood sugar level acquired within a predetermined time (for example, 5 minutes or 30 minutes) in the acquired blood sugar level group. The blood sugar level fluctuation trend is, for example, information indicating the slope of the graph drawn using the acquired blood sugar level group.

The blood sugar level fluctuation trend is, for example, a blood sugar level fluctuation speed or a blood sugar level fluctuation acceleration. The blood sugar level fluctuation speed is the speed of blood sugar level fluctuations. The blood sugar level fluctuation acieration is the acceleration of blood sugar level fluctuations.

The fluctuation information acquisition unit C32 acquires the lowest blood sugar level and the highest blood sugar level in the acquired blood sugar level group. In addition, the fluctuation information acquisition unit C32 acquires time information that is paired with the highest blood sugar level and time information that is paired with the lowest blood sugar level. Next, for example, the fluctuation information acquisition unit C32 acquires the blood sugar level fluctuation speed, using an arithmetic formula "(Highest blood sugar level in blood sugar level group - Lowest blood sugar level in blood sugar level group) / Time information paired with highest blood sugar level - Time information paired with lowest blood sugar level)".

For example, the fluctuation information acquisition unit C32 may calculate blood sugar level fluctuation speeds at predetermined time intervals, using the lowest blood sugar level and the highest blood sugar level within a predetermined time period (for example, 5 minutes or 30 minutes) in the acquired blood sugar level group, to acquire two or more blood sugar level fluctuation speeds, and acquire the representative value (for example, the maximum value, the average value, the median value, or the like) of the two or more blood sugar level fluctuation speeds as the blood sugar level fluctuation trend.

For example, the fluctuation information acquisition unit C32 may acquire blood sugar level speed (the slope of the graph) in the acquired blood sugar level group, differentiate the speed to acquire the acceleration, and acquire the representative value (for example, the maximum value, the average value, the median value, or the like) of the acceleration as the blood sugar level fluctuation acceleration.

For example, the fluctuation information acquisition unit C32 may acquire the lowest blood sugar level and the highest blood sugar level in the rising range of the graph constituted by the acquired blood sugar level group, and acquire the speed obtained by dividing the difference between these two blood sugar levels by the time interval between the two as the blood sugar level fluctuation trend.

Note that the blood sugar level fluctuation trend may be information indicating a change level (for example, "rapid", "normal", "blood sugar spike", or the like) acquired using the blood sugar level fluctuation degree, the blood sugar level fluctuation speed, the blood sugar level fluctuation acceleration, or the like.

### (a-3) In the case where fluctuation information is a score

Fluctuation information is, for example, a score indicating the favorability of the blood sugar level fluctuations over time. The fluctuation information acquisition unit C32 acquires fluctuation information that is a score corresponding to blood sugar fluctuations over time, using the blood sugar level group.

For example, the fluctuation information acquisition unit C32 acquires a lower score for a larger difference between the lowest blood sugar level and the highest blood sugar level in (a-1). For example, the fluctuation information acquisition unit C32 calculates a score, using a decreasing function whose parameter is the difference between the lowest blood sugar level and the highest blood sugar level in (a-1). For example, the fluctuation information acquisition unit C32 acquires a score that is paired with the difference between the lowest blood sugar level and the highest blood sugar level in (a-1), from a correspondence table. Note that, in such a case, the correspondence table contains two or more pieces of correspondence information that each include information indicating the range of the difference between the lowest blood sugar level and the highest blood sugar level, and a score.

For example, the fluctuation information acquisition unit C32 acquires a lower score for a higher blood sugar level fluctuation trend in (a-2). For example, the fluctuation information acquisition unit C32 calculates a score, using a decreasing function whose parameter is the blood sugar level fluctuation trend in (a-2). For example, the fluctuation information acquisition unit C32 acquires a score that is paired with the blood sugar level fluctuation trend in (a-2), from a correspondence table. Note that, in such a case, the correspondence table contains two or more pieces of correspondence information that each include information indicating the range of the blood sugar level fluctuation trend, and a score.

### (b) In the case of using the acquired blood sugar level group and reference information

For example, the fluctuation information acquisition unit C32 acquires fluctuation information, using the blood sugar level group and the past blood sugar level group. In such a case, fluctuation information is, for example, the result of comparison between the degree of a change in the blood sugar level indicated by reference information and the degree of change in the blood sugar level acquired from the current blood sugar level group.

### (b-1) In the case where fluctuation information is the difference between the blood sugar level group and reference information (a blood sugar level group) under the same condition (for example, "for 2 hours from the start of a meal", "for 1 hour from the start of exercise", or the like)

For example, the fluctuation information acquisition unit C32 acquires a condition (for example, time information or an event identifier) corresponding to the blood sugar level group acquired by the blood sugar level acquisition unit C31. Next, the fluctuation information acquisition unit C32 acquires a blood sugar level group that is reference information that satisfies the condition (for example, reference information that corresponds to the same time information and/or the same event identifier). For example, the fluctuation information acquisition unit C32 acquires information regarding the difference between two blood sugar level groups. Note that the information regarding the difference is, for example, a set of two blood sugar levels included in two blood sugar level groups, and the fluctuation information acquisition unit C32 acquires multiple sets of two blood sugar levels corresponding to the same time information or acquired after the same time period from the start of the event, and acquires the difference between the blood sugar levels for each set. Thereafter, the fluctuation information acquisition unit C32 acquires fluctuation information, using the difference between the two acquired blood sugar levels. In such a case, fluctuation information is, for example, the cumulative value of the difference between the two blood sugar levels, the average value of the difference between the two blood sugar levels, the median value of the difference between the two blood sugar levels, or the maximum value of the difference between the two blood sugar levels.

### (b-2) In the case where fluctuation information is the difference between blood sugar level fluctuation trends under the same condition

For example, as in the processing in (b-1), the fluctuation information acquisition unit C32 acquires the blood sugar level group acquired by the blood sugar level acquisition unit C31 and the blood sugar level group that is reference information that satisfies the condition corresponding to the aforementioned blood sugar level group. Next, the fluctuation information acquisition unit C32 acquires the blood sugar level fluctuation trend of the two blood sugar level groups. Next, the fluctuation information acquisition unit C32 acquires fluctuation information that is the difference between the respective blood sugar level fluctuation trends of the two blood sugar level groups.

### (b-3) In the case where fluctuation information is the difference between scores

Fluctuation information is, for example, a score corresponding to reference information. The fluctuation information acquisition unit C32 acquires fluctuation information that is a score corresponding to blood sugar fluctuations over time, using the blood sugar level group and reference information.

The fluctuation information acquisition unit C32 acquires a lower score for a larger value of information that is based on the difference acquired in (b-1) (for example, the cumulative value of the difference between the two blood sugar levels, the average value of the difference between the two blood sugar levels, the median value of the difference between the two blood sugar levels, or the maximum value of the difference between the two blood sugar levels). For example, the fluctuation information acquisition unit C32 calculates a score, using a decreasing function whose parameter is the difference acquired in (b-1). For example, the fluctuation information acquisition unit C32 acquires a score that is paired with the information that is based on the difference acquired in (b-1), from the correspondence table. Note that, in such a case, the correspondence table contains two or more pieces of correspondence information that each include information indicating the range of the information that is based on the difference acquired in (b-1), and a score.

For example, the fluctuation information acquisition unit C32 acquires a lower score for a larger difference between the blood sugar level fluctuation trends in (b-2). For example, the fluctuation information acquisition unit C32 calculates a score, using a decreasing function whose parameter is the difference between the blood sugar level fluctuation trends in (b-2). For example, the fluctuation information acquisition unit C32 acquires a score that is paired with the difference between the blood sugar level fluctuation trends in (b-2), from the correspondence table. Note that, in such a case, the correspondence table contains two or more pieces of correspondence information that each include information indicating the range of the difference between the blood sugar level fluctuation trends, and a score.

For example, the fluctuation information acquisition unit C32 acquires the differences between each blood sugar level in the blood sugar level group acquired in a predetermined period after a meal and the blood sugar level in reference information acquired in a predetermined period after a meal corresponding to the same elapsed time, and acquires a score using the multiple differences. The fluctuation information acquisition unit C32 typically acquires a lower score for a larger difference value.

For example, the fluctuation information acquisition unit C32 acquires the representative value (for example, the average value, the median value, or the maximum value) of the above-described plurality of difference values or the cumulative value of the differences, and acquires a score, using a decreasing function whose parameter is the representative value or the cumulative value of the differences.

For example, the fluctuation information acquisition unit C32 acquires a blood sugar level fluctuation trend from the blood sugar level group acquired in a predetermined period after a meal. Next, for example, the fluctuation information acquisition unit C32 acquires the difference between the blood sugar level fluctuation trend and the blood sugar level fluctuation trend that is reference information, and acquires a lower score for a lager difference. Note that the blood sugar level fluctuation trend typically is information indicating the degree of fluctuation of the blood sugar level within a unit time.

### (c) In the case of using the acquired blood sugar level group and the past blood sugar level group

For example, the fluctuation information acquisition unit C32 acquires fluctuation information, using the blood sugar level group and the past blood sugar level group. Note that the user for which the blood sugar level group is acquired and the user for which the past blood sugar level group is acquired are the same person.

In such a case, fluctuation information is, for example, the result of comparison between the degree of a change in the blood sugar level acquired from the past blood sugar level group and the degree of change in the blood sugar level acquired from the current blood sugar level group. It is preferable that the past blood sugar level group here is the past blood sugar level group in good health, but may be the past blood sugar level group in average health.

### (c-1) In the case where fluctuation information is the difference between the blood sugar level group and the past blood sugar level group under the same condition

For example, the fluctuation information acquisition unit C32 acquires a condition (for example, time information or an event identifier) corresponding to the blood sugar level group acquired by the blood sugar level acquisition unit C31. Next, the fluctuation information acquisition unit C32 acquires the past blood sugar level group that satisfies the condition (for example, the past blood sugar level group corresponding to the same time information and/or the same event identifier). For example, the fluctuation information acquisition unit C32 acquires information regarding the difference between two blood sugar level groups. Note that the information regarding the difference is, for example, a set of two blood sugar levels included in two blood sugar level groups, and the fluctuation information acquisition unit C32 acquires multiple sets of two blood sugar levels corresponding to the same time information or acquired after the same time period from the start of the event, and acquires the difference between the blood sugar levels for each set. Thereafter, the fluctuation information acquisition unit C32 acquires fluctuation information, using the difference between the two acquired blood sugar levels. In such a case, fluctuation information is, for example, the cumulative value of the difference between the two blood sugar levels, the average value of the difference between the two blood sugar levels, the median value of the difference between the two blood sugar levels, or the maximum value of the difference between the two blood sugar levels.

### (c-2) In the case where fluctuation information is the difference between blood sugar level fluctuation trends under the same condition

For example, as in the processing in (c-1), the fluctuation information acquisition unit C32 acquires the blood sugar level group acquired by the blood sugar level acquisition unit C31 and the past blood sugar level group that satisfies the condition corresponding to the aforementioned blood sugar level group. Next, the fluctuation information acquisition unit C32 acquires the blood sugar level fluctuation trend of the two blood sugar level groups. Next, the fluctuation information acquisition unit C32 acquires fluctuation information that is the difference between the respective blood sugar level fluctuation trends of the two blood sugar level groups.

### (c-3) In the case where fluctuation information is the difference between scores

Fluctuation information is, for example, a score corresponding to the past blood sugar level group. The fluctuation information acquisition unit C32 acquires fluctuation information that is a score corresponding to blood sugar fluctuations over time, using the blood sugar level group and the past blood sugar level group.

The fluctuation information acquisition unit C32 acquires a lower score for a larger value of information that is based on the difference acquired in (c-1) (for example, the cumulative value of the difference between the two blood sugar levels, the average value of the difference between the two blood sugar levels, the median value of the difference between the two blood sugar levels, or the maximum value of the difference between the two blood sugar levels). For example, the fluctuation information acquisition unit C32 calculates a score, using a decreasing function whose parameter is the difference acquired in (c-1). For example, the fluctuation information acquisition unit C32 acquires a score that is paired with the information that is based on the difference acquired in (c-1), from the correspondence table. Note that, in such a case, the correspondence table contains two or more pieces of correspondence information that each include information indicating the range of the information that is based on the difference acquired in (c-1), and a score.

For example, the fluctuation information acquisition unit C32 acquires a lower score for a larger difference between the blood sugar level fluctuation trends in (c-2). For example, the fluctuation information acquisition unit C32 calculates a score, using a decreasing function whose parameter is the difference between the blood sugar level fluctuation trends in (c-2). For example, the fluctuation information acquisition unit C32 acquires a score that is paired with the difference between the blood sugar level fluctuation trends in (c-2), from the correspondence table. Note that, in such a case, the correspondence table contains two or more pieces of correspondence information that each include information indicating the range of the difference between the blood sugar level fluctuation trends, and a score.

For example, the fluctuation information acquisition unit C32 acquires the differences between each blood sugar level in the blood sugar level group acquired in a predetermined period after a meal and the blood sugar level in the past blood sugar level group of the same user acquired in a predetermined period after a meal corresponding to the same elapsed time, and acquires a score using the multiple differences. The fluctuation information acquisition unit C32 typically acquires a lower score for a larger difference value.

The judgment unit C33 judges whether or not the blood sugar level-related information satisfies predetermined suggestion conditions, to acquire a judgment result. Note that blood sugar level-related information is information regarding one or more blood sugar levels acquired by the blood sugar level acquisition unit C31. Examples of the blood sugar level-related information include a blood sugar level itself and fluctuation information.

The suggestion conditions are conditions stored in the storage unit C1. The suggestion conditions are conditions regarding blood sugar level-related information. Examples of the suggestion conditions include, "the blood sugar level is no lower than a threshold value", "the blood sugar level is higher than a threshold value", "fluctuation information is no lower than a threshold value", "fluctuation information is higher than a threshold value", "The score is no lower than a threshold value", and "the score is lower than a threshold value".

It is preferable that the judgment unit C33 judges whether or not fluctuation information satisfies suggestion conditions.

The judgment unit C33 also uses other information to judge whether or not the blood sugar level or the fluctuation information satisfies the suggestion conditions. Note that other information is information other than the blood sugar level or the fluctuation information. Other information is information that is not related to the blood sugar level. Other information is, for example, environmental information acquired by the environment information acquisition unit C30. Other information is, for example, time, weather, and season. Other information is, for example, one or more pieces of user dynamic attribute information. Note that, for example, the processing unit C3 acquires user dynamic attribute information.

The suggestion information acquisition unit C34 acquires suggestion information when the judgment result acquired by the judgment unit C33 indicates that the suggestion conditions are satisfied. It is preferable that suggestion information is stored in the storage unit C1, but suggestion information may be acquired from an external device (not shown).

For example, if the judgement result indicates that the suggestion conditions are satisfied, the suggestion information acquisition unit C34 acquires suggestion basis information, and forms suggestion information, using the suggestion basis information and the acquired blood sugar level-related information. For example, when suggestion basis information is information having a variable to which blood sugar level-related information is to be assigned, if the judgment result indicates that the suggestion conditions are satisfied, the suggestion information acquisition unit C34 acquires the suggestion basis information and assigns the acquired blood sugar level-related information to the variable in the suggestion basis information, to form suggestion information.

For example, if the judgment result indicates that the suggestion conditions are satisfied, the suggestion information acquisition unit C34 acquires suggestion information corresponding to the acquired blood sugar level-related information. In such a case, the suggestion conditions related to the blood sugar level-related information and the suggestion information or the suggestion basis information are associated with each other.

For example, if the judgment result indicates that the suggestion conditions are satisfied, the suggestion information acquisition unit C34 acquires suggestion information corresponding to fluctuation information.

The suggestion information acquisition unit C34 determines a suggestion condition that matches the acquired blood sugar level-related information, from the correspondence information storage unit C13, and acquires suggestion information corresponding to the suggestion condition or suggestion information using suggestion basis information corresponding to the suggestion condition.

It is preferable that, if the judgment unit C33 judges that the judgment result acquired in response to the blood sugar level acquisition unit C31 acquiring the blood sugar level satisfies the suggestion conditions, the suggestion information acquisition unit C34 immediately acquires suggestion information.

The suggestion information acquisition unit C34 may also use the blood sugar level and information other than the fluctuation information to acquire the suggestion information.

The output unit C4 outputs various kinds of information. Examples of various kinds of information include suggestion information, blood sugar level-related information, and a score. That is to say, the output unit C4 may output the blood sugar level-related information and the score acquired by the processing unit C3.

Here, the output is a concept that encompasses display on a display screen, projection using a projector, printing by a printer, the output of a sound, transmission to an external device, accumulation on a recording medium, delivery of a processing result to another processing device or another program, and the like.

The suggestion information output unit C41 outputs the suggestion information acquired by the suggestion information acquisition unit C34. It is preferable that the suggestion information output unit C41 immediately outputs the suggestion information when the suggestion information acquisition unit C34 acquires the suggestion information.

It is preferable that the storage unit C1, the reference information storage unit C11, the past blood sugar level group storage unit C12, and the correspondence information storage unit C13 are non-volatile recording media, but they may be realized using volatile recording media.

There is no limitation on the process in which information is stored in the storage unit C1 or the like. For example, information may be stored in the storage unit C1 or the like via a recording medium, or information transmitted via a communication line or the like may be stored in the storage unit C1 or the like, or information input via an input device may be stored in the storage unit C1 or the like.

The acceptance unit C2 can be realized using a device driver for the input means such as a touch panel or a keyboard, or control software or the like for controlling the menu screen.

The processing unit C3, the environment information acquisition unit C30, the blood sugar level acquisition unit C31, the fluctuation information acquisition unit C32, the judgment unit C33, and the suggestion information acquisition unit C34 can typically be realized using a processor, a memory, or the like. The processing procedures performed by the processing unit C3 or the like typically are realized using software, and the software is recorded on a recording medium such as a ROM. However, such processing procedures may be realized using hardware (a dedicated circuit). Note that the processor is a CPU, an MPU, a GPU, or the like, and there is no limitation on the type thereof.

It is preferable that th blood sugar level acquisition unit C31 is a non-invasive blood sugar level sensor. It is preferable that th blood sugar level acquisition unit C31 is a non-invasive blood sugar level sensor. sing visible light, near-infrared light, or the like. One example of such a technology is the technology described on the Internet homepage (URL: https://www.qst.go.jp/site/press/1230.html). In addition, there is a technology for measuring a blood sugar level by simply applying a mid-infrared laser to a finger (Internet homepage (URL: https://dm-net.co.jp/calendar/2017/027218.php)). However, there is no limitation on the means or method used by the blood sugar level acquisition unit C31 to acquire a blood sugar level.

The output unit C4 and the suggestion information output unit C41 may be regarded as including or not including an output device such as a display or a speaker. The output unit C4 or the like can be realized using the driver software of the output device, the driver software of the output device and the output device, or the like. The output unit C4 or the like may be realized using a wireless or wired communication means.

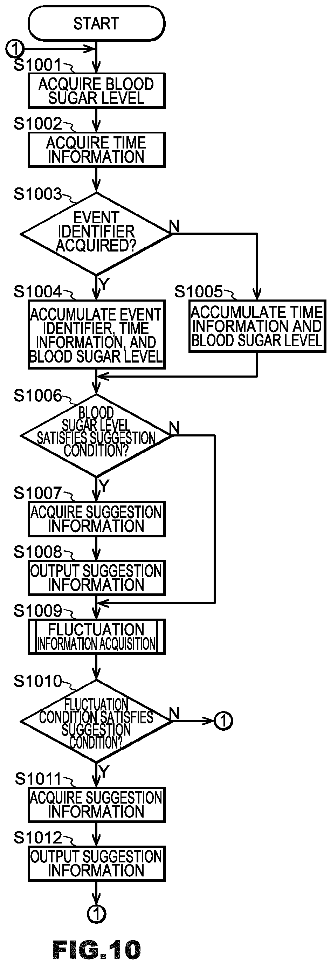

Next, examples of operations of the health support device C will be described with reference to the flowchart in FIG. 10.

(Step S1001) The blood sugar level acquisition unit C31 acquires a blood sugar level. Note that there is no limitation on the time the time at which the blood sugar level acquisition unit C31 acquires the blood sugar level, or the trigger therefor. It is preferable that the blood sugar level acquisition unit C31 constantly acquires the blood sugar level at short intervals (for example, 1 second, 5 seconds, etc.).

The blood sugar level acquisition unit C31 may acquire the blood sugar level received by the acceptance unit C2. In such a case, it is preferable that the blood sugar level is associated with the user identifier.

(Step S1002) The blood sugar level acquisition unit C31 acquires time information from a clock (not shown).

(Step S1003) The acceptance unit C2 judges whether or not an event identifier has been acquired. If an event identifier has been acquired, processing proceeds to step S1004, and if an event identifier has not been acquired, processing proceeds to step S1005. Note that once an event identifier is acquired, processing may proceed to step S1004 for a predetermined period of time (for example, 150 minutes, 2 hours, etc.). Alternatively, once an event identifier is acquired, processing may proceed to step S1004 until an instruction to terminate the acquisition of the blood sugar level is accepted. The acceptance unit C2 may acquire an event identifier input from the user, or automatically acquire an event identifier. Note that proceeding to step S1004 means that the judgment result in step S1003 is "Y".

(Step S1004) The blood sugar level acquisition unit C31 accumulates the blood sugar level acquired in step S1001 in the storage unit C1 in association with the time information acquired in step S1002 and the event identifier acquired in step S1003. Processing proceeds to step S1006. The blood sugar level acquisition unit C31 may accumulate the blood sugar level or the like in association with the user identifier.

(Step S1005) The blood sugar level acquisition unit C31 accumulates the blood sugar level acquired in step S1001 in the storage unit C1 in association with the time information acquired in step S1002. The blood sugar level acquisition unit C31 may accumulate the blood sugar level or the like in association with the user identifier.

(Step S1006) The judgment unit C33 judges whether or not the blood sugar level accumulated in step S1004 or S1005 satisfies the suggestion conditions. If the processing conditions are satisfied, processing proceeds to step S1007, and if the processing conditions are not satisfied, processing proceeds to step S1009. Note that the processing in this step may be omitted and processing may be proceeds to step S1010. The suggestion conditions used here may be suggestion conditions paired with the user identifier corresponding to the blood sugar level.

(Step S1007) The suggestion information acquisition unit C34 acquires suggestion information corresponding to the suggestion conditions.

(Step S1008) The suggestion information output unit C41 outputs the suggestion information acquired in step S1007.

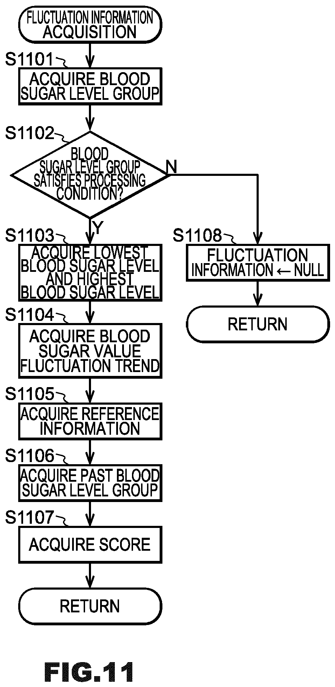

(Step S1009) The fluctuation information acquisition unit C32 performs fluctuation information acquisition processing. An example of fluctuation information acquisition processing will be described with reference to the flowchart in FIG. 11.

(Step S1010) The judgment unit C33 judges whether or not the fluctuation information acquired in step S1009 satisfies the suggestion conditions. If the suggestion conditions are satisfied, processing proceeds to step S1011, and if the suggestion conditions are not satisfied, processing proceeds to step S1001.

(Step S1011) The suggestion information acquisition unit C34 acquires suggestion information corresponding to the suggestion conditions from the correspondence information storage unit C13.

(Step S1012) The suggestion information output unit C41 outputs the suggestion information acquired in step S1011. Processing returns to step S1001.

Note that, in the flowchart in FIG. 10, processing ends when the power is turned off or an interruption occurs to end the processing.

Next, an example of the fluctuation information acquisition processing in step S1009 will be described with reference to the flowchart in FIG. 11.

(Step S1101) The fluctuation information acquisition unit C32 acquires a blood sugar level group that includes two or more blood sugar levels stored in the storage unit C1. Note that the blood sugar level group typically includes the immediately previous blood sugar level acquired. Here, the fluctuation information acquisition unit C32 may acquire a blood sugar level that is paired with the user identifier.

(Step S1102) The fluctuation information acquisition unit C32 judges whether or not the blood sugar level group acquired in step S1101 satisfies a processing condition. If the processing condition is satisfied, processing proceeds to step S1103, and if the processing condition is not satisfied, processing proceeds to step S1108.

Note that the processing condition is a condition for acquiring fluctuation information. In addition, the processing condition is a condition for judging whether or not the blood sugar level group with which fluctuation information can be acquired has been acquired. The processing condition is, for example, that a predetermined number or more of blood sugar levels have been accumulated in the storage unit C1, that blood sugar levels corresponding to a period no shorter than or longer than a predetermined period have been accumulated in the storage unit C1, or that blood sugar levels corresponding to a period no shorter than or longer than a predetermined period from the occurrence of an event (for example, the start of a meal, the start of exercise, or an insulin injection has been given) have been accumulated in the storage unit C1.

(Step S1103) The fluctuation information acquisition unit C32 acquires the lowest blood sugar level and the highest blood sugar level from the blood sugar level group acquired in step S1101.

(Step S1104) The fluctuation information acquisition unit C32 acquires pieces of time information respectively corresponding to the lowest blood sugar level and the highest blood sugar level acquired in step S1103. The fluctuation information acquisition unit C32 calculates a blood sugar level fluctuation trend, using the lowest blood sugar level, the highest blood sugar level, and the two pieces of time information. Note that an example of a blood sugar level fluctuation trend is a blood sugar level fluctuation speed.

(Step S1105) The fluctuation information acquisition unit C32 acquires reference information from the reference information storage unit C11.

(Step S1106) The fluctuation information acquisition unit C32 acquires a past blood sugar level group from the past blood sugar level group storage unit C12.

(Step S1107) The fluctuation information acquisition unit C32 acquires a score, using one or more pieces of information of: the blood sugar level group acquired in step S1101; the lowest blood sugar level; the highest blood sugar level; the blood sugar level fluctuation trend; the reference information; and the past blood sugar level group.

For example, the fluctuation information acquisition unit C32 assigns the blood sugar level fluctuation speed acquired in S1104 and the score acquired in step S1107 to a variable "fluctuation information". Processing returns to higher-level processing.

Note that an example of a score acquisition method is as described above.

(Step S1108) The fluctuation information acquisition unit C32 assigns "NULL" to the variable "fluctuation information". Processing returns to higher-level processing.

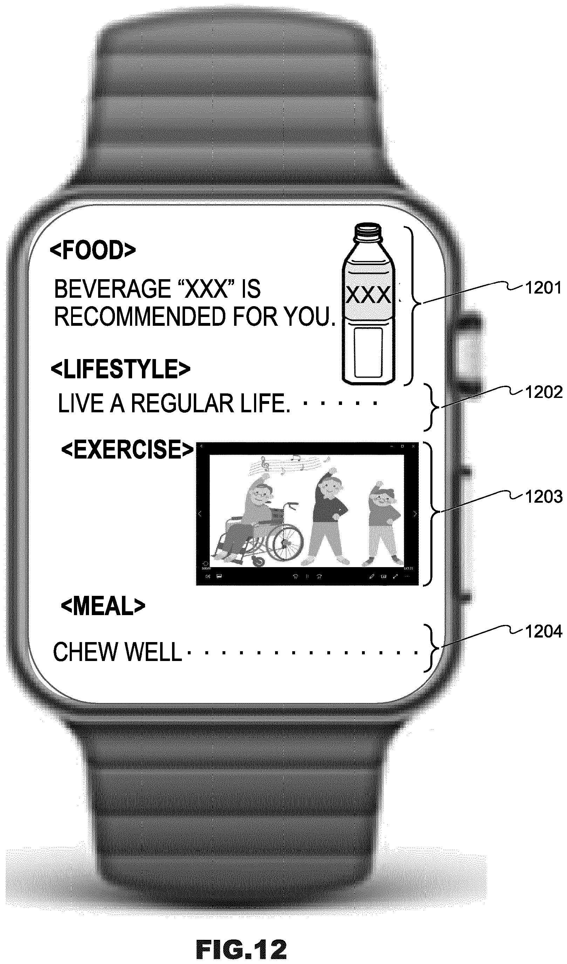

Hereinafter, specific operations of the health support device C according to the present embodiment will be described. An example of the external appearance of the health support device C is shown in FIG. 12. The health support device C is, for example, a device having the shape of a wristwatch.

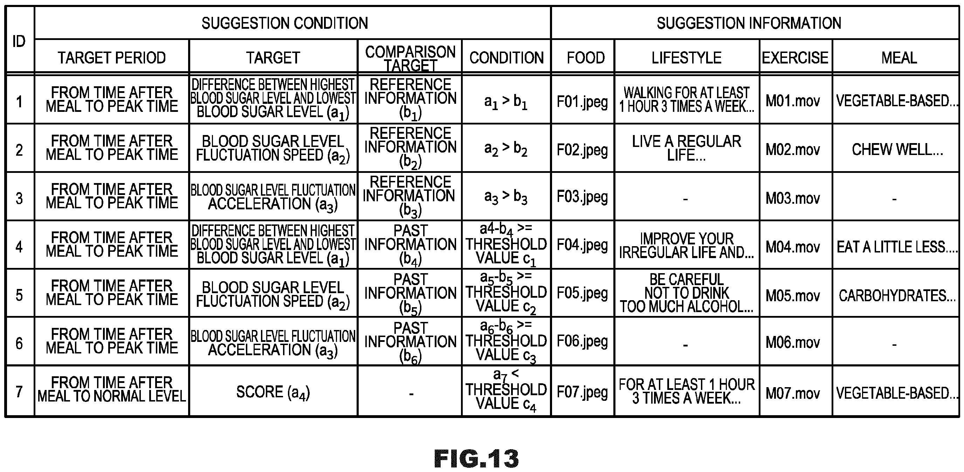

It is assumed that the correspondence information storage unit C13 in the health support device C stores the correspondence information management table shown in FIG. 13. The correspondence information management table is a table for managing correspondence information. The correspondence information management table contains one or more records each include "ID", "suggestion condition", and "suggestion information". The "suggestion condition" includes "target period", "target", "comparison information", and "condition". "Suggestion information" includes "food", "lifestyle", "exercise", and "meal". "ID" is information identifying the record".

"Suggestion condition" is information defining the suggestion condition. The suggestion condition here does not include other information, but may be a condition that includes other information such as environmental information. In addition, "suggestion condition" may only include "target", "comparison information", and "condition".

"Target period" is information indicating the period in which the blood sugar level included in the blood sugar level group subjected to the judgment regarding whether or not the suggestion condition is satisfied is acquired, and indicates the period specified by the time information associated with the blood sugar level. "From meal to peak time" is a condition that is satisfied when it is detected that the blood sugar level reaches a peak after the start of a meal. "From meal to normal time" is a condition that is satisfied when it is detected that, after the blood sugar level reaches a peak after the start of a meal, the blood sugar level falls to the blood sugar level at the start of the meal or a value no higher than a threshold value (a normal or ordinary blood sugar level).

"Target" is information specifying the information that is used when the judgment unit C33 performs judgment processing. "Target" here is, for example, the difference between the lowest blood sugar level and the highest blood sugar level (a₁), a blood sugar level fluctuation speed (a₂), a blood sugar level fluctuation acceleration (a₃), or a score (a₄).

"Comparison information" is information regarding the comparison target. "Comparison information" is, for example, reference information (any one of b₁ to b₃), or past information (any one of b₃ to b₆). Past information is information acquired using the past blood sugar level group of the same user as the user for which the blood sugar level group subjected to the judgment was acquired, and is the same type of information as "Target" that is paired with the past information. Past information here is the difference between the lowest blood sugar level and the highest blood sugar level acquired from the past blood sugar level group, a blood sugar level fluctuation speed, or a blood sugar level fluctuation acceleration. Note that "comparison information" may be the blood sugar level itself.

"Condition" is a condition for outputting suggestion information. Note that threshold values c₁ to c₄ in the condition may be common to all users, or may be set for each user.

"Suggestion information" is suggestion information that is output when the suggestion condition is satisfied, or suggestion basis information that is information used to form suggestion information. Examples of suggestion basis information include a template for suggestion information, a program for acquiring suggestion information, a file name for forming suggestion information, and so on.

Suggestion information typically is information that is to be provided to the user in order to improve the state of the user regarding the blood sugar level. "Food" is information specifying advice on foods to be ingested. "Lifestyle" is information specifying advice on improving lifestyle habits. "Exercise" is information specifying advice on exercise. "Meal" is information specifying advice on meals. Note that "food" here is the file name of an image. "Lifestyle" here is a string. "Exercise" here is the file name of a movie. "Meal" here is a string. However, there is no limitation on the data type of "suggestion information". "Suggestion information" may be a set of two or more pieces of information of two or more data types.

In such a situation, it is assumed that the user has entered an event identifier "meal start information" into the health support device C and has started eating. As a result, the acceptance unit C2 of the health support device C accepts the event identifier "meal start information". Note that the event identifier "meal start information" is input by providing an instruction using a button on the screen, but the event identifier may be input using a voice or the like, and any method may be employed.

Next, the blood sugar level acquisition unit C31 starts acquiring blood sugar levels. For example, the blood sugar level acquisition unit C31 acquires blood sugar levels generally on a regular basis, and accumulates the blood sugar levels in the storage unit C1 in pair with time information. Note that the meal start information here is a relative time from the acceptance of the event identifier "meal start information".

Next, for example, the blood sugar level acquisition unit C31 acquires time information, using a timer (not shown). Thereafter, the blood sugar level acquisition unit C31 accumulates the blood sugar levels in the storage unit C1 in association with the time information and the event identifier "meal start information".

Next, the fluctuation information acquisition unit C32 acquires a blood sugar level group that is a set of blood sugar levels stored in the storage unit C1. Thereafter, the fluctuation information acquisition unit C32 judges whether or not the acquired blood sugar level group satisfies a processing condition. The processing condition here is the "target period" in the correspondence information management table in FIG. 13. That is to say, the fluctuation information acquisition unit C32 judges whether or not the blood sugar levels included in the blood sugar level group have reached the peak blood sugar level and have started decreasing, from the acquired blood sugar level group. Here, the processing condition corresponding to the "target period" is not satisfied, and therefore "NULL" is assigned to the variable "fluctuation information". That is to say, fluctuation information is not acquired and suggestion information is not output.

It is assumed that, as a result of the health support device C continuously performing the above-described processing, the fluctuation information acquisition unit C32 detects that the blood sugar levels included in the blood sugar level group in the storage unit C1 have reached the peak blood sugar level and have started decreasing. That is to say, for example, after acquiring a blood sugar level lower than the immediately previous blood sugar level (a candidate for the highest blood sugar level), if the fluctuation information acquisition unit C32 detects that only blood sugar levels lower than the candidate for the highest blood sugar level are acquired for a predetermined period, the fluctuation information acquisition unit C32 judges that the time information indicated by the candidate for the highest blood sugar level is the peak time. Note that the method for judging the peak time of the graph is a well-known technique and there is no limitation.

Next, the fluctuation information acquisition unit C32 acquires the difference between the lowest blood sugar level and the highest blood sugar level (a₁), the blood sugar level fluctuation speed (a₂), and the blood sugar level fluctuation acceleration (a₃), which are "targets" paired with the target period "from meal to peak time", from the correspondence information management table in FIG. 13.

Next, the judgment unit C33 acquires information regarding the comparison targets and conditions for the IDs "1" to "6". Next, the judgment unit C33 judges whether or not the fluctuation information acquired by the fluctuation information acquisition unit C32 satisfies the conditions for the records indicated by the IDs "1" to "6" is satisfied. Here, for example, it is assumed that the judgment unit C33 judges that the condition indicated by the ID "2" is satisfied.

Next, the suggestion information acquisition unit C34 acquires suggestion information indicated by the ID "2" from the correspondence information management table in FIG. 13. The suggestion information indicated by the ID "2" is "<Food> F02.jpeg <Lifestyle> Live a regular life ... <Exercise> M02.mov <Meal> Chew well...".

Next, the suggestion information output unit C41 outputs the suggestion information indicated by the ID "2". Note that the suggestion information output unit C41 acquires the image "F02.jpeg" from the storage unit C 1 and outputs the image. In addition, the suggestion information output unit C41 acquires the movie "M02.mov" from the storage unit C1 and outputs the movie.

Examples of such outputs are shown in FIG. 12. 1201 in FIG. 12 indicates an image of advice on foods to be ingested. 1202 indicates a string of advice on improving lifestyle habits. 1203 indicates a movie of advice on exercise. 1204 indicates a string of advice on meals.

Thereafter, the processing performed by the blood sugar level acquisition unit C31 to acquire and accumulate blood sugar levels, and so on progress.

It is assumed that the fluctuation information acquisition unit C32 detects that the blood sugar levels included in the blood sugar level group in the storage unit C1 have reached the peak, have started decreasing, and have returned to the normal blood sugar level. That is to say, for example, after acquiring the highest blood sugar level, if the fluctuation information acquisition unit C32 detects that the blood sugar level has decreased and the difference from the blood sugar level at the start of the meal is 0 or no higher than a threshold value, the fluctuation information acquisition unit C32 judges that the record matches the target period "from time after meal to normal level". Note that such a judgment method is a well-known technique and there is no limitation.

Next, it is assumed that the fluctuation information acquisition unit C32 acquires the score (S₄) that is the "target" paired with the target period "from time after meal to normal level". Note that the method for acquiring the score (S₄) can be realized using various algorithms as described above, and there is no limitation.

Next, the judgment unit C33 acquires the condition "score (a₄) < threshold value c₄" indicated by the ID "7" from the correspondence information management table in FIG. 13. Next, the judgment unit C33 judges whether or not the acquired "score (S₄) satisfies the condition "score (a₄) < threshold value c₄". Here, for example, it is assumed that the judgment unit C33 judges that the condition is satisfied.

Next, the suggestion information acquisition unit C34 acquires suggestion information indicated by the ID "7" from the correspondence information management table in FIG. 13. In addition, the suggestion information acquisition unit C34 forms suggestion information to be output, using the acquired suggestion information.

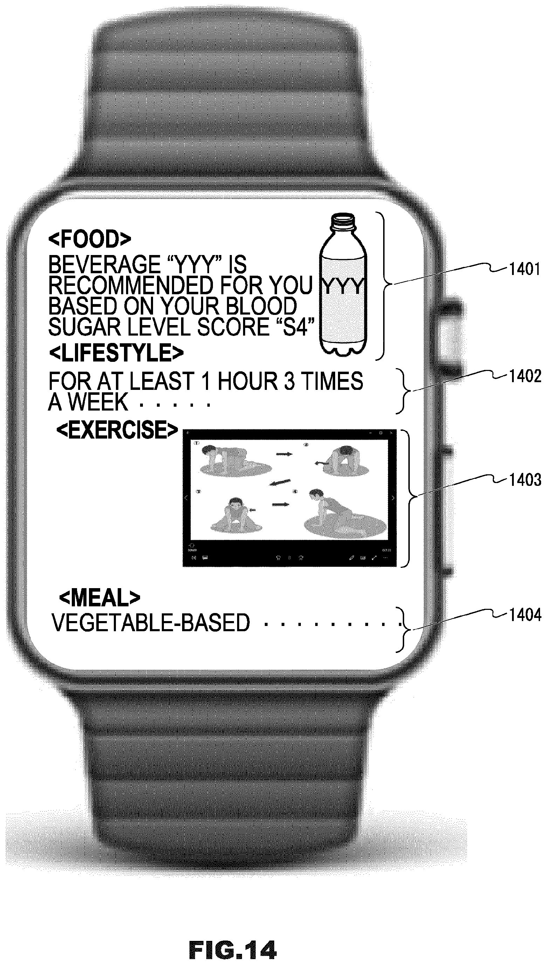

Next, the suggestion information output unit C41 outputs the suggestion information thus formed. An example of such an output is shown in FIG. 6. 1401 in FIG. 14 indicates an image of advice on foods to be ingested. 1402 indicates a string of advice on improving lifestyle habits. 1403 indicates a movie of advice on exercise. 1404 indicates a string of advice on meals.

As described above, according to the present embodiment, appropriate advice can be provided based on the user's blood sugar level.

In addition, according to the present embodiment, it is possible to provide multifaceted advice such as advice on foods to be ingested, advice on improving lifestyle habits, advice on exercise, and advice on meals, based on the user's blood sugar level.

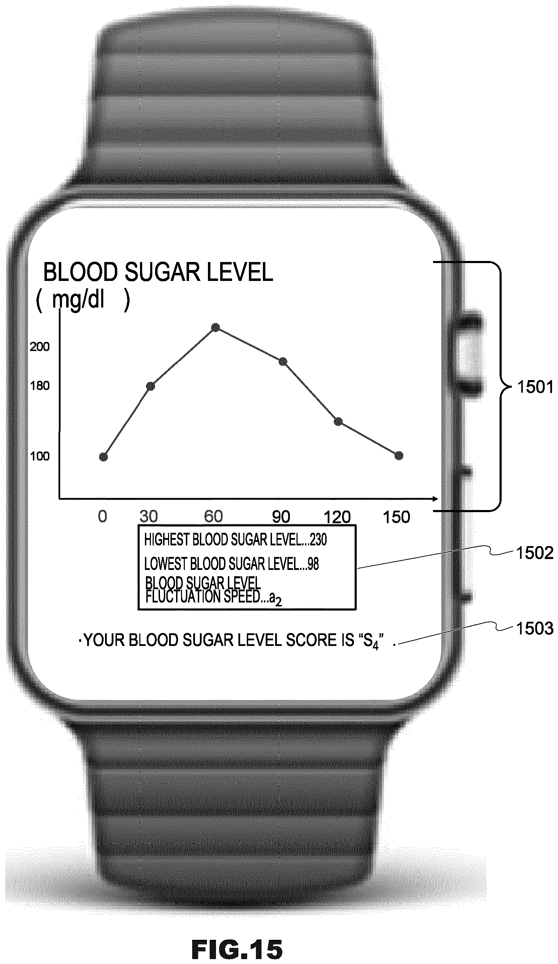

Note that, in the present embodiment, the output unit C4 may output blood sugar level information and a score. An example output in such a case is shown in FIG. 15. 1501 in FIG. 15 indicates an example of outputting the blood sugar levels acquired by the blood sugar level acquisition unit C31 in chronological order as a graph. 1502 indicates the highest blood sugar level and the lowest blood sugar level acquired by the blood sugar level acquisition unit C31 and the blood sugar level fluctuation speed acquired by the fluctuation information acquisition unit C32. 1503 indicates information that includes the acquired score, and is information used to alert the user.

Note that the processing in the present embodiment may be realized using software. This software may be distributed through software downloading or the like. Also, this software may be recorded on a recording medium such as a CD-ROM and distributed. Note that the same applies to the other embodiments in the present description. The software that realizes the health support device C according to the present embodiment is the program described below. That is to say, this program is a program for enabling a computer to function as: a blood sugar level acquisition unit that acquires a blood sugar level of one user; a judgment unit that judges whether or not blood sugar level-related information regarding the blood sugar level satisfies a predetermined suggestion condition, to acquire a judgment result; a suggestion information acquisition unit that acquires suggestion information when the judgment result indicates that the suggestion condition is satisfied; and a suggestion information output unit that outputs the suggestion information.

### Fourth Embodiment

The present embodiment is different from the first embodiment in that the health support device C functions together with a terminal device C5 used by the user, to realize a health support system D.

That is to say, the health support device C performs the processing described in the first embodiment, using a blood sugar level received from a terminal device C5, to acquire suggestion information and so on, and transmits the information to the terminal device C5. The terminal device C5 receives and outputs suggestion information and so on. Note that suggestion information and so may only be constituted by suggestion information, or include blood sugar level information and fluctuation information in addition to suggestion information.

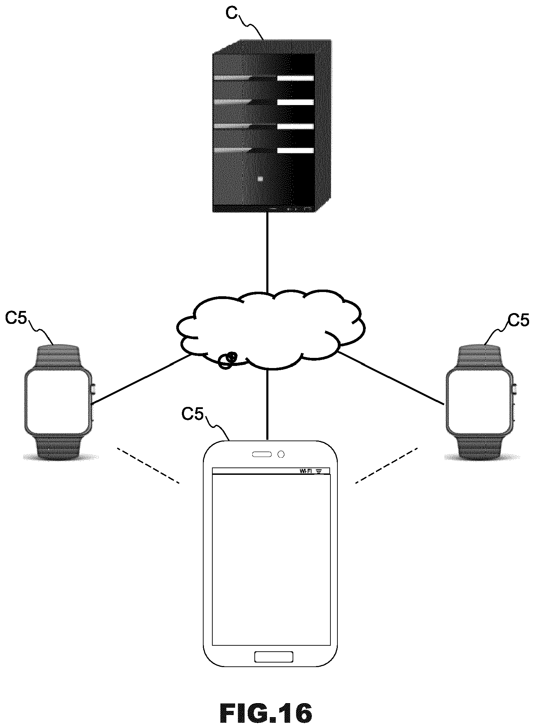

FIG. 16 is an example of a conceptual diagram for the health support system D according to the present embodiment. The health support system D includes one or more health support devices C and one or more terminal devices C5. Although it is preferable that the health support device(s) C and the terminal device(s) C5 are in a one-to-n relationship (where n is 2 or more), they may be in a one-to-one relationship. For example, when the health support device(s) C and the terminal device(s) C5 are in a one-to-one relationship, each health support device C is, for example, a smart phone, a personal computer, a tablet terminal, or the like. Each terminal device C5 is, for example, a smart watch, a smart phone, a personal computer, a table terminal, or the like.

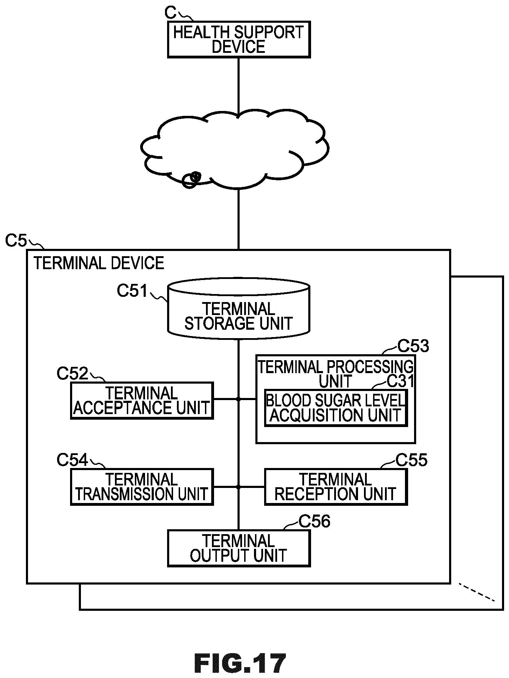

FIG. 17 is a block diagram for the health support system D according to the present embodiment. Each terminal device C5 includes a terminal storage unit C51, a terminal acceptance unit C52, a terminal processing unit C53, a terminal transmission unit C54, a terminal reception unit C55, and a terminal output unit C56. The terminal processing unit C53 includes a blood sugar level acquisition unit C31.

The acceptance unit C2 included in the health support device C here receives various kinds of instructions and information from a terminal device C5. Examples of the various kinds of instructions and information include not only a suggestion information output instruction, a fluctuation information output instruction, a blood sugar level output instruction, and an event identifier, but also a blood sugar level.

The output unit C4 included in the health support device C here transmits various kinds of information to a terminal device C5. Examples of the various kinds of information include suggestion information, blood sugar level information, and fluctuation information. Note that the output unit C4 may display various kinds of information or output audio.

The terminal storage unit C51 included in each terminal device C5 stores various kinds of information. Examples of the various kinds of information include a user identifier, a blood sugar level, information including a pair consisting of a blood sugar level and time information, a blood sugar level group, and a group of pairs each consisting of a blood sugar level and time information.

It is preferable that the terminal storage unit C51 is a non-volatile recording medium, but may be realized using a volatile recording medium.

There is no limitation on the process in which information is stored in the terminal storage unit C51. For example, information may be stored in the storage unit C51 via a recording medium, or information transmitted via a communication line or the like may be stored in the storage unit C51, or information input via an input device may be stored in the storage unit C51.

The terminal acceptance unit C52 accepts various kinds of instructions and information. Examples of the various kinds of instructions and information include a suggestion information output instruction and an event identifier. The suggestion information output instruction is an instruction to output suggestion information.

The means for inputting various kinds of instructions and information may be any means such as a touch panel, a keyboard, a mouse, a means that employs a menu screen, or the like. The terminal acceptance unit C52 can be realized using a device driver for the input means such as a touch panel or a keyboard, or control software or the like for controlling the menu screen.

The terminal processing unit C53 performs various kinds of processing. Examples of the various kinds of processing include the processing that is performed by the blood sugar level acquisition unit C31.

For example, the terminal processing unit C53 adds the user identifier of the terminal storage unit C51 to the various kinds of instructions and information accepted by the terminal acceptance unit C52, to form various kinds of instructions and information to be transmitted.

The terminal processing unit C53 may typically be realized using a processor, a memory, and so on. The processing procedures performed by the processing unit C53 or the like typically are realized using software, and the software is recorded on a recording medium such as a ROM. However, such processing procedures may be realized using hardware (a dedicated circuit). Note that the processor is a CPU, an MPU, a GPU, or the like, and there is no limitation on the type thereof.

The terminal transmission unit C54 transmits various kinds of instructions and information to the health support device C. Examples of the various kinds of instructions and information include a suggestion information output instruction, an event identifier, a blood sugar level, information including a pair consisting of a blood sugar level and time information, a blood sugar level group, and a group of pairs each consisting of a blood sugar level and time information. It is preferable that the terminal transmission unit C54 transmits various kinds of instructions and information in association with the user identifier.

The terminal transmission unit C54 may typically be realized using a wireless or wired communication means, but may be realized using a broadcast means.

The terminal reception unit C55 receives various kinds of information from the health support device C. Examples of the various kinds of information include suggestion information, blood sugar level information, and fluctuation information.

The terminal reception unit C55 may typically be realized using a wireless or wired communication means, but may be realized using a broadcast receiving means.

The terminal output unit C56 outputs various kinds of information. Examples of the various kinds of information include suggestion information, blood sugar level information, and fluctuation information.

The terminal output unit C56 may be regarded as including or not including an output device such as a display or a speaker. The terminal output unit C56 can be realized using the driver software of the output device, the driver software of the output device and the output device, or the like.

Next, examples of operations of a terminal device C5 included in the health support system D will be described. The terminal acceptance unit C52 accepts an event identifier. Next, the terminal processing unit C53 reads out a user identifier in the terminal storage unit C51, and forms information that includes the user identifier and the event identifier. Next, the terminal transmission unit C54 transmits the information to the health support device C. Note that, through such processing, the health support device C accumulates the user identifier, the event identifier, and time information in association with each other.

Next, the blood sugar level acquisition unit C31 included in the terminal processing unit C53 acquires a blood sugar level. The terminal processing unit C53 reads out a user identifier in the terminal storage unit C51, and forms information that includes the user identifier and a blood sugar level. Next, the terminal transmission unit C54 transmits the information to the health support device C. Note that, through such processing, the health support device C accumulates the user identifier, the event identifier, time information, and the blood sugar level in association with each other.

The terminal device C5 continues processing to transmit information that includes the user identifier and the blood sugar level. The terminal reception unit C55 receives suggestion information from the health support device C. Next, the terminal output unit C56 outputs the received suggestion information. An example output of the result of the above-described processing is shown in FIG. 12.

As described above, according to the present embodiment, it is possible to provide each of one or more users with a suggestion for improving the state of the blood sugar level of the user.

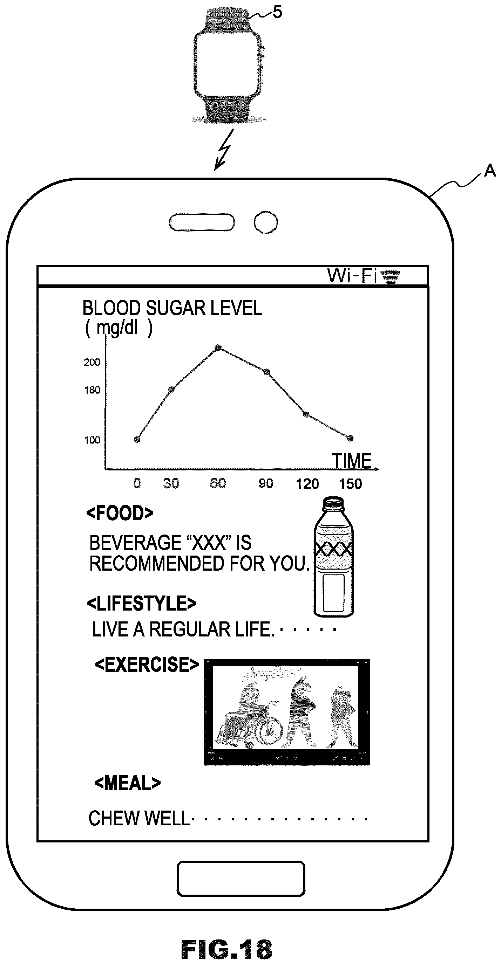

Note that, in the present embodiment, when the health support device(s) C and the terminal device(s) C5 are in a one-to-one relationship, each terminal device C5 performs processing to only acquire a blood sugar level and transmit it to a health support device C, for example. In such a case, the terminal reception unit C55 and the terminal output unit C56 may be omitted from each terminal device C5. In such a case, each health support device C is, for example, a smart phone, a personal computer, a server, or the like. The server is a so-called cloud server, an ASP server, or the like, and there is no limitation. In addition, when a health support device C is a smart phone or the like, the above-described processing is realized by an application in the smart phone or the like. A conceptual diagram for such a case is shown in FIG. 18. In FIG. 18, the communication means between the health support device C and the terminal device C5 may be a distance wireless communication such as Bluetooth (registered trademark), communication via the Internet, or the like, and there is no limitation on the communication means.

### Fifth Embodiment

The present embodiment describes a blood sugar level estimation system that includes a blood sugar level estimation device that acquires and outputs a blood sugar level by applying near-infrared light information that can be acquired by emitting near-infrared light (hereinafter referred to as "NIRS" as appropriate) to a user, to learning information (for example, a learning model) formed using two or more pieces of training data each including NIRS information that can be acquired by applying near-infrared light to a human body, and a blood sugar level. Note that training data may include information other than NIRS information. Examples of information other than NIRS information include one or more user dynamic attribute values, one or more user static attribute values, and one or more device characteristic values. The blood sugar level estimation device may receive NIRS information from a device, and transmit the estimated blood sugar level to an external device. Furthermore, learning information may be a learning model acquired through machine learning.

The present embodiment also describes a blood sugar level estimation system that includes a blood sugar level estimation device that estimates a blood sugar level, using learning information acquired using two or more wavelengths to acquire training data. Note that the number of wavelengths during learning may be greater than the number of wavelengths during prediction.

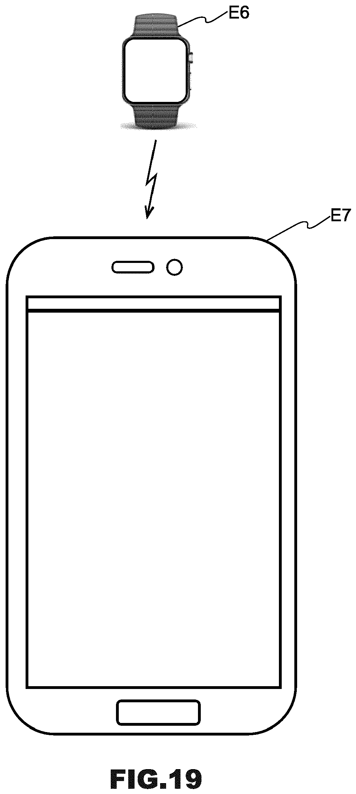

FIG. 19 is an example of a conceptual diagram for a blood sugar level estimation system C according to the present embodiment. The blood sugar level estimation system E includes a NIRS device E6 and a blood sugar level estimation device E7. The blood sugar level estimation system E may be a system that includes two or more NIRS devices E6 and one blood sugar level estimation device E7. Note that the NIRS device E6 and the blood sugar level estimation device E7 may be integrated into a single device.

The NIRS device E6 includes a near-infrared light emitting unit and a near-infrared light receiving unit, acquires NIRS information, which will be described later, or a NIRS information source, which will be described later, and passes them to the blood sugar level estimation device E7. It is preferable that the NIRS device E6 is watch-shaped, but there is no limitation on the shape thereof. The NIRS device E6 may be any device that can emit NIRS to any part of the human body and receive reflected light.

The blood sugar level estimation device E7 is a device that acquires NIRS information based on information from the NIRS device E6, and estimates a blood sugar level, using the NIRS information and learning information. The blood sugar level estimation device E7 may be a so-called personal computer, a smart phone, a table terminal, a server, or the like, and there is no limitation on the type thereof. The server is a so-called cloud server, an ASP server, or the like, and there is no limitation.

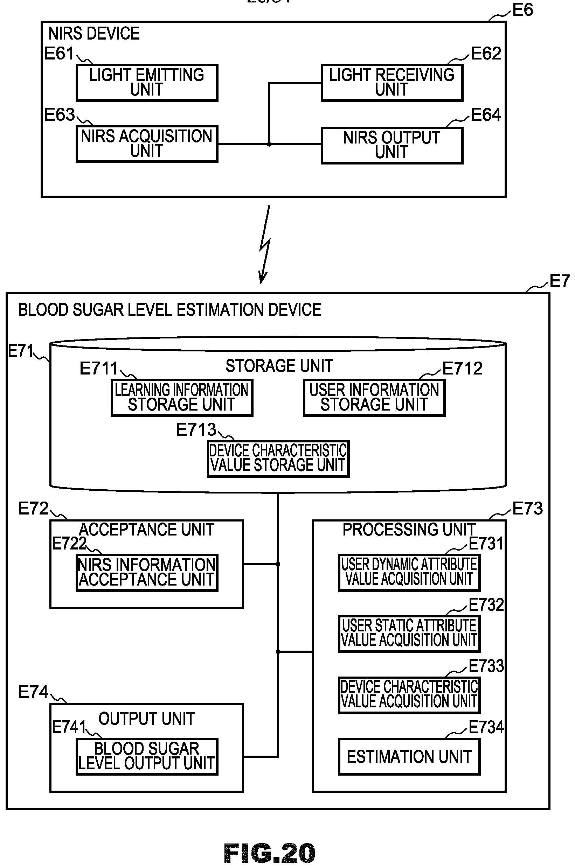

FIG. 20 is a block diagram for the blood sugar level estimation system E according to the present embodiment. The NIRS device E6 included in the blood sugar level estimation system E includes a light emitting unit E61, a light receiving unit E62, a NIRS acquisition unit E63, and a NIRS output unit E64.

The blood sugar level estimation device E7 includes a storage unit E71, an acceptance unit E72, a processing unit E73, and an output unit E74. The storage unit E71 includes a learning information storage unit E711, a user information storage unit E712, and a device characteristic value storage unit E713. The acceptance unit E72 includes a NIRS information acceptance unit E721. The processing unit E73 includes a user dynamic attribute value acquisition unit E731, a user static attribute value acquisition unit E732, a device characteristic value acquisition unit E733, and an estimation unit E734. The output unit E74 includes a blood sugar level output unit E741.

The light emitting unit E61 included in the NIRS device E6 emits near-infrared light. The light emitting unit E61 typically emits near-infrared light to a human body. For example, the light emitting unit E61 emits near-infrared light to the wrist, fingertip, earlobe, or the like of the human body. However, there is no limitation on the area to which the light emitting unit E61 emits near-infrared light.

The NIRS device E6 may include two or more light emitting units E61. It is preferable that each light emitting unit E61 emits near-infrared light with a wavelength in the range of approximately 760 nm to approximately 1300 nm. It is preferable that the two light-emitting units E61 included in the NIRS device E6 emit near-infrared light with wavelengths of, for example, approximately 1200 nm and approximately 1300 nm, respectively. One light-emitting unit E61 may emit two or more near-infrared light rays with different wavelengths. In addition, one light-emitting unit E61 may emit two or more near-infrared light rays with at different points in time. It is preferable that the wavelengths of the two or more near-infrared light rays are in the range of approximately 760 nm to approximately 1300 nm. For example, the wavelengths of the two or more near-infrared light rays include a wavelength of approximately 1200 nm and a wavelength of approximately 1300 nm. The wavelengths of the two or more near-infrared light rays include, for example, a wavelength of approximately 760 nm and a wavelength of approximately 850 nm. The wavelengths of the two or more near-infrared light rays are, for example, four wavelengths, namely approximately 760 nm, approximately 850 nm, approximately 1200 nm, and approximately 1300 nm. The light emitting unit E61 can be realized using, for example, a light-emitting diode, an LED, or the like, but may be realized using any means.

The light receiving unit E62 receives reflected light when the light-emitting unit E61 emits near-infrared light to the human body. The light receiving unit E62 may receive transmitted light when the light-emitting unit E61 emits near-infrared light to the human body. The light receiving unit E62 can be realized using a well-known device.

The NIRS acquisition unit E63 acquires one or more pieces of NIRS information that can be acquired by emitting near-infrared light to the user. The NIRS acquisition unit E63 acquires one or more pieces of NIRS information regarding the light received by the light receiving unit E62. The NIRS acquisition unit E63 typically acquires one or more pieces of NIRS information regarding the reflected light of the near-infrared light emitted by the light emitting unit E61 and the reflected light received by the light receiving unit E62.

It is preferable that the NIRS acquisition unit E63 acquires one or more pieces of NIRS information that can be acquired by emitting near-infrared light of two or more wavelengths to the user.

Note that NIRS information is, for example, an optical signal. NIRS information is, for example, a light intensity, or a set of light intensities for each wavelength. NIRS information is, for example, the intensity of the reflected light of near-infrared light with wavelengths of approximately 1200 nm and approximately 1300 nm.

The NIRS acquisition unit E63 may acquire one or more user dynamic attribute values. Examples of the one or more user dynamic attribute values include one or more pieces of information of: the skin temperature; the acceleration; the pulse; the pulse wave; the pulse rate; the saturated oxygen concentration in blood; the blood pressure; the skin color; the muscle mass; and the amount of hemoglobin in blood, of one user.

For example, the NIRS acquisition unit E63 may include one or more sensors and use such sensors to acquire one or more user dynamic attribute values. For example, the NIRS acquisition unit E63 includes a temperature sensor and acquires the user's skin temperature. For example, the NIRS acquisition unit E63 includes a gyro or acceleration sensor, and acquires the acceleration. For example, the NIRS acquisition unit E63 includes a pulse sensor, and acquires a pulse. For example, the NIRS acquisition unit E63 includes a pulse wave meter, and acquires a pulse wave. For example, the NIRS acquisition unit E63 includes a heart rate monitor, and acquires the pulse rate. For example, the NIRS acquisition unit E63 includes a blood oxygen concentration measuring device, and acquires the saturated oxygen concentration in blood. For example, the NIRS acquisition unit E63 includes a blood pressure meter, and acquires a blood pressure. For example, the NIRS acquisition unit E63 includes an image sensor, and acquires a skin color. For example, the NIRS acquisition unit E63 includes a body component analyzer, and acquires a muscle mass. For example, the NIRS acquisition unit E63 includes a hemoglobin measuring device, and acquires the amount of hemoglobin in blood.

The NIRS acquisition unit E63 may acquire one or more user static attribute values from a storage unit (not shown) of the NIRS device E6.

The NIRS acquisition unit E63 may acquire one or more device characteristic values from a storage unit (not shown) of the NIRS device E6.

The NIRS output unit E64 outputs one or more pieces of NIRS information acquired by the NIRS acquisition unit E63. The NIRS output unit E64 may output one or more user dynamic attribute values acquired by the NIRS acquisition unit E63. The NIRS output unit E64 may output one or more user static attribute values acquired by the NIRS acquisition unit E63. The NIRS output unit E64 may output one or more device characteristic values acquired by the NIRS acquisition unit E63.

The output here is typically a transmission to the blood sugar level estimation device E7. When the NIRS device E6 and the blood sugar level estimation device E7 are integrated into one device, the output is the delivery to the blood sugar level estimation device E7. The NIRS output unit E64 may accumulate one or more pieces of NIRS information or the like on a recording medium, transmits them to another device, or deliver a processing result to another processing device or another program.

The terminal storage unit E71 included in the blood sugar level estimation device E7 stores various kinds of information. Examples of the various kinds of information include learning information, which will be described later, user information, which will be described later, and device characteristic values, which will be described later.

The learning information storage unit E711 stores learning information. Learning information is information used when acquiring a blood sugar level using one or more pieces of NIRS information. Learning information is information acquired using two or more pieces of training data. Training data is information that includes one or more pieces of NIRS information and blood sugar levels. NIRS information is information acquired using reflected light of near-infrared light emitted to the human body. NIRS information may be information acquired using transmitted light as well.

Note that, in training data, for example, one or more pieces of NIRS information are explanatory variables, and the blood sugar level is an objective variable.

It is preferable that training data includes one or more user dynamic attribute values. A user dynamic attribute value is a dynamic attribute value of the user. A user dynamic attribute value is information that nay change from time to time or frequently. It is preferable that the one or more user dynamic attribute values are one or more pieces of information of: the skin temperature; acceleration; the pulse; the pulse wave; the pulse rate; the saturated oxygen concentration in blood; the blood pressure; the skin color; the muscle mass; and the amount of hemoglobin in blood, of a person with a human body.

It is preferable that training data includes one or more user static attribute values. A user static attribute value is a static attribute value of a person. The one or more user static attribute values are, for example, a sex, an age, an age group, a height, a weight, and a forearm circumference.

It is preferable that training data includes one or more device characteristic values. A device characteristic values is a characteristic value of the device (here, the NIRS device E6) used to acquire one or more pieces of NIRS information. Examples of the one or more device characteristic values include distance information specifying the distance between two or more light receiving units E62, the number of light receiving units E62, and the number of light emitting units E61.

The learning information is, for example, a learning model, a correspondence table and an arithmetic formula. Hereinafter, details of learning information will be described. It is preferable that learning information is information acquired by a learning device 9, which will be described later.

### (1) In the case where learning information is a learning model

A learning model is information acquired through machine learning processing, using two or more pieces of training data. There is no limitation on the machine learning algorithm. For example, deep learning, a decision tree, a random forest, an SVR, or the like, may be used as the machine learning algorithm, but there is no limitation on the type of machine learning. In addition, the learning model is a learning model for receiving information that includes one or more pieces of NIRS information as an input, and outputting a blood sugar level. The learning model may be referred to a classifier, a predictor, a model, or the like. There is no limitation on the module that performs machine learning processing. The module is, for example, various functions in the TensorFlow library, an R language random forest module, tinySVM, or the like.

### (2) In the case where learning information is a correspondence table

The correspondence table is information that contains two or more pieces of correspondence information that indicate correspondence between information that includes one or more pieces of NIRS information and blood sugar levels. Correspondence information may be information that includes one or more pieces of NIRS information and blood sugar levels. Correspondence information may be information that includes pointers directed to information that includes one or more pieces of NIRS information and pointers directed to blood sugar levels. Correspondence information may be any information in which information that includes one or more pieces of NIRS information and blood sugar levels are associated with each other. It is preferable that the correspondence table is information acquired by the learning device 9, which will be described later. The information that includes one or more pieces of NIRS information may be constituted only by one or more pieces of NIRS information, or information that also includes one or more kinds of information, of one or more user dynamic attribute values, one or more user static attribute values, and one or more device characteristic values.

### (3) In the case where learning information is an arithmetic formula

The arithmetic formula is an arithmetic formula having one or more pieces of NIRS information as parameters, and is used to acquire blood sugar levels. The arithmetic formula may be, for example, an arithmetic formula that also has, as parameters, one or more kinds of information, of one or more user dynamic attribute values, one or more user static attribute values, and one or more device characteristic values.

The user information storage unit E712 stores one or more pieces of user information. The user information includes one or more user static attribute values. The one or more user static attribute values are, for example, the age, age group, height, weight, and forearm circumference, of the user. The user information may include one or more user dynamic attribute values. User information may be associated with the user identifier identifying the user. The user identifier is, for example, a user ID, a mail address, a mobile phone number, or the like.

The device characteristic value storage unit E713 stores a set of one or more device characteristic values. The set of one or more device characteristic values may be associated with a user identifier.

The acceptance unit E72 accepts various kinds of instructions and information. Examples of the various kinds of instructions and information include user information, one or more user static attribute values, one or more user dynamic attribute values, and one or more device characteristic values.

For example, the acceptance unit E72 accepts one or more user static attribute values input from the user. For example, the acceptance unit E72 accepts one or more device characteristic values input from the user.

The means for inputting various kinds of instructions and information may be any means such as a touch panel, a keyboard, a mouse, a means that employs a menu screen, or the like.

For example, the acceptance unit E72 receives various kinds of instructions and information from another device. The other device is, for example, the NIRS device E6 or a user terminal device (not shown).

For example, the acceptance unit E72 receives one or more kinds of information of: one or more user dynamic attribute values; one or more user static attribute values; and one or more device characteristic values, from the NIRS device E6.

The NIRS information acceptance unit E721 accepts one or more pieces of NIRS information. For example, the NIRS information acceptance unit E721 receives one or more pieces of NIRS information from the NIRS device E6.

The processing unit E73 performs various kinds of processing. Examples of the various kinds of processing include the processing that is performed by the user dynamic attribute value acquisition unit E731, the user static attribute value acquisition unit E732, the device characteristic value acquisition unit E733, and the estimation unit E734.

The user dynamic attribute value acquisition unit E731 acquires one or more user dynamic attribute values. For example, the user dynamic attribute value acquisition unit E731 acquires the one or more user dynamic attribute values received by the acceptance unit E72. Note that the blood sugar level estimation device E7 may include one or more sensors that acquire one or more user dynamic attribute values, and acquire one or more user dynamic attribute values using such sensors. Examples of the one or more user dynamic attribute values include one or more pieces of information of: the skin temperature; the acceleration; the pulse; the pulse wave; the pulse rate; the saturated oxygen concentration in blood; the blood pressure; the skin color; the muscle mass: and the amount of hemoglobin in blood, of one user.

The user static attribute value acquisition unit E732 acquires one or more user static attribute values of the user. For example, the user static attribute value acquisition unit E732 acquires one or more user static attribute values from the user information storage unit E712. For example, the user static attribute value acquisition unit E732 acquires one or more user static attribute values corresponding to the received user identifier from the user information storage unit E712. For example, the user static attribute value acquisition unit E732 acquires one or more user static attribute values received. Note that the one or more user static attribute values are, for example, one or more pieces of information of: the age; the age group, the height; the weight; and the forearm circumference, of the user.

The device characteristic value acquisition unit E733 acquires one or more device characteristic values of a device that is used to acquire one or more pieces of NIRS information of the user. For example, the device characteristic value acquisition unit E733 acquires one or more device characteristic values from the device characteristic value storage unit E713. For example, the device characteristic value acquisition unit E733 acquires one or more device characteristic values corresponding to the received user identifier from the device characteristic value acquisition unit E733. For example, the device characteristic value acquisition unit E733 acquires one or more device characteristic values received.

The device characteristic value acquisition unit E733 may inspect the NIRS device E6 to automatically acquire one or more device characteristic values. For example, the device characteristic value acquisition unit E733 may perform image recognition on a camera image capturing the NIRS device E6 to extract the contours of the light emitting units E61, calculate the coordinates of the centers of gravity of the figures surrounded by the contours, and acquire distance information indicating the distance between the centers of gravity of the two or more contours.

Note that the one or more device characteristic values are, for example, one or more pieces of information of: distance information specifying the distance between two or more light receiving units that receive the reflected light from the near-infrared light emitting units E61; the number of light receiving units E62; and the number of light emitting unit E61.

The estimation unit E734 acquires an estimated blood sugar level, using the one or more pieces of NIRS information acquired by the NIRS acquisition unit E63 and learning information. The estimation unit E734 performs prediction processing to acquire the blood sugar level, using the one or more pieces of NIRS information acquired by the NIRS acquisition unit E63 and learning information.

For example, the estimation unit E734 acquires the estimated blood sugar level, using the one or more pieces of NIRS information acquired by the NIRS acquisition unit E63, the one or more user dynamic attribute values acquired by the user dynamic attribute value acquisition unit E731, and learning information.

For example, the estimation unit E734 acquires the estimated blood sugar level, using the one or more pieces of NIRS information acquired by the NIRS acquisition unit E63, the one or more user static attribute values acquired by the user static attribute value acquisition unit E732, and learning information.

For example, the estimation unit E734 acquires the estimated blood sugar level, using the one or more pieces of NIRS information acquired by the NIRS acquisition unit E63, one or more device characteristic values acquired by the device characteristic value acquisition unit E733, and learning information.

For example, the estimation unit E734 acquires the estimated blood sugar level, using the one or more pieces of NIRS information acquired by the NIRS acquisition unit E63, two or more kinds of information of: one or more user dynamic attribute values; one or more user static attribute values; and one or more device characteristic values, and learning information.

Hereinafter, examples of operations for prediction processing that is performed by the estimation unit E734 in the cases where learning information is a learning model, a correspondence table, and an arithmetic formula will be described.

### (1) In the case where learning information is a learning model

The estimation unit E734 provides the prediction processing module for machine learning with the one or more pieces of NIRS information acquired by the NIRS acquisition unit E63 and learning information, and executes the module to perform prediction processing and acquire an estimated blood sugar level. There is no limitation on the machine learning algorithm. Deep learning, a decision tree, a random forest, an SVR, or the like, may be used as the machine learning prediction processing algorithm, but there is no limitation on the type of machine learning. There is no limitation on the module that performs machine learning prediction processing. The module is, for example, various functions in the TensorFlow library, an R language random forest module, tinySVM, or the like.

For example, the estimation unit E734 provides the machine learning prediction processing module with the one or more pieces of NIRS information acquired by the NIRS acquisition unit E63, one or more kinds of information of one or more user dynamic attribute values acquired by the user dynamic attribute value acquisition unit E731; one or more user static attribute values acquired by the user static attribute value acquisition unit E732; and one or more device characteristic values acquired by the device characteristic value acquisition unit E733, and learning information, and executes the module to perform prediction processing, and acquires the estimated blood sugar level.

### (2) In the case where learning information is a correspondence table

The estimation unit E734 determines one or more pieces of correspondence information that satisfy an approximation condition for a vector that includes the one or more pieces of NIRS information acquired by the NIRS acquisition unit E63, in the pieces of correspondence information contained in the correspondence table, and acquires the estimated blood sugar level, using the blood sugar levels included in the one or more pieces of correspondence information. Note that the approximation condition is, for example, that the degree of approximation is the highest, that the degree of approximation is within a threshold value, or that the degree of approximation is greater than a threshold value. Note that the degree of approximation is information that takes a smaller value for a longer distance between two vectors, and is a value calculated using a decreasing function having the distance between the two vectors as a parameter, for example. Note that there is no limitation on the method for acquiring the degree of approximation between two vectors. Note that the vector that includes one or more pieces of NIRS information is, for example, a vector that includes the one or more pieces of NIRS information as elements. Alternatively, the vector that includes one or more pieces of NIRS information is, for example, a vector that includes one or more pieces of NIRS information and one or more kinds of information of: one or more user dynamic attribute values; one or more user static attribute values; and one or more device characteristic values, as elements.

For example, the estimation unit E734 acquires a blood sugar level that is closest to the vector that includes the one or more pieces of NIRS information acquired by the NIRS acquiring unit E63 from the correspondence table. Note that the blood sugar level is the estimated blood sugar level.

For example, the estimation unit E734 acquires a representative value of the two or more blood sugar levels included in the correspondence information that has a vector that satisfies the approximation condition with respect to the degree of approximation to the vector that includes the one or more pieces of NIRS information acquired by the NIRS acquisition unit E63. The representative value is the estimated blood sugar level. The representative value is, for example, an average value, a median value, or a weighted average value. A weighted average value is a value acquired by giving a larger weight to a shorter distance between two vectors.

### (3) In the case where learning information is an arithmetic formula

The estimation unit E734 provides the arithmetic formula with the one or more pieces of NIRS information acquired by the NIRS acquisition unit E63, and executes the arithmetic formula to acquire an estimated blood sugar level.

The estimation unit E734 provides the arithmetic formula with the one or more pieces of NIRS information acquired by the NIRS acquisition unit E63, and one or more kinds of information of the one or more user dynamic attribute values acquired by the user dynamic attribute value acquisition unit E731; the one or more user static attribute values acquired by the user static attribute value acquisition unit E732; and the one or more device characteristic values acquired by the device characteristic value acquisition unit E733, and executes the arithmetic formula to acquire an estimated blood sugar level.

The output unit E74 outputs various kinds of information. Examples of the various kinds of information include a blood sugar level. Here, the output is a concept that encompasses display on a display screen, projection using a projector, printing by a printer, the output of a sound, transmission to an external device, accumulation on a recording medium, delivery of a processing result to another processing device or another program, and the like.

For example, the blood sugar level output unit E741 outputs the blood sugar level acquired by the estimation unit E734. The blood sugar level output unit E741 may output the blood sugar level acquired by the estimation unit E734 or blood sugar level-related information regarding the blood sugar level, to the NIRS device E6 or a terminal device corresponding to the NIRS device E6. Note that the terminal device is, for example, a smart phone or a table terminal.

The NIRS acquisition unit E63, the processing unit E73, the user dynamic attribute value acquisition unit E731, the user static attribute value acquisition unit E732, the device characteristic value acquisition unit E733, and the estimation unit E734 can typically be realized using a processor, a memory, or the like. The processing procedures performed by the processing unit E73 or the like typically are realized using software, and the software is recorded on a recording medium such as a ROM. However, such processing procedures may be realized using hardware (a dedicated circuit). Note that the processor is a CPU, an MPU, a GPU, or the like, and there is no limitation on the type thereof.

For example, the NIRS output unit E64 can be realized using a wireless or wired communication means.

It is preferable that the storage unit E71, the learning information storage unit E711, the user information storage unit E712, and the device characteristic value storage unit E713 are non-volatile recording media, but may be realized using volatile recording media.

There is no limitation on the process in which information is stored in the storage unit E71 or the like. For example, information may be stored in the storage unit storage unit E71 or the like via a recording medium, or information transmitted via a communication line or the like may be stored in the storage unit E71 or the like, or information input via an input device may be stored in the storage unit E71 or the like.

For example, the acceptance unit E72 and the NIRS information acceptance unit E721 can be realized using a wireless or wired communication means. The acceptance unit E72 can be realized using a device driver for the input means such as a touch panel or a keyboard, or control software or the like for controlling the menu screen.

For example, the output unit E74 and the blood sugar level output unit E741 can be realized using a wireless or wired communication means. The output unit E74 and the blood sugar level output unit E741 may be realized using an output device such as a display or a speaker. The output unit E74 and the blood sugar level output unit E741 can be realized using the driver software of the output device, the driver software of the output device and the output device, or the like.

Next, examples of the operations of the blood sugar level estimation system E will be described. First, examples of operations of the NIRS device E6 will be described.

Each of the one or more light emitting units E61 included in the NIRS device E6 emits near-infrared light. Each of the one or more light receiving units E62 receives the reflected near-infrared light. Next, the NIRS acquisition unit E63 acquires one or more pieces of NIRS information from the received optical signal. For example, the NIRS acquisition unit E63 acquires one or more user dynamic attribute values. For example, the NIRS acquisition unit E63 acquires one or more user static attribute values. For example, the NIRS acquisition unit E63 acquires one or more device characteristic values. Next, the NIRS output unit E64 passes the one or more pieces of NIRS information or the like acquired by the NIRS acquisition unit E63 to the blood sugar level estimation device E7. Note that the one or more pieces of NIRS information or the like may be only one or more pieces of NIRS information, or one or more pieces of NIRS information and one or more kinds of information of: one or more user dynamic attribute values; one or more user static attribute values; and one or more device characteristic values. Note that, for example, the NIRS output unit E64 transmits one or more pieces of NIRS information or the like to the blood sugar level estimation device E7.

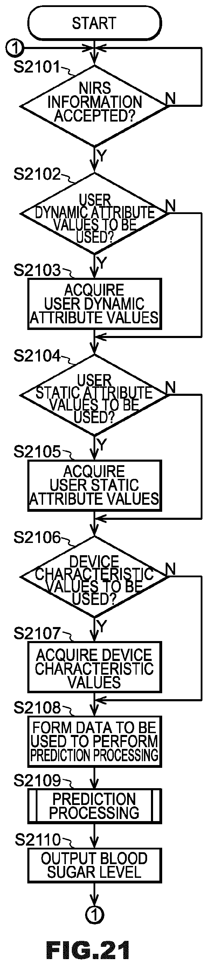

Next, examples of operations of the blood sugar level estimation device E7 will be described with reference to the flowchart in FIG. 21.

(Step S2101) The NIRS information acceptance unit E721 judges whether or not one or more pieces of NIRS information have been accepted. If one or more pieces of NIRS information have been accepted, processing proceeds to step S2102, and if one or more pieces of NIRS information have not been accepted, processing returns to step S2101. Here, the NIRS information acceptance unit E721 may accept one or more kinds of information of: one or more user dynamic attribute values; one or more user static attribute values; and one or more device characteristic values.

(Step S2102) The processing unit E73 judges whether or not to use the user dynamic attribute values when acquiring a blood sugar level. If the user dynamic attribute values are to be used, processing proceeds to step S2103, and if the user dynamic attribute values are not to be used, processing proceeds to step S2104. Note that whether or not the user dynamic attribute values are to be used may be determined in advance, or judged based on a user instruction or the like, or judged based on whether or not the user dynamic attribute values can be acquired.

(Step S2103) The user dynamic attribute value acquisition unit E731 acquires one or more user dynamic attribute values.

(Step S2104) The processing unit E73 judges whether or not to use the user static attribute values when acquiring a blood sugar level. If the user static attribute values are to be used, processing proceeds to step S2105, and if the user static attribute values are not to be used, processing proceeds to step S2106. Note that whether or not the user static attribute values are to be used may be determined in advance, or judged based on a user instruction or the like, or judged based on whether or not the user static attribute values are stored.

(Step S2105) The user static attribute value acquisition unit E732 acquires one or more user static attribute values.

(Step S2106) The processing unit E73 judges whether or not to use the device characteristic values when acquiring a blood sugar level. If the device characteristic values are to be used, processing proceeds to step S2107, and if the device characteristic values are not to be used, processing proceeds to step S2108. Note that whether or not the device characteristic values are to be used may be determined in advance, or judged based on a user instruction or the like, or judged based on whether or not the device characteristic values are stored.

(Step S2107) The device characteristic value acquisition unit E733 acquires one or more device characteristic values.

(Step S2108) The estimation unit E734 forms data that is used to perform prediction processing. The estimation unit E734 acquires a vector that is to be provided in machine learning together with the learning model. The data to be used to perform prediction processing may only be one or more pieces of NIRS information. The data to be used to perform prediction processing may be one or more pieces of NIRS information and information that includes one or more kinds of information of: one or more user dynamic attribute values; one or more user static attribute values; and one or more device characteristic values.

(Step S2109) The estimation unit E734 performs prediction processing, using the data formed in step S2108 and the learning information, to acquire a blood sugar level. Note that an example of the prediction processing is described above. Note that the estimation unit E734 is

(Step S2110) The blood sugar level output unit E741 outputs the blood sugar level acquired in step S2109. Processing returns to step S2101. For example, the blood sugar level output unit E741 transmits the blood sugar level to an external device such as the NIRS device E6.

Note that, in the flowchart in FIG. 21, processing ends when the power is turned off or an interruption occurs to end the processing.

Hereinafter, specific examples of operations of the blood sugar level estimation system E according to the present embodiment will be described. A conceptual diagram for the blood sugar level estimation system E is shown in FIG. 19.

It is now assumed that a learning model acquired using a machine learning algorithm (for example, a random forest) is stored in the learning information storage unit E711 of the blood sugar level estimation device E7, which is a smart phone. It is assumed that this learning model is a learning model acquired by the learning device 9, which will be described later, using a large amount of training data that contains an explanatory variable including one or more pieces of NIRS information and an objective variable that is a blood sugar level. In addition, it is assumed that the one or more pieces of NIRS information here are the light intensities of the reflected near-infrared light rays of different wavelengths. Here, it is assumed that NIRS information is a four-dimensional vector (the intensity of the reflected light of near-infrared light with a wavelength of 760 nm, the intensity of the reflected light of near-infrared light with a wavelength of 850 nm, the intensity of the reflected light of near-infrared light with a wavelength of 1200 nm, the intensity of the reflected light of near-infrared light with a wavelength of 1300 nm). In addition, it is assumed that training data includes one or more user static attribute values, one or more user dynamic attribute values, and one or more device characteristic values. Here, it is assumed that the one or more user static attribute values are a sex, an age, a height, a weight, and a forearm circumference. In addition, it is assumed that the one or more user dynamic attribute values are: the skin temperature; the acceleration; the pulse; the pulse wave; the pulse rate; the saturated oxygen concentration in blood; the blood pressure; the skin color; the muscle mass; and the amount of hemoglobin in blood, of a person. Furthermore, it is assumed that the one or more device characteristic values are pieces of distance information specifying the distance between two light receiving units E62. That is to say, it is assumed that the structure of training data is "<explanatory variables> (the intensity of the reflected light of near-infrared light with a wavelength of 760 nm, the intensity of the reflected light of near-infrared light with a wavelength of 850 nm, the intensity of the reflected light of near-infrared light with a wavelength of 1200 nm, the intensity of the reflected light of near-infrared light with a wavelength of 1300 nm), sex, age, height, weight, forearm circumference, skin temperature, acceleration, pulse, pulse wave, pulse rate, saturated oxygen concentration in blood, blood pressure, skin color, muscle mass, amount of hemoglobin in blood, distance information regarding the light receiving units E62),<objective variable> blood sugar level".

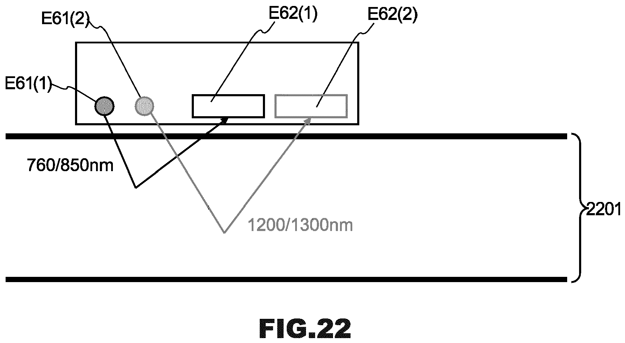

In addition, here, as shown in FIG. 22, it is assumed that the NIRS device E6 includes a light emitting unit E61(1), a light emitting unit E61(2), and two light receiving units E62 respectively corresponding to the light emitting units E61. The light emitting unit E61(1) emits near-infrared light with a wavelength of 760 nm and near-infrared light with a wavelength of 850 nm. The light emitting unit E61(2) emits near-infrared light with a wavelength of 1200 nm and near-infrared light with a wavelength of 1300 nm. The light receiving unit E62(1) receives the reflected light of the near-infrared light emitted from the light emitting unit E61(1). The light receiving unit E62(2) receives the reflected light of the near-infrared light emitted from the light emitting unit E61(2). In such a case, the NIRS information to be used by the blood sugar level estimation device E7 to estimate a blood sugar level is a four-dimensional vector (the intensity of the reflected light of near-infrared light with a wavelength of 760 nm, the intensity of the reflected light of near-infrared light with a wavelength of 850 nm, the intensity of the reflected light of near-infrared light with a wavelength of 1200 nm, the intensity of the reflected light of near-infrared light with a wavelength of 1300 nm). Note that 2201 in FIG. 22 indicates an internal tissue of a human body. An upper portion of 2201 is closer to the skin surface, and a lower portion is a deeper portion.

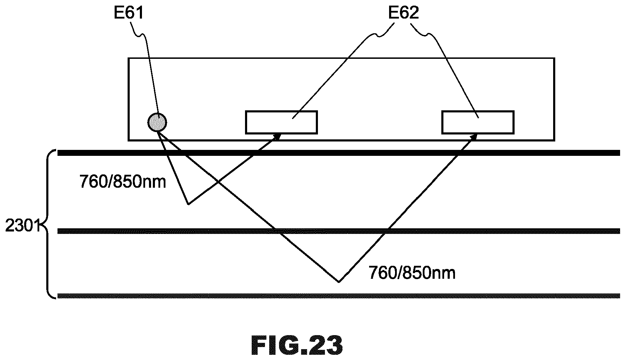

Note that the light emitting unit E61 and the light receiving units E62 of the NIRS device E6 may be formed as shown in FIG. 23. In FIG. 23, multiple reflected light rays of the near-infrared light emitted from one light emitting unit E61, each having a different path length, are received by the light receiving unit E62(2). Although the multiple reflected light rays with different optical path lengths are constituted by two reflected light rays, it may be constituted by three or more reflected light rays. The light emitting unit E61 in FIG. 23 emits near-infrared light with a wavelength of 760 nm and near-infrared light with a wavelength of 850 nm. In such a case, the NIRS information to be used by the blood sugar level estimation device E7 to estimate a blood sugar level is a four-dimensional vector (the intensity of the reflected light of near-infrared light of a short optical path length with a wavelength of 760 nm, the intensity of the reflected light of near-infrared light of a short optical path length with a wavelength of 850 nm, the intensity of the reflected light of near-infrared light of a long optical path length with a wavelength of 1200 nm, the intensity of the reflected light of near-infrared light of a long optical path length with a wavelength of 1300 nm). Note that 2301 in FIG. 23 indicates an internal tissue of a human body. An upper portion of 2301 is closer to the skin surface, and a lower portion is a deeper portion.

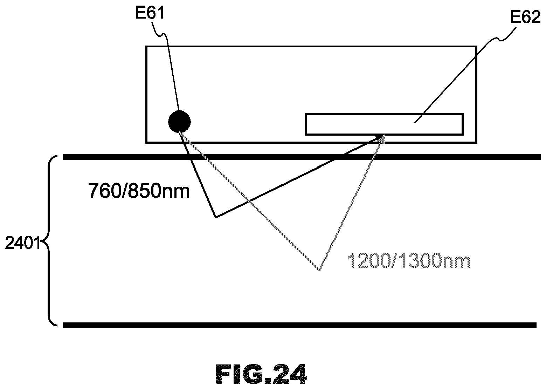

Note that the light emitting unit E61 and the light receiving unit E62 of the NIRS device E6 may be formed as shown in FIG. 24. In FIG. 24, one light emitting unit E61 and one light receiving unit E62 are present. The light emitting unit E61 emits near-infrared light of multiple different wavelengths. Here, the light emitting unit E61 emits near-infrared light with a wavelength of 760 nm, near-infrared light with a wavelength of 850 nm, near-infrared light with a wavelength of 1200 nm, and near-infrared light with a wavelength of 1300 nm. In such a case, the NIRS information to be used by the blood sugar level estimation device E7 to estimate a blood sugar level is a four-dimensional vector (the intensity of the reflected light of near-infrared light with a wavelength of 760 nm, the intensity of the reflected light of near-infrared light with a wavelength of 850 nm, the intensity of the reflected light of near-infrared light with a wavelength of 1200 nm, the intensity of the reflected light of near-infrared light with a wavelength of 1300 nm). Note that 2401 in FIG. 24 indicates an internal tissue of a human body. An upper portion of 2401 is closer to the skin surface, and a lower portion is a deeper portion.

In FIGS. 22 to 24, it is preferable that each of the light emitting units E61 constantly emits near-infrared light with two or more wavelengths, but may emit light in a time division manner.

In addition, it is assumed that the NIRS device E6 here includes various sensors such as a temperature sensor and an acceleration sensor, and can acquire the one or more user dynamic attribute values described above.

Here, it is also assumed that the one or more user static attribute values described above are stored in the user information storage unit E712 in the blood sugar level estimation device E7. In addition, it is assumed that the one or more device characteristic values described above are stored in the device characteristic values storage unit E713 in the blood sugar level estimation device E7.

In such a situation, for example, it is assumed that near-infrared light rays of four wavelengths, namely approximately 760 nm, approximately 850 nm, approximately 1200 nm, and approximately 1300 nm are emitted from one or more light emitting units E61 of the wristwatch-type NIRS device E6 to the wrist region of a human body. One or more light receiving units E62 included in the NIRS device E6 receive the reflected light rays with the respective wavelengths. Next, the NIRS acquisition unit E63 acquires one or more pieces of NIRS information. Here, the one or more pieces of NIRS information is constituted by a four-dimensional vector (the intensity of reflected near-infrared light with a wavelength of 760 nm, the intensity of reflected near-infrared light with a wavelength of 850 nm, the intensity of reflected near-infrared light with a wavelength of 1200 nm, the intensity of reflected near-infrared light with a wavelength of 1300 nm).

Next, the NIRS output unit E64 transmits four pieces of NIRS information and one or more user dynamic attribute values to the blood sugar level estimation device E7.

Next, the acceptance unit E72 in the blood sugar level estimation device E7 receives the four pieces of NIRS information and the one or more user dynamic attribute values from the NIRS device E6.

Next, the user static attribute value acquisition unit E732 acquires one or more user static attribute values stored in the user information storage unit E712.

In addition, the device characteristic value acquisition unit E733 acquires one or more device characteristic values from the device characteristic value storage unit E713.

Next, the estimation unit E734 forms data to be provided to machine learning prediction module, using the four pieces of NIRS information and one or more user dynamic attribute values thus received, the one or more user static attribute values thus acquired, and the one or more device characteristic values thus acquired. The data structure of such data is as follows: (the intensity of the reflected light of near-infrared light with a wavelength of 760 nm, the intensity of the reflected light of near-infrared light with a wavelength of 850 nm, the intensity of the reflected light of near-infrared light with a wavelength of 1200 nm, the intensity of the reflected light of near-infrared light with a wavelength of 1300 nm, sex, age, height, weight, forearm circumference, skin temperature, acceleration, pulse, pulse wave, pulse rate, saturated oxygen concentration in blood, blood pressure, skin color, muscle mass, amount of hemoglobin in blood, distance information regarding the light receiving units E62).

Next, the estimation unit E734 provides the formed data and the leaner in the learning information storage unit E711 to the machine learning predication module (for example, a random forest prediction module), and executes the module and acquires the estimated blood sugar level.

Next, the blood sugar level output unit E741 outputs the acquired blood sugar level.

As described above, according to the present embodiment, an appropriate human blood sugar level can be acquired non-invasively.

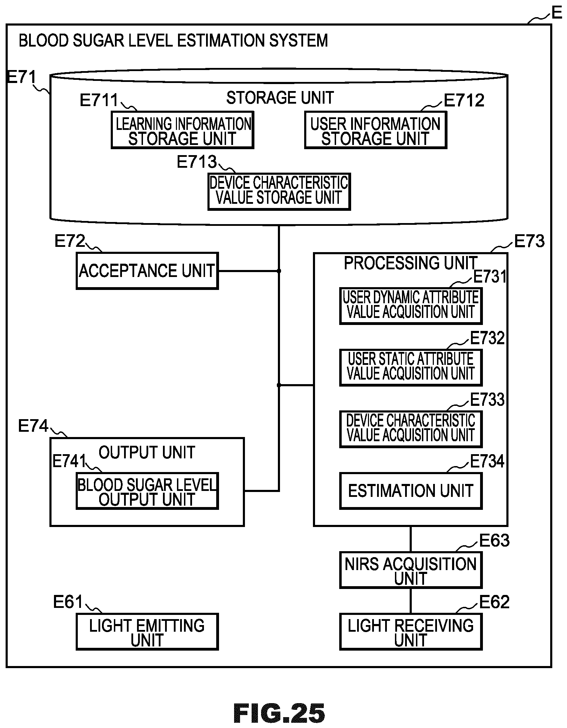

Note that, in the present embodiment, the NIRS device E6 and the blood sugar level estimation device E7 may be integrated into a single device. In such a case, an example of block diagram for the blood sugar level estimation system E in which the NIRS device E6 and the blood sugar level estimation device E7 are integrated is shown in FIG. 25. In FIG. 25, multiple light emitting units E61 and multiple light receiving units E62 may be provided.

In addition, in the present embodiment, the number of near-infrared light rays with two or more different wavelengths used to acquire learning information may be greater than the number of near-infrared light rays with two or more different wavelengths used in the processing performed to estimate the blood sugar level.

Furthermore, the processing in the present embodiment may be realized using software. This software may be distributed through software downloading or the like. Also, this software may be recorded on a recording medium such as a CD-ROM and distributed. Note that the same applies to the other embodiments in the present description. The software that realizes the blood sugar level estimation device E7 according to the present embodiment is the program described below. That is to say, this program is a program that enables a computer that can access a learning information storage unit that stores learning information acquired using two or more pieces of training data that each include one or more pieces of NIRS information acquired using a reflected light of a near-infrared light emitted to a human body, and a blood sugar level, to function as: a NIRS acquisition unit that acquires one or more pieces of NIRS information that can be acquired by emitting near-infrared light to a user; an estimation unit that acquires an estimated blood sugar level, using the one or more pieces of NIRS information acquired by the NIRS acquisition unit and the learning information; and a blood sugar level output unit that outputs the blood sugar level acquired by the estimation unit.

### Sixth Embodiment

The present embodiment is different from the fifth embodiment in that NIRS devices and a blood sugar level estimation device are in a many-to-1 relationship. That is to say, in the present embodiment, each user has a NIRS device. The blood sugar level estimation device functions as a server that receives pieces of NIRS information regarding one or more users from the NIRS devices, estimates a blood sugar level, and transmits the blood sugar level. Note that the blood sugar level may be transmitted to the NIRS devices or another device.

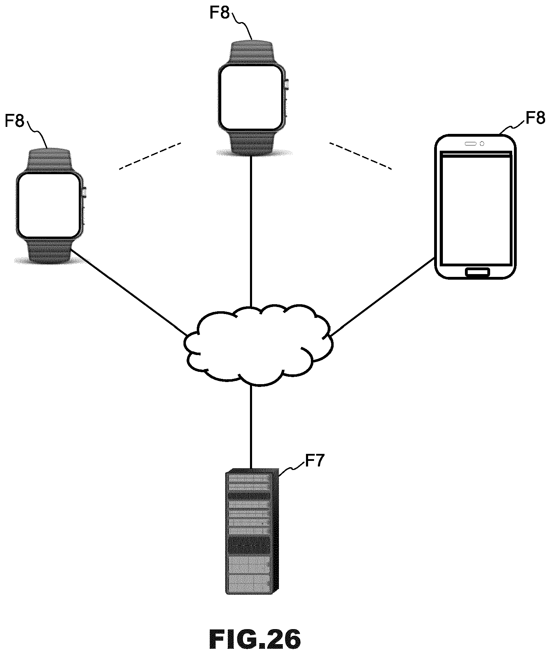

FIG. 26 is a block diagram for the blood sugar level estimation system F according to the present embodiment. The blood sugar level estimation system F includes one or more NIRS devices F8 and a blood sugar level estimation device F7. Each of the NIRS devices F8 has the function of receiving a blood sugar level from the NIRS device F8 and outputs the blood sugar level, in addition to the configuration of the NIRS device E6. Note that each NIRS device F8 included in the blood sugar level estimation system F may have the same configuration as the NIRS device E6. The blood sugar level estimation device F7 is a so-called server. The server is a cloud server, an ASP server, or the like, and there is no limitation. Each of the one or more NIRS devices F8 and the blood sugar level estimation device F7 can communicate with each other via a network such as the Internet or a LAN.

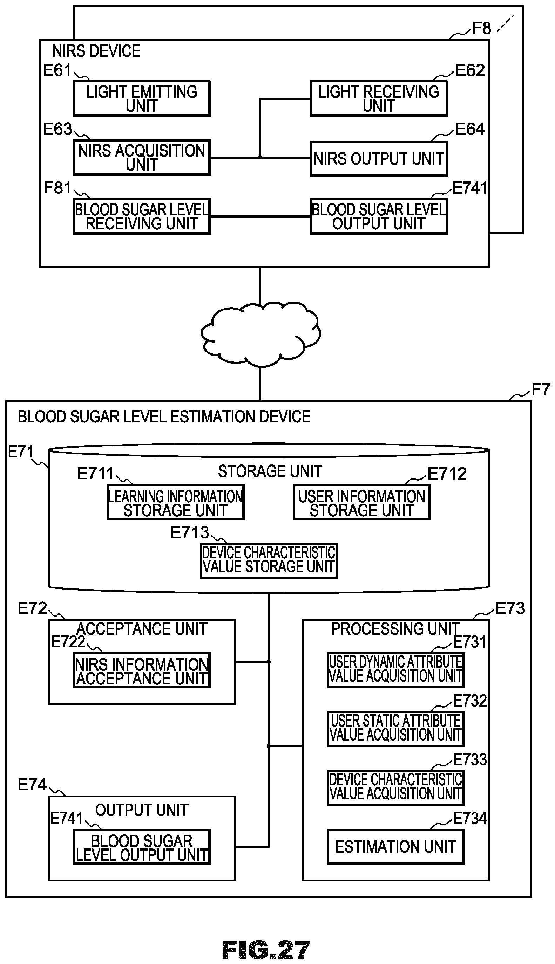

FIG. 27 is a block diagram for the blood sugar level estimation system F according to the present embodiment. Each of the NIRS devices F8 included in the blood sugar level estimation system F includes the light emitting unit E61, the light receiving unit E62, the NIRS acquisition unit E63, the NIRS output unit E64, a blood sugar level reception unit F81, and the blood sugar level output unit E741.

The NIRS output unit E64 in the NIRS device F8 outputs one or more pieces of NIRS information acquired by the NIRS acquisition unit E63 to the blood sugar level estimation device F7. The NIRS output unit E64 may transmit one or more kinds of information of: one or more user dynamic attribute values; one or more user static attribute values; and one or more device characteristic values, to the blood sugar level estimation device F7. The NIRS output unit E64 may transmit the user identifier identifying the user to the blood sugar level estimation device F7. The NIRS output unit E64 can be realized using a wireless or wired communication means.

The blood sugar level reception unit F81 receives the estimated blood sugar level from the blood sugar level estimation device F7. The blood sugar level reception unit F81 can be realized using a wireless or wired communication means.

The blood sugar level output unit E741 in each NIRS device F8 outputs the blood sugar level received by the blood sugar level reception unit F81. Here, the output typically is displaying on a display screen, but may be a concept that encompasses projection using a projector, printing by a printer, the output of a sound, transmission to an external device, accumulation on a recording medium, delivery of a processing result to another processing device or another program, and the like. The blood sugar level output unit E741 may be regarded as including or not including an output device such as a display or a speaker. The blood sugar level output unit E741 can be realized using the driver software of the output device, the driver software of the output device and the output device, or the like.

The learning information storage unit E711 in the blood sugar level estimation device F7 may store learning information for each set of explanatory variables to be used. That is to say, the learning information storage unit E711 may store the learning information in the case where the explanatory variables are only one or more pieces of NIRS information, or the learning information in the case where the explanatory variables are one or more pieces of NIRS information and one or more kinds of information of: one or more user dynamic attribute values; one or more user static attribute value; and one or more device characteristic values. In addition, the learning information storage unit E711 may store learning information in association with user identifiers.

It is preferable that the user information storage unit E712 stores user information in association with user identifiers. The user information includes one or more kinds of information of: one or more user static attribute values; and one or more user dynamic attribute values.

It is preferable that the device characteristic value storage unit E713 stores one or more device characteristic values in association with user identifiers.

The NIRS information acceptance unit E721 in the blood sugar level estimation device F7 receives one or more pieces of NIRS information transmitted by each NIRS device F8. The NIRS information acceptance unit E721 may also receive one or more user dynamic attribute values, one or more user static attribute values, and one or more device characteristic values transmitted by each NIRS device F8. The NIRS information acceptance unit E721 may receive user identifiers. The NIRS information acceptance unit E721 can be realized using a wireless or wired communication means.

The blood sugar level output unit E741 in the blood sugar level estimation device F7 transmits the blood sugar level acquired by the estimation unit E734. It is preferable that the blood sugar level is transmitted to the NIRS device F8, but may be transmitted to another device. The other device is, for example, a user's terminal device (not shown). The terminal device is, for example, a smart phone, a table terminal, a so-called personal computer, or the like, but there is no limitation on the type thereof.

Next, examples of operations of the blood sugar level estimation system F will be described. First, examples of operations of each NIRS device F8 will be described.

One or more light emitting units E61 included in the NIRS device F8 emit near-infrared light. One or more light receiving units E62 receive reflected light of near-infrared light. Next, the NIRS acquisition unit E63 acquires one or more pieces of NIRS information from the received optical signal. The NIRS acquisition unit E63 also acquires one or more user dynamic attribute values, for example. The NIRS output unit E64 transmits the one or more pieces of NIRS information acquired by the NIRS acquisition unit E63 to the blood sugar level estimation device F7. Here, the NIRS output unit E64 may transmit one or more user dynamic attribute values or the user identifier to the blood sugar level estimation device F7. Note that the user identifier is stored in a storage unit (not shown) in the NIRS device E6.

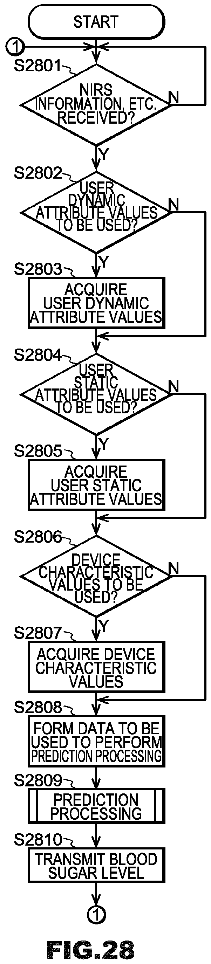

Next, examples of operations of the blood sugar level estimation device F7 will be described with reference to the flowchart in FIG. 28.

(Step S2801) The NIRS information acceptance unit E721 judges whether or not NIRS information and so on have been received. If NIRS information and so on have been received, processing proceeds to step S2802, and if NIRS information and so on have not been received, processing returns to step S2801. Note that NIRS information and so on include one or more pieces of NIRS information. For example, NIRS information and so on may include one or more user dynamic identifiers, one or more user static identifiers and one or more device characteristic values.

(Step S2802) The processing unit E73 judges whether or not to use the user dynamic attribute values when acquiring a blood sugar level. If the user dynamic attribute values are to be used, processing proceeds to step S2803, and if the user dynamic attribute values are not to be used, processing proceeds to step S2804. Note that whether or not to use user dynamic attribute values may be determined in advance, or judged based on a user instruction or the like, or judged based on whether or not user dynamic attribute values can be acquired.

(Step S2803) The user dynamic attribute value acquisition unit E731 acquires one or more user dynamic attribute values. For example, the user dynamic attribute value acquisition unit E731 acquires one or more user dynamic attribute values that have been received.

(Step S2804) The processing unit E73 judges whether or not to use the user static attribute values when acquiring a blood sugar level. If the user static attribute values are to be used, processing proceeds to step S2805, and if the user static attribute values are not to be used, processing proceeds to step S2806. Note that whether or not to use user static attribute values may be determined in advance, or judged based on a user instruction or the like, or judged based on whether or not user static attribute values are stored.

(Step S2805) The user static attribute value acquisition unit E732 acquires one or more user static attribute values. For example, the user static attribute value acquisition unit E732 acquires one or more user static attribute values corresponding to the received user identifier, from the user information storage unit E712.

(Step S2806) The processing unit E73 judges whether or not to use device characteristic values when acquiring a blood sugar level. If the device characteristic values are to be used, processing proceeds to step S2807, and if the device characteristic values are not to be used, processing proceeds to step S2808. Note that whether or not to use device characteristic values may be determined in advance, or judged based on a user instruction or the like, or judged based on whether or not device characteristic values are stored.

(Step S2807) The device characteristic value acquisition unit E733 acquires one or more device characteristic values. For example, the device characteristic value acquisition unit E733 acquires one or more device characteristic values corresponding to the received user identifier from the device characteristic value storage unit E713.

(Step S2808) The estimation unit E734 forms data to be used to perform prediction processing. For example, the estimation unit E734 acquires a vector that is to be provided in machine learning together with a learning model.

(Step S2809) The estimation unit E734 performs prediction processing using the data formed in step S2808 and the learning information in the learning information storage unit E711, to acquire a blood sugar level. Note that an example of prediction processing is described above.

(Step S2810) The blood sugar level output unit E741 transmits the blood sugar level acquired in step S2809. Processing returns to step S2801. For example, the blood sugar level output unit E741 transmits the blood sugar level to the NIRS device F8.

Note that, in the flowchart in FIG. 28, processing ends when the power is turned off or an interruption occurs to end the processing.

As described above, according to the present embodiment, it is possible to provide a server that can non-invasively acquire appropriate blood sugar levels of two or more users.

The software that realizes the NIRS device F8 according to the present embodiment is the program described below. That is to say, this program is a program for enabling a computer to function as: a NIRS acquisition unit that acquires one or more pieces of NIRS information that can be acquired by emitting near-infrared light to a user; a NIRS output unit that transmits the one or more pieces of NIRS information to a blood sugar level estimation device; a blood sugar level reception unit that receives a blood sugar level in response to the one or more pieces of NIRS information being transmitted; and a blood sugar level output unit that outputs the blood sugar level.

The software that realizes the blood sugar level estimation device F7 according to the present embodiment is the program described below. That is to say, this program is a program for enabling a computer to function as: a NIRS information acceptance unit that receives one or more pieces of NIRS information that can be acquired by emitting near-infrared light to a user; an estimation unit that acquires an estimated blood sugar level, using the one or more pieces of NIRS information acquired by the NIRS acquisition unit and the learning information; and a blood sugar level output unit that transmits the blood sugar level acquired by the estimation unit.

### Seventh Embodiment

The present embodiment describes a learning system that includes a learning device that acquires learning information.

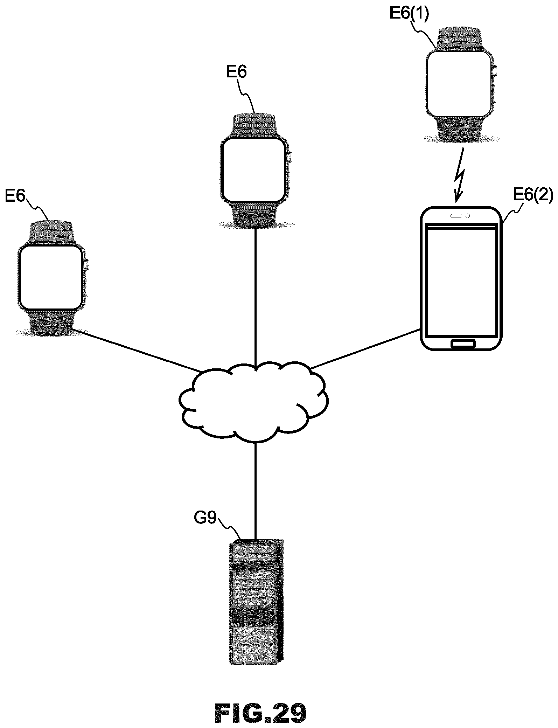

FIG. 29 is a block diagram for a learning system E according to the present embodiment. The learning system G includes one or more NIRS devices E6 and a learning device G9. Note that the functions of the NIRS device E6 may be shared by two or more devices as represented as the NIRS device E6(1) and the NIRS device E6(2) in FIG. 29. It is preferable that the NIRS device E6 is a device used by two or more users.

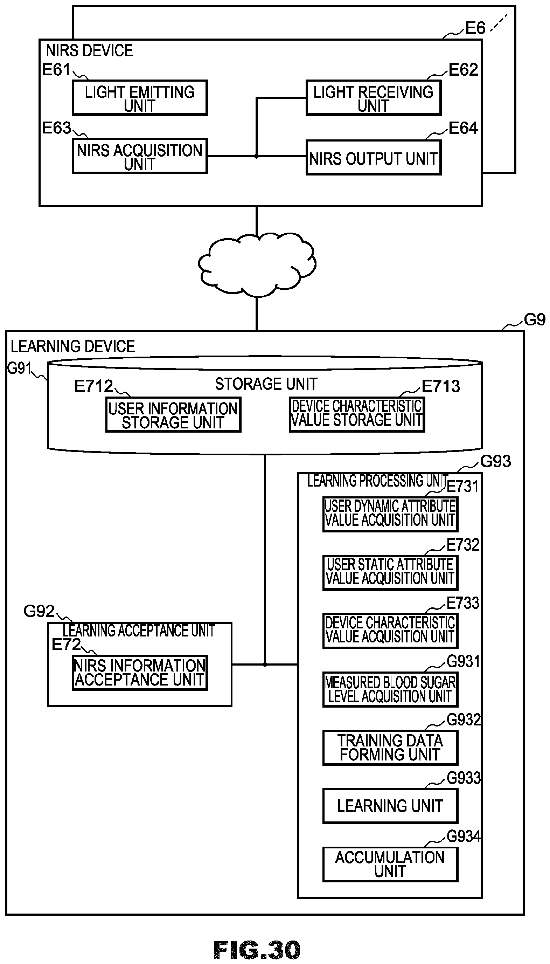

FIG. 30 is a block diagram for the learning system G according to the present embodiment. The NIRS device E6 included in the learning system G includes a light emitting unit E61, a light receiving unit E62, a NIRS acquisition unit E63, and a NIRS output unit E64.

The learning device G9 includes a learning storage unit G91, a learning acceptance unit G92, and a learning processing unit G93. The learning storage unit G91 includes the user information storage unit E712 and the device characteristic value storage unit E713.

The learning acceptance unit G92 includes the NIRS information acceptance unit E721. The learning processing unit G93 includes the user dynamic attribute value acquisition unit E731, the user static attribute value acquisition unit E732, the device characteristic value acquisition unit E733, a measured blood sugar level acquisition unit G931, a training data forming unit G932, a learning unit G933, and an accumulation unit G934.

The NIRS acquisition unit E63 in the NIRS device E6 acquires one or more pieces of NIRS information. The NIRS acquisition unit E63 here may acquire one or more user dynamic attribute values, one or more user static attribute values and one or more device characteristic values.

The NIRS acquisition unit E63 may acquire a measured blood sugar level (hereinafter referred to as the "measured blood sugar level" as appropriate). The measured blood sugar level is, for example, information acquired using a well-known blood sugar measurer. However, there is no limitation on the method for acquiring the measured blood sugar level. Note that the measured blood sugar level typically is an accurate blood sugar level.

The NIRS output unit E64 transmits the one or more pieces of NIRS information acquired by the NIRS acquisition unit E63 to the learning device G9. The NIRS output unit E64 here may also transmit one or more user dynamic attribute values, one or more user static attribute values, and one or more device characteristic values acquired by the NIRS acquisition unit E63 to the learning device G9. The NIRS output unit E64 may transmit the user identifier to the learning device G9. The NIRS output unit E64 may also transmit the measured blood sugar level to the learning device G9.

The learning storage unit G91 included in the learning device G9 stores various kinds of information. Examples of the various kinds of information include user information and device characteristic values. It is preferable that the user information and the device characteristic values are associated with the user identifier.

The learning acceptance unit G92 receives various kinds of information. The learning acceptance unit G92 receives various kinds of information from the NIRS device E6. Examples of the various kinds of information include one or more pieces of NIRS information. Examples of the various kinds of information include one or more user dynamic attribute values, one or more user static attribute values, one or more device characteristic values, and a measured blood sugar level.

The learning processing unit G93 performs various kinds of processing. The various kinds of processing is the processing that is performed by the user dynamic attribute value acquisition unit E731, the user static attribute value acquisition unit E732, the device characteristic value acquisition unit E733, the measured blood sugar level acquisition unit G931, the training data forming unit G932, the learning unit G933, and the accumulation unit G934.

For example, the user dynamic attribute value acquisition unit E731 acquires the one or more user dynamic attribute values received by the learning acceptance unit G92. For example, the user dynamic attribute value acquisition unit E731 acquires the one or more user dynamic attribute values that are paired with the user identifier received by the learning acceptance unit G92, from the user information storage unit E712.

For example, the user static attribute value acquisition unit E732 acquires the one or more user static attribute values received by the learning acceptance unit G92. For example, the user static attribute value acquisition unit E732 acquires the one or more user static attribute values that are paired with the user identifier received by the learning acceptance unit G92, from the user information storage unit E712.

For example, the device characteristic value acquisition unit E733 acquires the one or more device characteristic values received by the learning acceptance unit G92. For example, the device characteristic value acquisition unit E733 acquires the one or more device characteristic values that are paired with the user identifier received by the learning acceptance unit G92, from the device characteristic value storage unit E713.

The measured blood sugar level acquisition unit G931 acquires the user's measured blood sugar levels. In addition, for example, the measured blood sugar level acquisition unit G931 acquires the measured blood sugar levels received by the learning acceptance unit G92.

The training data forming unit G932 forms training data, using one or more pieces of NIRS information and measured blood sugar levels.

It is preferable that the training data forming unit G932 forms training data, using one or more pieces of NIRS information and measured blood sugar levels, and one or more kinds of information of: one or more user dynamic attribute values; one or more user static attribute values; and one or more device characteristic values.

The training data forming unit G932 typically acquires two or more pieces of training data.

The learning unit G933 acquires learning information, using the training data acquired by the training data forming unit G932. The learning unit G933 typically acquires learning information, using the two or more pieces of training data acquired by the training data forming unit G932.

The learning unit G933 acquires learning information, using any of the methods shown in (1) to (3) below.

### (1) In the case where learning information is a learning model

The learning unit G933 provides the module that performs machine learning processing with the two or more pieces of training data acquired by the training data forming unit G932, and executes the module to acquire a learning model. For example, deep learning, a decision tree, a random forest, an SVR, or the like, may be used as the algorithm for performing machine learning processing, but there is no limitation on the type of machine learning. The learning model is a learning model for receiving one or more pieces of NIRS information or the like input thereto, and outputting a blood sugar level. There is no limitation on the module that performs machine learning processing. The module is, for example, various functions in the TensorFlow library, an R language random forest module, tinySVM, or the like.

### (2) In the case where learning information is a correspondence table

The learning unit G933 acquires correspondence information indicating the correspondence between a set of explanatory variables included in the two or more pieces of training data, and measured blood sugar levels included in the pieces of training data, for each piece of training data. Thereafter, the learning unit G933 forms a correspondence table that includes two or more pieces of correspondence information. Note that the set of explanatory variables includes one or more pieces of NIRS information. It is preferable that the set of explanatory variables include one or more kinds of information of: one or more user dynamic attribute values; one or more user static attribute values; and one or more device characteristic values.

### (3) In the case where learning information is an arithmetic formula

The learning unit G933 acquires an arithmetic formula that receives the set of explanatory variables included in the two or more pieces of training data input thereto, and outputs measured blood sugar levels included in the one or more pieces of training data. For example, the learning unit G933 performs a regression analysis to acquire the arithmetic formula. For example, the learning unit G933 performs a multiple regression analysis to acquire the arithmetic formula.

The accumulation unit G934 accumulates the learning information acquired by the learning unit G933. For example, learning information may be accumulated in the learning storage unit G91, but may be accumulated in any other device, and there is no limitation.

It is preferable that the learning storage unit G91 is a non-volatile recording medium, but may be realized using a volatile recording medium.

There is no limitation on the process in which information is stored in the learning storage unit G91. For example, information may be stored in the learning storage unit G91 via a recording medium, or information transmitted via a communication line or the like may be stored in the learning storage unit G91, or information input via an input device may be stored in the learning storage unit G91.

For example, the learning acceptance unit G92 can be realized using a wireless or wired communication means.

The learning processing unit G93, the measured blood sugar level acquisition unit G931, the training data forming unit G932, the learning unit G933, and the accumulation unit G934 can typically be realized using a processor, a memory, or the like. The processing procedures performed by the learning processing unit G93 or the like typically are realized using software, and the software is recorded on a recording medium such as a ROM. However, such processing procedures may be realized using hardware (a dedicated circuit). Note that the processor is a CPU, an MPU, a GPU, or the like, and there is no limitation on the type thereof.

Next, examples of operations of the learning system G will be described. First, examples of operations of the NIRS device E6 here will be described.

Each of the one or more light emitting units E61 in the NIRS device E6 emits near-infrared light. Each of the one or more light receiving units E62 receives the reflected near-infrared light. Next, the NIRS acquisition unit E63 acquires one or more pieces of NIRS information from the received optical signal. For example, the NIRS acquisition unit E63 acquires one or more user dynamic attribute values. For example, the NIRS acquisition unit E63 acquires a measured blood sugar level. There is no limitation on the method for acquiring the measured blood sugar level. The NIRS output unit E64 delivers the one or more pieces of NIRS information acquired by the NIRS acquisition unit E63 and the measured blood sugar level to the learning device G9. Here, the NIRS output unit E64 may also deliver one or more user dynamic attribute values or the like to the learning device G9. Note that, for example, the NIRS output unit E64 transmits the one or more pieces of NIRS information or the like to the learning device G9.

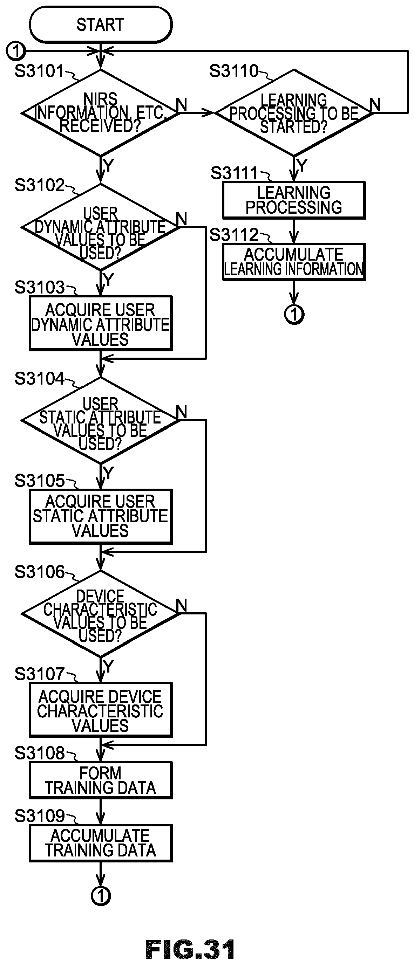

Next, examples of operations of the learning device G9 will be described with reference to the flowchart in FIG. 31.

(Step S3101) The NIRS information acceptance unit E721 judges whether or not NIRS information or the like has been received. If NIRS information or the like has been received, processing proceeds to step S3102, and if NIRS information or the like has not been received, processing proceeds to step S3110. Note that NIRS information or the like include one or more pieces of NIRS information and measured blood sugar levels. For example, NIRS information or the like include one or more user dynamic identifiers, one or more user static identifiers, and one or more device characteristic values.

(Step S3102) The processing unit E73 judges whether or not to use the user dynamic attribute values when acquiring a blood sugar level. If the user dynamic attribute values are to be used, processing proceeds to step S3103, and if the user dynamic attribute values are not to be used, processing proceeds to step S3104. Note that whether or not the user dynamic attribute values are to be used may be determined in advance, or judged based on a user instruction or the like, or judged based on whether or not the user dynamic attribute values can be acquired.

(Step S3103) The user dynamic attribute value acquisition unit E731 acquires one or more user dynamic attribute values. For example, the user dynamic attribute value acquisition unit E731 acquires the one or more user dynamic attribute values that have been received.

(Step S3104) The processing unit E73 judges whether or not to use the user static attribute values when acquiring a blood sugar level. If the user static attribute values are to be used, processing proceeds to step S3105, and if the user static attribute values are not to be used, processing proceeds to step S3106. Note that whether or not the user static attribute values are to be used may be determined in advance, or judged based on a user instruction or the like, or judged based on whether or not the user static attribute values are stored.

(Step S3105) The user static attribute value acquisition unit E732 acquires one or more user static attribute values. For example, the user static attribute value acquisition unit E732 acquires the one or more user static attribute values corresponding to the received user identifier, from the user information storage unit E712.

(Step S3106) The processing unit E73 judges whether or not to use the device characteristic values when acquiring a blood sugar level. If the device characteristic values are to be used, processing proceeds to step S3107, and if the device characteristic values are not to be used, processing proceeds to step S3108. Note that whether or not the device characteristic values are to be used may be determined in advance, or judged based on a user instruction or the like, or judged based on whether or not the device characteristic values are stored.

(Step S3107) The device characteristic value acquisition unit E733 acquires the one or more device characteristic values. For example, the device characteristic value acquisition unit E733 acquires the one or more device characteristic values corresponding to the received user identifier, from the device characteristic value storage unit E713.

(Step S3108) The measured blood sugar level acquisition unit G931 acquires the received measured blood sugar levels. In addition, the training data forming unit G932 acquires the one or more pieces of NIRS information received. In addition, the training data forming unit G932 forms training data, using one or more pieces of NIRS information and measured blood sugar levels.

(Step S3109) The training data forming unit G932 accumulates the training data formed in step S3108, in the learning storage unit G91. The processing returns to step S3101.

(Step S3110) The learning unit G933 judges whether or not to start learning processing. If learning processing is to be started, processing proceeds to step S3111, and if learning processing is not to be started, processing returns to step S3101. Note that, for example, the learning unit G933 judges that learning processing is to be started when accepting a user instruction. For example, the learning unit G933 judges that learning processing is to be started if pieces of training data no less than a threshold value are accumulated in the learning storage unit G91. Note that there is no limitation on the conditions for starting learning processing.

(Step S3111) The learning unit G933 acquires two or more pieces of training data that have been accumulated in the learning storage unit G91. The learning unit G933 acquires learning information by performing learning processing, using the two or more pieces of training data.

(Step S3112) The accumulation unit G934 accumulates the learning information acquired in step S3111. The processing returns to step S3101.

Note that, in the flowchart in FIG. 31, processing ends when the power is turned off or an interruption occurs to end the processing.

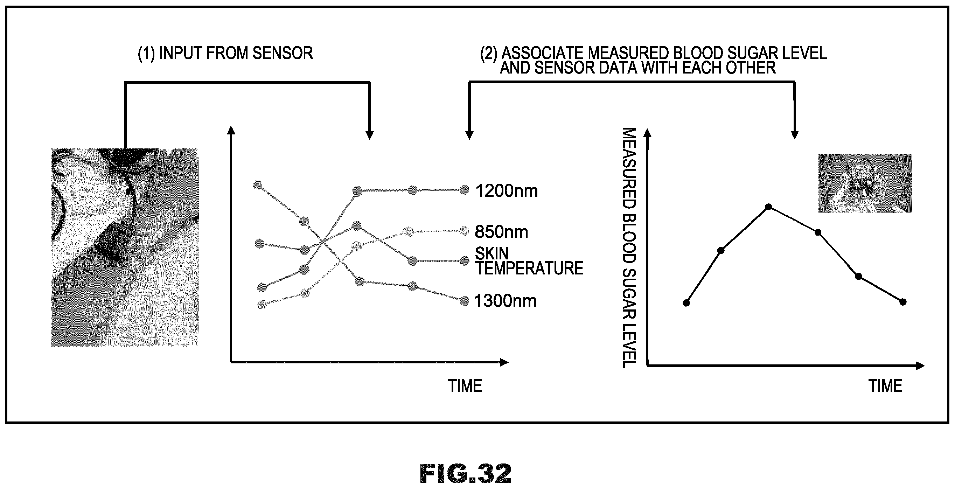

Hereinafter, specific operations of the learning system G according to the present embodiment will be described with reference to FIG. 32. A conceptual diagram for the learning system G is shown in FIG. 30.

The NIRS device E6 placed on the subject's wrist emits near-infrared light rays with wavelengths of 850 nm, 1200 nm, and 1300 nm, and receives the reflected light rays. Thereafter, the NIRS device E6 acquires the intensity of each of the reflected light rays corresponding to the wavelength, in time series ((1) in FIG. 32). In addition, the NIRS device E6 also acquires pieces of time-series information regarding the skin temperature, which is an example of the user dynamic attribute values, using a temperature sensor included in the NIRS device E6 ((1) in FIG. 32). In addition, the NIRS device E6 acquires pieces of time-series information regarding the measured blood sugar level. The NIRS device E6 acquires a large number of pieces of training data in which inputs from the sensor acquired at several points in time in time series (the intensities of the reflected light rays corresponding to the wavelengths and the skin temperatures) are associated with measured blood sugar levels respectively acquired at the same points in time ((2) in FIG. 32). Thereafter, the NIRS device E6 transmits the large number of pieces of training data to the learning device G9.

Next, the learning device G9 receives and accumulates the large number of pieces of training data. Then, when pieces of training data no less than a threshold value are accumulated, the large number of pieces training data are given to the learning module for machine learning, and the learning module is executed and a learning device is acquired and accumulated.

As described above, according to the present embodiment, it is possible to acquire learning information for non-invasively acquiring an appropriate human blood sugar level.

Note that, in the present embodiment, the NIRS device E6 and the learning device G9 may be integrated. In such a case, the learning device G9 is a learning device that includes: one or more light emitting units that emit near-infrared light to a user's human body; one or more light receiving units that correspond to the one or more light emitting units and receive reflected light of the near-infrared light emitted by the one or more light emitting units; a blood sugar level acquisition unit that acquires the user's blood sugar level; a NIRS information acquisition unit that acquires one or more pieces of NIRS information regarding the reflected light, using the reflected light received by the one or more light receiving units; a training data forming unit that forms training data, using the one or more pieces of NIRS information and the blood sugar level; a learning unit that acquires learning information, using the training data; and an accumulation unit that accumulates the learning information.

The software that realizes the learning device G9 according to the present embodiment is the program described below. That is to say, this program is a program for enabling a computer to function as: an NIRS acquisition unit that, using reflected light of near-infrared light emitted from one or more light emitting units and received by one or more light receiving units, acquires one or more pieces of NIRS information regarding the reflected light; a measured blood sugar level acquisition unit that acquires a blood sugar level of a user; a training data forming unit that forms training data, using the one or more pieces of NIRS information and the blood sugar level; a learning unit that acquires learning information, using the training data; and an accumulation unit that accumulates the learning information.

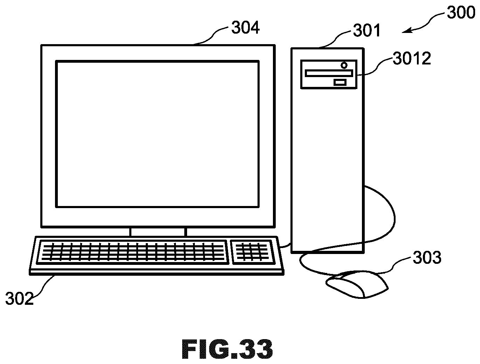

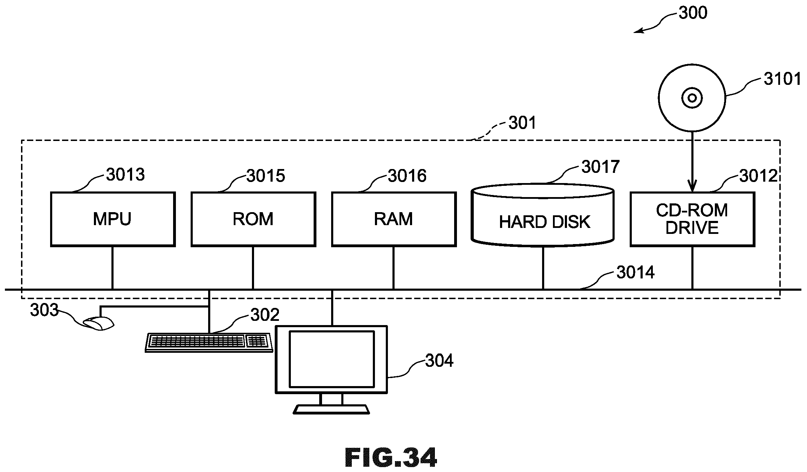

FIG. 33 shows an example of the external appearance of a computer that executes the program described in the present specification and realizes each of the devices according to the various embodiments described above, such as the health support device A and the terminal device C5. The above-described embodiments can be realized using computer hardware and a computer program executed thereon. FIG. 33 is an overview diagram for this computer system 300, and FIG. 34 is a block diagram for the system 300.

In FIG. 33, the computer system 300 includes a computer 301 that includes a CD-ROM drive, a keyboard 302, a mouse 303, a monitor 304, and a microphone 305.

In FIG. 34, the computer 301 includes, in addition to the CD-ROM drive 3012, an MPU 3013, a bus 3014 that is connected to the CD-ROM drive 3012 and so on, a ROM 3015 for storing programs such as a boot-up program, a RAM 3016 that is connected to the MPU 3013 and is used to temporarily store application program instructions and provide a temporary storage space, and a hard disk 3017 for storing application programs, system programs, and data. Here, although not shown in the drawings, the computer 301 may further include a network card that provides connection to a LAN.

The program that enables the computer system 300 to perform the functions of the health support device A and so on according to the above-described embodiments may be stored in the CD-ROM 3101, inserted into the CD-ROM drive 3012, and furthermore transferred to the hard disk 3017. Alternatively, the program may be transmitted to the computer 301 via a network (not shown) and stored on the hard disk 3017. The program is loaded into the RAM 3016 when the program is to be executed. The program may be directly loaded from the CD-ROM 3101 or the network.

The program does not necessarily have to include an operating system (OS), a third-party program, or the like that enables the computer 301 to perform the functions of the health support device A and so on according to the above-described embodiments. The program need only contain the part of the instruction that calls an appropriate function (module) in a controlled manner to achieve a desired result. How the computer system 300 works is well known and the detailed descriptions thereof will be omitted.

In the above-described program, the step of transmitting information, the step of receiving information, and so on do not include processing performed by hardware, such as processing performed by a modem or an interface card in the step of transmitting (processing that can only be performed by hardware).

There may be a single or multiple computers executing the above-described program. That is to say, centralized processing or distributed processing may be performed. In other words, the health support device A may be a stand-alone device or be constituted by two or more devices.

Also, as a matter of course, in each of the above-described embodiments, two or more communication means that are present in one device may be physically realized using one medium.

Also, in the above-described embodiments, each kind of processing may be realized as centralized processing that is performed by a single device, or distributed processing that is performed by multiple devices.

As a matter of course, the present invention is not limited to the above-described embodiments, and various changes are possible, and such variations are also included within the scope of the present invention.

### Industrial Applicability

As described above, the health support device according to an aspect of the present invention achieves the effects of being capable of acquiring appropriate information regarding blood sugar levels and alert the user, and being capable of acquiring appropriate information regarding changes in blood sugar levels and alert the user, and is useful as a health support device or the like.

## Claims

1. A health support device comprising:
a blood sugar level acquisition unit that acquires a blood sugar level of a user;
a judgment unit that judges whether or not blood sugar level-related information regarding the blood sugar level satisfies a predetermined output condition to acquire a judgment result; and
an output unit that outputs blood sugar level information regarding the blood sugar level when the judgment result indicates that the output condition is satisfied.

2. The health support device according to claim 1,
wherein the blood sugar level acquisition unit acquires a blood sugar level group constituted by two or more blood sugar levels of the user acquired in a time series,
the health support device further comprises a fluctuation information acquisition unit that acquires fluctuation information regarding blood sugar level fluctuations over time, using the blood sugar level group,
the judgment unit judges whether or not the fluctuation information satisfies a predetermined output condition, to acquire the judgment result, and
the output unit outputs the fluctuation information when the judgment result indicates that the output condition is satisfied.

3. The health support device according to claim 2, further comprising
a reference information storage unit that stores reference information regarding a refence blood sugar level,
wherein the fluctuation information acquisition unit uses the blood sugar level group and the reference information to acquire the fluctuation information.

4. The health support device according to claim 2, further comprising
a past blood sugar level group storage unit that stores a past blood sugar level group constituted by two or more past blood sugar levels of the user,
wherein the fluctuation information acquisition unit uses the blood sugar level group and the past blood sugar level group to acquire the fluctuation information.

5. The health support device according to claim 2,
wherein the output condition is a warning condition for outputting a warning, and
the fluctuation information acquisition unit acquires fluctuation information regarding the warning when the judgment result indicates that the warning condition is satisfied.

6. The health support device according to claim 1,
wherein the judgment unit judges whether or not blood sugar level-related information regarding the blood sugar level satisfies a predetermined suggestion condition, to acquire a judgment result, and
the health support device further comprising:
a suggestion information acquisition unit that acquires suggestion information when the judgment result indicates that the suggestion condition is satisfied; and
a suggestion information output unit that outputs the suggestion information.

7. The health support device according to claim 6,
wherein the blood sugar level acquisition unit acquires a blood sugar level group constituted by two or more blood sugar levels of one user acquired in a time series on one day,
the health support device further comprises a fluctuation information acquisition unit that acquires fluctuation information regarding blood sugar level fluctuations over time, using the blood sugar level group,
the judgement unit judges whether or not the fluctuation information satisfies the suggestion condition, and
the suggestion information acquisition unit acquires suggestion information corresponding to the fluctuation information when the judgment result indicates that the suggestion condition is satisfied.

8. The health support device according to claim 7, further comprising
a reference information storage unit that stores reference information regarding a refence blood sugar level,
wherein the fluctuation information acquisition unit uses the blood sugar level group and the reference information to acquire the fluctuation information.

9. The health support device according to claim 7, further comprising
a past blood sugar level group storage unit that stores a past blood sugar level group constituted by two or more past blood sugar levels of the user,
wherein the fluctuation information acquisition unit uses the blood sugar level group and the past blood sugar level group to acquire the fluctuation information.

10. The health support device according to claim 6,
wherein the suggestion information acquisition unit determines a suggestion condition that matches acquired blood sugar level-related information from a correspondence information storage unit that stores two or more pieces of correspondence information that each indicate a correspondence between a suggestion condition regarding blood sugar level related information and suggestion information or suggestion basis information that is a basis of suggestion information, and acquires suggestion information corresponding to the suggestion condition or suggestion information that uses suggestion basis information corresponding to the suggestion condition.

11. The health support device according to claim 6,
wherein the judgment unit uses the blood sugar level and other information other than the fluctuation information to judge whether or not the blood sugar level or the fluctuation information satisfies the suggestion condition.

12. The health support device according to claim 6,
wherein the suggestion information acquisition unit uses the blood sugar level and other information other than the fluctuation information to acquire the suggestion information.

13. The health support device according to claim 11, further comprising
an environment information acquisition unit that acquires environment information regarding user environment,
wherein the other information is the environmental information.

14. The health support device according to claim 7,
wherein the fluctuation information acquisition unit acquires fluctuation information that is a score corresponding to fluctuations over time of a blood sugar level, using the blood sugar level group.

15. A blood sugar level estimation device that acquires a blood sugar level that is to be acquired by the blood sugar level acquisition unit according to claim 1, comprising:
a learning information storage unit that stores learning information acquired using two or more pieces of training data that each include one or more pieces of NIRS information acquired using a reflected light of near-infrared light emitted to a human body and a blood sugar level;
an NIRS acquisition unit that acquires one or more pieces of NIRS information that can be acquired by emitting near-infrared light to a user; and
an estimation unit that acquires an estimated blood sugar level, using one or more pieces of NIRS information acquired by the NIRS acquisition unit and the learning information.

16. The blood sugar level estimation device according to claim 15,
wherein the training data includes one or more pieces of NIRS information that can be acquired by emitting near-infrared light of two or more wavelengths to a human body, and
the NIRS acquisition unit acquires one or more pieces of NIRS information that can be acquired by emitting near-infrared light of two or more wavelengths to a user.

17. The blood sugar level estimation device according to claim 15,
wherein the training data includes one or more user static attribute values that are static attribute values of a person with the human body,
the blood sugar level estimation device further comprises a user static attribute value acquisition unit that acquires one or more user static attribute values of the user, and
the estimation unit uses the one or more pieces of NIRS information acquired by the NIRS acquisition unit, the one or more user static attribute values acquired by the user static attribute value acquisition unit, and the learning information to acquire the estimated blood sugar level.

18. The blood sugar level estimation device according to claim 15,
wherein the training data includes one or more device characteristic values that are characteristic values of a device that is used to acquire the one or more pieces of NIRS information,
the blood sugar level estimation device further comprises a device characteristic value acquisition unit that acquires one or more device characteristic values of a device that is used to acquire one or more pieces of NIRS information of the user, and
the estimation unit uses the one or more pieces of NIRS information acquired by the NIRS acquisition unit, the one or more device characteristic values acquired by the device characteristic values acquisition unit, and the learning information to acquire the estimated blood sugar level.

19. The blood sugar level estimation device according to claim 15,
wherein the learning information is a learning model acquired through machine learning processing using the two or more pieces of training data.

20. A learning device that acquires learning information that is used by the blood sugar level estimation device according to claim 15, comprising:
an NIRS acquisition unit that, using reflected light of near-infrared light emitted from one or more light emitting units and received by one or more light receiving units, acquires one or more pieces of NIRS information regarding the reflected light;
a measured blood sugar level acquisition unit that acquires a blood sugar level of a user;
a training data forming unit that forms training data, using the one or more pieces of NIRS information and the blood sugar level; and
a learning unit that acquires learning information, using the training data.

21. The learning device according to claim 20, further comprising
a user static attribute value acquisition unit that acquires one or more user static attribute values that are static attribute values of the user,
wherein the training data forming unit uses the one or more pieces of NIRS information, the one or more user static attribute values, and the blood sugar level to form the training data.

22. The learning device according to claim 20, further comprising
a device characteristic value acquisition unit that acquires one or more device characteristic values of a device that includes the one or more light receiving units and the one or more light transmitting units,
wherein the training data forming unit uses the one or more pieces of NIRS information, the one or more device characteristic values, and the blood sugar level to form the training data.

23. A health support method that is realized using a blood sugar level acquisition unit, a judgment unit, and an output unit, comprising:
a blood sugar level acquisition step in which the blood sugar level acquisition unit acquires a blood sugar level of a user;
a judgment step in which the judgment unit judges whether or not the blood sugar level satisfies a predetermined output condition; and
an output step in which the output unit outputs blood sugar level information regarding the blood sugar level when the judgment result indicates that the output condition is satisfied.

24. A recording medium having recorded thereon a program for enabling a computer to function as:
a blood sugar level acquisition unit that acquires a blood sugar level of a user;
a judgment unit that judges whether or not the blood sugar level satisfies a predetermined output condition; and
an output unit that outputs blood sugar level information regarding the blood sugar level when the judgment result indicates that the output condition is satisfied.
